# EUROPÄISCHE PATENTSCHRIFT

(11) **EP 1 957 481 B1**
(45) Veröffentlichungstag und Bekanntmachung des Hinweises auf die Patenterteilung: **02.03.2011**
(21) Anmeldenummer: 06818896.0
(22) Anmeldetag: 29.11.2006
(51) Int. Cl.: C07D 403/04, A61P 37/00, A61K 31/495

(54) **IN 3-POSITION HETEROCYCLISCH SUBSTITUIERTE PYRROLIDIN(THI)ONE**
PYRROLIDIN(THI)ONES HETEROCYCLICALLY SUBSTITUTED IN 3-POSITION
PYRROLIDIN(THI)ONES A SUBSTITUTION HETEROCYCLIQUE EN POSITION 3

(30) Priorität: 02.12.2005 DE 102005057912
(43) Veröffentlichungstag der Anmeldung: 20.08.2008
(73) Patentinhaber: Grünenthal GmbH, 52078 Aachen (DE)
(72) Erfinder: FRORMANN, Sven, 52066 Aachen (DE); FROSCH, Stefanie, 52078 Aachen (DE); GRIEBEL, Carsten, 52080 Aachen (DE); SAUNDERS, Derek, 52072 Aachen (DE); THEIL, Fritz, 12247 Berlin (DE); GRAUBAUM, Heinz, 12557 Berlin (DE)
(86) Internationale Anmeldenummer: PCT/EP2006/011440
(87) Internationale Veröffentlichungsnummer: WO 2007/062817

(56) Entgegenhaltungen:
- JP-A- 2003 300 886
- JOENSSON N A ET AL: "CHEMICAL STRUCTURE AND TERATOGENIC PROPERTIES. PART 2: SYNTHESIS AND TERATOGENIC ACTIVITY IN RABBITS OF SOME DERIVATIVES OF PHTHALIMIDE, ISOINDOLINE-1-ONE, 1,2-BENZISOTHIAZOLINE-3-ONE-1,1-DIOXIDE AND 4(3H)-QUINAZOLINONE" ACTA PHARMACEUTICA SUECICA, XX, XX, Bd. 9, 1972, Seiten 431-446, XP009082387 ISSN: 0001-6675

## Beschreibung

Die Erfindung betrifft in 3-Position heterocyclisch substituierte Pyrrolidin(thi)one der allgemeinen Formel (I) 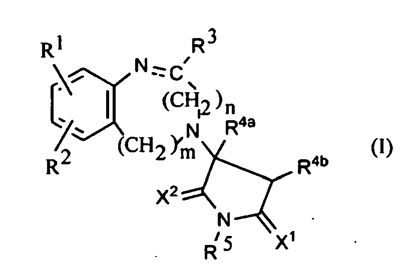 ihre Herstellung sowie ihre Verwendung in Arzneimitteln.

Autoimmunerkrankungen entstehen aufgrund einer Reaktivität des Immunsystems gegen körpereigene Strukturen. Dabei ist die normalerweise vorhandene Toleranz gegenüber körpereigenem Gewebe aufgehoben. In der Pathogenese der verschiedenen Autoimmunerkrankungen spielen neben Antikörpern insbesondere T-Lymphozyten und Monozyten/Makrophagen eine entscheidende Rolle. Aktivierte Monozyten/Makrophagen sezernieren eine Vielzahl verschiedener entzündungsfördemder Mediatoren, die direkt oder indirekt für die Zerstörung der von der Autoimmunerkrankung betroffenen Gewebe verantwortlich sind. Die Aktivierung von Monozyten/Makrophagen erfolgt entweder in der Interaktion mit T-Lymphozyten oder über bakterielle Produkte wie Lipopolysaccharid (LPS). Eine von aktivierten Monozyten/Makrophagen gebildete entzündungsfördernde Substanz ist das Interleukin-12 (IL-12).

IL-12 ist ein heterodimeres Molekül, welches aus einer kovalent verbundenen p35 und p40 Kette besteht. Es wird von antigenpräsentierenden Zellen (Monozyten/Makrophagen, dendritische Zellen, B-Lymphozyten) nach Aktivierung durch verschiedene mikrobielle Produkte wie LPS, Lipopeptide, bakterielle DNA oder in der Interaktion mit aktivierten T-Lymphozyten gebildet (Trinchieri 1995. Ann.Rev.lmmunol. 13: 251). IL-12 hat eine zentrale immunregulatorische Bedeutung und ist verantwortlich für die Entwicklung entzündungsfördernder TH1 Reaktivitäten. Beim Vorliegen einer TH1 Immunreaktion gegen Eigenantigene kommt es zum Auftreten schwerer Erkrankungen, wie in zahlreichen tierexperimentellen und ersten klinischen Untersuchungen klar dokumentiert wird. In verschiedenen Tiermodellen für Erkrankungen wie Rheumatoide Arthritis, Multiple Sklerose, Diabetes mellitus sowie entzündliche Darm-, Haut- und Schleimhauterkrankungen zeigt sich die pathophysiologische Bedeutung von IL-12 (Trembleau et al. 1995. Immunol.Today 16: 383; Müller et al. 1995. J.Immunol. 155: 4661; Neurath et al. 1995. J.Exp.Med. 182: 1281; Segal et al. 1998. J.Exp.Med. 187: 537; Powrie et al. 1995. Immunity 3: 171; Rudolphi et al. 1996. Eur.J.lmmunol. 26: 1156; Bregenholt et al. 1998. Eur.J.Immunol. 28: 379). Durch Applikation von IL-12 ließ sich die jeweilige Erkrankung auslösen bzw. nach Neutralisierung von endogenem IL-12 zeigte sich ein abgeschwächter Krankheitsverlauf bis hin zu einer Heilung der Tiere. Antikörper gegen IL-12 sind bereits in der klinischen Prüfung für die Behandlung von Morbus Crohn, Psoriasis und Multiple Sklerose.

Das Zytokin IL-10 inhibiert die Synthese der entzündungsfördernden Zytokine TNFα, IL-1, IL-6, IL-8, IL-12 und GM-CSF durch humane und murine Monozyten/Makrophagen (Fiorentino et al., 1991. J.Immunol. 146: 3444; De Waal Malefyt et al. 1991. J.Exp.Med. 174:1209). Dadurch kommt es indirekt auch zu einer Hemmung der Synthese von IFN-γ durch TH1 Lymphozyten. Interessanterweise tritt die Bildung von IL-10 durch Monozyten/Makrophagen mit einer geringen zeitlichen Verzögerung gegenüber der Synthese der entzündungsfördernden Zytokine auf. Die Behandlung von antigenpräsentierenden Zellen mit IL-10 resultiert in deren Deaktivierung. Solche Zellen sind nicht in der Lage, T-Lymphozyten zur Proliferation bzw. zur Synthese von IFN-γ zu aktivieren. Jedoch sezernieren diese T-Lymphozyten selbst große Mengen an IL-10 und vermögen Entzündungsreaktionen zu unterdrücken, wie am Beispiel eines Tiermodells zu entzündlichen Darmerkrankungen gezeigt werden konnte (Groux et al., 1997. Nature 389: 737). Auch die Entwicklung entzündlicher Hauterkrankungen kann durch IL-10 verhindert werden (Enk et al., 1994. J.Exp.Med. 179: 1397).

Zusammenfassend lässt sich sagen, dass ein Überschuss an IL-12 bzw. ein Mangel an IL-10 die Pathophysiologie einer Vielzahl entzündlicher/Autoimmun-Erkrankungen bedingt. Ansätze zur Wiederherstellung des Gleichgewichts zwischen entzündungsfördernden (IL-12) und entzündungshemmenden (IL-10) Zytokinen haben daher ein großes therapeutisches Potential bei oben genannten Erkrankungen.

IL-12 ist darüber hinaus auch an der Regulation des Überlebens von Zellen beteiligt. Unkontrolliertes Zellwachstum wird u. a. durch Apoptose (programmierter Zelltod) reguliert. An T-Lymphozyten wurde gezeigt, dass IL-12 eine anti-apoptotische Wirkung besitzt und das Überleben von T-Zellen fördert (Clerici et al. 1994. Proc.Natl.Acad.Sci.USA 91: 11811; Estaquier et al. 1995. J.Exp.Med. 182: 1759). Eine lokale Überproduktion von IL-12 kann daher zum Überleben von Tumorzellen beitragen. Inhibitoren der Bildung von IL-12 besitzen daher auch in der Tumortherapie ein großes therapeutisches Potential.

Eine Substanz mit dem immunmodulatorischen Wirkprinzip der IL-12 Hemmung und der IL-10 Steigerung ist Thalidomid. Klinische Studien haben in jüngster Zeit den positiven Einfluss von Thalidomid auf folgende Erkrankungen gezeigt: Erythema nodosum leprosum (Sampaio et al. 1993. J.Infect.Dis. 168: 408), Aphthosis (Jacobson et al. 1997. N.EngLJ.Med. 336: 1487), chronische Abstoßungsreaktionen (Vogelsang, et al. 1992. N.Engl.J.Med. 326: 1055), entzündliche Darmerkrankungen (Ehrenpreis et al. 1999. Gastroenterology 117: 1271; Vasiliauskas et al. 1999. Gastroenterology 117: 1278) sowie zahlreiche Hauterkrankungen (Bernal et al. 1992. Int.J.Derm. 31: 599). Klinische Studien laufen derzeit auch zur Therapie für eine Reihe von Tumorerkrankungen (Rajkumar, 2001. Oncology 15: 867). Eine Wirksamkeit bei multiplem Myelom scheint sicher (Singhal, 1999. N.Engl.J.Med. 341: 1565).
Thalidomid löst jedoch auch eine Reihe von Nebenwirkungen aus, zu denen Sedation, Teratogenität und Neuropathie zählen. Ferner ist die Substanz schwer löslich und stark hydrolyseempfindlich.

Die der Erfindung zugrunde liegende Aufgabe bestand deshalb darin, neue Verbindungen zu generieren, die das oben beschriebene immunmodulierende Prinzip besitzen.

Diese an die zu generierenden Verbindungen gestellten Anforderungen werden von bestimmten substituierten Pyrrolidin(thi)onen erfüllt.

Gegenstand der Erfindung sind dementsprechend in 3-Position heterocyclisch substituierte Pyrrolidin(thi)one der allgemeinen Formel (I), 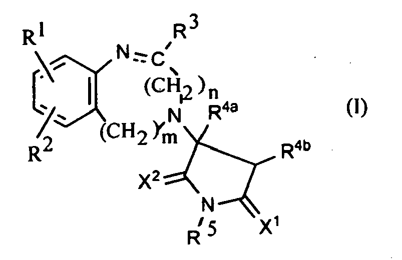 in denen
- R¹ und R²,: gleich oder verschieden voneinander, H, Br, Cl, F, I, CF₃, OH, NO₃, NR⁵ R⁶, Alkyl, Alkoxy, Alkylthio, Alkylsulfinyl, Alkylsulfonyl, Aryl, Heteroaryl, jeweils verzweigt oder unverzweigt sein können oder zusammengenommen einen ankondensierten Benzolring bedeuten, wobei die Ringe gegebenenfalls mit R¹ und/oder R² substituiert und R¹ und R² wie oben definiert sind, und R⁵ und R⁶ ausgewählt sind aus H, Alkyl- oder Acylresten,
- R³: H, die Methylgruppe oder, für den Fall, dass eine C-N-Einfachbindung vorliegt, zusammen mit dem C-Atom eine Carbonyl-Gruppe darstellt,
- R^{4a} und/oder R^{4b}: H, Alkyl, Aryl oder Heteroaryl bedeutet,
- R⁵: für H, Aryl, Heteroaryl, Alkyl, eine CH₂-OH- Gruppe oder einen Rest CH₂-NR⁶R⁷, in dem R⁶ und R⁷ gleich oder verschieden sind und eine Alkylgruppe mit 1-6 C-Atomen (geradkettig oder verzweigt) oder zusammen mit dem N-Atom einen Pyrrolidin-, Piperidin-, Hexamethylenimin- oder Morpholinring bedeuten, steht,
- X¹ und/oder X²: für O oder S stehen und die verbleibenden X¹ oder X² H₂ bedeuten,
- n: 0 oder 1 und
- m: 1 oder 2 bedeutet.

Besonders bevorzugte Verbindungen sind solche mit C=N-Doppeibindung, in denen R¹ und R², gleich oder verschieden voneinander, H, Br, Cl, F, CF₃, NO₂, NH₂, C₁₋₃-Alkyl, C₁₋₃-Alkoxy, oder zusammengenommen einen ankondensierten Benzolring bedeuten, R³ für H oder eine Methylgruppe , R^{4a} für H oder eine Methylgruppe, R^{4b} für H oder eine Phenylgruppe und R⁵ für H oder Methyl steht, X¹ und X² für O stehen und n = 0 und m = 1 ist.

Wiederum bevorzugt sind Verbindungen mit C=N-Doppelbindung, in denen R¹ und R², gleich oder verschieden voneinander, H, Cl, oder F bedeuten, R³, R^{4a}, R^{4b} und R⁵ Wasserstoff bedeuten, X¹ und X² für O stehen, und n=Oundm=1 1 ist.

Zu weiteren bevorzugten Verbindungen zählen:
3-(5-Chlor-7-fluor-4*H*-chinazolin-3-yl)-pyrrolidin-2,5-dion **(1)**
3-(7-Chlor-5-fluor-4*H*-chinazolin-3-yl)-pyrrolidin-2,5-dion **(2)**
3-(7-Fluor-4*H*-chinazolin-3-yl)-pyrrolidin-2,5-dion und dessen Hydrochlorid **(3)**
3-(5,7-Difluor-4*H*-chinazolin-3-yl)-pyrrolidin-2,5-dion und dessen Hydrochlorid **(4)**
3-(5,7-Dichlor-4*H*-chinazolin-3-yl)-pyrrolidin-2,5-dion **(5)**
3-(5-Brom-4*H*-chinazolin-3-yl)-pyrrolidin-2,5-dion **(6)**
3-(7-Trifluormethyl-4*H*-chinazolin-3-yl)pyrrolidin-2,5-dion **(7)**
3-(5,8-Dichlor-4*H*-chinazolin-3-yl)-pyrrolidin-2,5-dion **(8a)**
3-(5-Methyl-4*H*-chinazolin-3-yl)-pyrrolidin-2,5-dion **(8b)**
3-(4*H*-Benzo[g]chinazolin-3-yl)-pyrrolidin-2,5-dion **(8c)**
3-(6,7-Diftuor-4*H*-chinazolin-3-yl)-pyrrolidin-2,5-dion **(8d)**
3-(6,8-Dichlor-4*H*-chinazolin-3-yl)-pyrrolidin-2,5-dion **(8e)**
3-(6-Nitro-4*H*-chinazolin-3-yl)-pyrrolidin-2,5-dion **(8f)**
3-(7-Chlor-4*H*-chinazolin-3-yl)-pyrrolidin-2,5-dion **(8g)**
3-(7-Nitro-4*H*-chinazolin-3-yl)-pyrrolidin-2,5-dion **(8h)**
3-(8-Brom-6-methyl-4*H*-chinazolin-3-yl)-pyrrolidin-2,5-dion **(8i)**
3-(8-Chlor-4*H*-chinazolin-3-yl)-pyrrolidin-2,5-dion **(8j)**
3-(8-Methoxy-4*H*-chinazolin-3-yl)-pyrrolidin-2,5-dion **(8k)**
3-(6-Benzyloxy-4*H*-chinazolin-3-yl)-pyrrolidin-2,5-dion **(8l)**
3-(5,6-Dichlor-4*H*-chinazolin-3-yl)-pyrrolidin-2,5-dion **(9a)**
3-(7-Methoxy-4*H*-chinazolin-3-yl)-pyrrolidin-2,5-dion **(9b)**
3-(5-Chlor-4*H*-chinazolin-3-yl)-pyrrolidin-2,5-dion **(10a)**
3-(6,7-Dimethoxy-4*H*-chinazolin-3-yl)-pyrrolidin-2,5-dion **(10b)**
**3-(6-Chlor-4*H*-chinazolin-3-yl)-pyrrolidin-2,5-dion (10c)**
3-(4*H*-Chinazolin-3-yl)-pyrrolidin-2,5-dion **(11a)**
3-(5-Fluor-4*H*-chinazolin-3-yl)-pyrrolidin-2,5-dion **(11b)**
3-(8-Trifluormethyl-4*H*-chinazolin-3-yl)-pyrrolidin-2,5-dion **(11c)**
1-Methyl-3-(4*H*-chinazolin-3-yl)-pyrrolidin-2,5-dion **(11d)**
7-Fluor-1-methyl-3-(4*H*-chinazolin-3-yl)-pyrrolidin-2,5-dion **(11e)**
3-(5-Methoxy-4*H*-chinazolin-3-yl)-pyrrolidin-2,5-dion **(12a)**
3-(5-Ethoxy-4*H*-chinazolin-3-yl)-pyrrolidin-2,5-dion **(12b)**
3-(5-Pentyloxy-4*H*-chinazolin-3-yl)-pyrrolidin-2,5-dion **(12c)**
3-(5-Benzyloxy-4*H*-chinazolin-3-yl)-pyrrolidin-2,5-dion **(12d)**
3-(5-lsopropoxy-4*H*-chinazolin-3-yl)-pyrrolidin-2,5-dion **(12e)**
3-(5-(2-Methoxyethoxy)-4*H*-chinazolin-3-yl)-pyrrolidin-2,5-dion **(12f)**
3-(5-Ethansulfonyl-4*H*-chinazolin-3-yl)-pyrrolidin-2,5-dion **(13a)**
3-(5-Ethansulfinyl-4*H*-chinazolin-3-yl)-pyrrolidin-2,5-dion **(13c)**
3-(5-Ethylthio-4*H*-chinazolin-3-yl)-pyrrolidin-2,5-dion **(13b)**
3-(5-Nitro-4*H*-chinazolin-3-yl)-pyrrolidin-2,5-dion **(14)**
1-Methyl-3-(2-methyl-4*H*-chinazolin-3-yl)-pyrrolidin-2,5-dion **(15a)**
3-(2-Methyl-4*H*-chinazolin-3-yl)-pyrrolidin-2,5-dion **(15b)**
3-(5,6-Dichlor-2-methyl-4*H*-chinazolin-3-yl)-pyrrolidin-2,5-dion **(15c)**
3-(5,7-Dichlor-2-methyl-4*H*-chinazolin-3-yl)-pyrrolidin-2,5-dion **(15d)**
3-(5,7-Difluor-2-methyl-4*H*-chinazolin-3-yl)-pyrrolidin-2,5-dion und dessen Hydrochlorid **(15e)**
3-(5,8-Dichlor-2-methyl-4*H*-chinazolin-3-yl)-pyrrolidin-2,5-dion **(15f)**
3-(5-Benzyloxy-2-methyl-4*H*-chinazolin-3-yl)-pyrrolidin-2,5-dion **(15g)**
3-(5-Brom-2-methyl-4*H*-chinazolin-3-yl)-pyrrolidin-2,5-dion **(15h)**
3-(5-Chlor-2-methyl-4*H*-chinazolin-3-yl)-pyrrolidin-2,5-dion **(15i)**
3-(5-Chlor-7-fluor-2-methyl-4*H*-chinazolin-3-yl)-pyrrolidin-2,5-dion und dessen Hydrochlorid **(15j)**
3-(5-Ethoxy-2-methyl-4*H*-chinazolin-3-yl)-pyrrolidin-2,5-dion **(15k)**
3-(5-FLuor-2-methyl-4*H*-chinazolin-3-yl)-pyrrolidin-2,5-dion **(15l)**
3-(5-Isopropoxy-2-methyl-4*H*-chinazolin-3-yl)-pyrrolidin-2,5-dion **(15m)**
3-(2,5-Dimethyl-4*H*-chinazolin-3-yl)-pyrrolidin-2,5-dion **(15n)**
3-(5-Methoxy-2-methyl-4*H*-chinazolin-3-yl)-pyrrolidin-2,5-dion **(15o)**
3-(2-Methyl-5-nitro-4*H*-chinazolin-3-yl)-pyrrolidin-2,5-dion **(15p)**
3-(2-Methyl-5-pentyloxy-4*H*-chinazolin-3-yl)-pyrrolidin-2,5-dion **(15q)**
3-(5-Ethylthio-2-Methyl-4*H*-chinaznlin-3-yl)-pyrrolidin-2,5-dion **(15r)**
3-(5-Ethansulfonyl-2-methyl-4*H*-chinazolin-3-yl)-pyrrolidin-2,5-dion **(15s)**
3-(2-Methyl-4*H*-benzo[g]chinazolin-3-yl)-pyrrolidin-2,5-dion **(15t)**
3-(6,7-Difluor-2-methyl-4*H*-chinazolin-3-yl)-pyrrolidin-2,5-dion **(15u)**
3-(6,7-Dimethoxy-2-methyl-4*H*-chinazolin-3-yl)-pyrrolidin-2,5-dion **(15v)**
3-(6,8-Dichlor-2-methyl-4*H*-chinazolin-3-yl)-pyrrolidin-2,5-dion **(15w)**
3-(6-Benzyloxy-2-methyl-4*H*-chinazolin-3-yl)-pyrrolidin-2,5-dion **(15x)**
3-(6-Chlor-2-methyl-4*H*-chinazolin-3-yl)-pyrrolidin-2,5-dion **(15y)**
3-(2-Methyl-6-nitro-4*H*-chinazolin-3-yl)-pyrrolidin-2,5-dion **(15z)**
3-(2-Methyl-7-tritluormethyl-4*H*-chinazolin-3-yl)-pyrrolidin-2,5-dion **(15aa)**
3-(7-Chlor-2-methyl-4*H*-chinazolin-3-yl)-pyrrolidin-2,5-dion **(15bb)**
3-(7-Fluor-2-methyl-4*H*-chinazolin-3-yl)-pyrrolidin-2,5-dion **(15cc)**
3-(7-Methoxy-2-methyl-4*H*-chinazolin-3-yl)-pyrrolidin-2,5-dion **(15dd)**
3-(2-Methyl-7-nitro-4*H*-chinazolin-3-yl)-pyrrolidin-2,5-dion **(15ee)**
3-(8-Brom-2,6-dimethyl-4*H*-chinazolin-3-yl)-pyrrolidin-2,5-dion **(15ff)**
3-(2-Methyl-8-trifluormethyl-4*H*-chinazolin-3-yl)-pyrrolidin-2,5-dion **(15gg)**
3-(8-Chlor-2-methyl-4*H*-chinazolin-3-yl)-pyrrolidin-2,5-dion **(15hh)**
3-(8-Methoxy-2-methyl-4*H*-chinazolin-3-yl)-pyrrolidin-2,5-dion **(15ii)**
3-(5-(2-Methoxyethoxy)-2-methyl-4*H*-chinazolin-3-yl) pyrrolidin-2,5-dion **(15jj)**
3-(7-Amino-4*H*-chinazolin-3-yl)-pyrrolidin-2,5-dion-Hydrochlorid **(16a)**
3-(7-Amino-2-methyl-4*H*-chinazolin-3-yl)-pyrrolidin-2,5-dion Hydrochlorid **(16b)**
3-(6-Amino-4*H*-chinazolin-3-yl)-pyrrolidin-2,5-dion Hydrochlorid **(16c)**
3-(6-Amino-2-methyl-4*H*-chinazolin-3-yl)-pyrrolidin-2,5-dion-Hydrochlorid **(16d)**
3-(5-Amino-4*H*-chinazolin-3-yl)-pyrrolidin-2,5-dion Hydrochlorid **(16e)**
3-(5-Amino-2-methyl-4*H*-chinazolin-3-yl)-pyrrolidin-2,5-dion Hydrochlorid **(16f)**
3-(5-Hydroxy-4*H*-chinazolin-3-yl)-pyrrolidin-2,5-dion Hydrochlorid **(17a)**
3-(6-Hydroxy-2-methyl-4*H*-chinazolin-3-yl)-pyrrolidin-2,5-dion Hydrochlorid **(17b)**
3-(5-Hydroxy-2-methyl-4*H*-chinazolin-3-yl)-pyrrolidin-2,5-dion Hydrochlorid **(17c)**
3-(5-Hydroxy-2-methyl-4*H*-chinazolin-3-yl)-pyrrolidin-2,5-dion Hydrochlorid **(17d)**
3-(7-Fluor-4*H*-chinazolin-3-yl)-5-thioxopyrrolidin-2-on-Hydrobromid **(18a)**
3-(4*H*-Chinazolin-3-yl)-5-thioxopyrrolidin-2-on-Hydrobromid **(18b)**
3-Methyl-3-(4*H*-chinazolin-3-yl)-pyrrolidin-2,5-dion **(19a)**
3-(5-Brom-4*H*-chinazolin-3-yl)-3-methyl-pyrrolidin-2,5-dion **(19b)**
3-(2-Oxo-1,4-dihydro-2*H*-chinazolin-3-yl)-pyrrolidin-2,5-dion **(20a)**
3-(7-Fluor-2-oxo-1,4-dihydro-2*H*-chinazolin-3-yl)-pyrrolidin-2,5-dion **(20b)**
3-(2-Oxo-1,4-dihydro-2*H*-chinazolin-3-yl)-4-phenylpyrrolidin-2,5-dion **(20c)**
3-(2-Thio-1,4-dihydro-2*H*-chinazolin-3-yl)-pyrrolidin-2,5-dion **(20d)**
3-(2-(Methylthio)-4*H*-chinazolin-3-yl)pyrrolidin-2,5-dion Hydroiodid **(21)**
3-(2-(Dimethylamino)-4-chinazolin-3-yl)pyrrolidin-2,5-dion Hydroiodid **(22)**
3-(4*H*-Chinazolin-3-yl)-pyrrolidin-2-on **(23).**

Ganz besonders bevorzugt sind davon die Verbindungen:
3-(7-Fluor-4*H*-chinazolin-3-yl)-pyrrolidin-2,5-dion und dessen Hydrochlorid **(3)**
3-(5,7-Difiuor-4*H*-chinazolin-3-yl)-pyrrolidin-2,5-dion und dessen Hydrochlorid **(4).**

Der Ausdruck "C₁-C₁₀-Alkyl" bedeutet im Sinne dieser Erfindung Kohlenwasserstoffe mit 1 bis 10 Kohlenstoffatomen. Beispielhaft seien Methyl, Ethyl, Propyl, Isopropyl, n-Butan, sek. Butyl, tert. Butyl, n-Pentan, Neopentyl, n-Butan, sek. Butyl, tert. Butyl, n-Hexan, n-Heptan, n-Octan, n-Nonan, n-Decan unsubstituiert oder ein oder mehrfach substituiert genannt.

Der Ausdruck "C₂-C₁₀-Alkenyl" bzw. "C₂-C₁₀-Alkinyl bedeutet im Sinne dieser Erfindung Kohlenwasserstoffe mit 2 bis 10 Kohlenstoffatomen. Beispielhaft genannt seien Ethenyl, Propenyl, Butenyl, Pentenyl, Hexenyl, Heptenyl, Octenyl unsubstituiert oder ein oder mehrfach substituiert bzw. Ethinyl, Propinyl, Butinyl, Pentinyl, Hexinyl, Heptinyl, Octinyl unsubstituiert oder ein oder mehrfach substituiert.

Der Ausdruck C₃-C₇-Cycloalkyl bedeutet im Sinne dieser Erfindung cyclische Kohlenwasserstoffe mit 3 bis 7 Kohlenstoffatomen. Beispielhaft seien Cyclopropyl, Cyclobutyl, Cyclopentyl, Cyclohexyl, Cycloheptyl, Cyclohexenyl oder Cycloheptenyl gesättigt oder ungesättigt, unsubstituiert oder ein oder mehrfach substituiert genannt. Dabei versteht man im Sinne der Erfindung unter einem "entsprechenden Heterocyclus" ein C₃-C₇-Cycloalkyl, bei dem mindestens ein C-Atom im Ring durch S, O oder N ersetzt ist. Beispielhaft seien hierfür Pyrrolidin, Pyran, Thiolan, Piperidin oder Tetrahydrofuran aufgeführt.

Der Ausdruck "Aryl" bedeutet im Sinne dieser Erfindung Phenyle oder Naphthyle.

Der Ausdruck "Alkylaryl" bedeutet im Sinne dieser Erfindung mit C₁-C₁₀-Alkylen substituierte Aryle, wobei die Ausdrücke Aryl und Alkyl die gleiche Bedeutung haben wie oben.

Der Ausdruck "Heteroaryl" bedeutet im Sinne dieser Erfindung 5- oder 6-gliedrige, gegebenenfalls mit einem ankondensierten Arylsystem versehene, aromatische Verbindungen, die ein oder zwei Heteroatome aus der Gruppe von Stickstoff, Sauerstoff und/oder Schwefel enthalten. In dieser Gruppe seien beispielhaft Furan, Thiophen, Pyrrol, Pyridin, Pyrimidin, Chinolin, Isochinolin, Phthalazin oder Chinazolin aufgeführt.

Die erfindungsgemäßen Verbindungen können als reine Enantiomere oder nichtracemische Enantiomerengemische, Racemate, Diastereomere oder Diastereomerengemische sowohl in Form ihrer freien Basen als auch von Salzen mit physiologisch verträglichen organischen oder anorganischen Säuren vorliegen.

Ein weiterer Gegenstand der vorliegenden Erfindung sind Verfahren zur Herstellung der erfindungsgemäßen Verbindungen der Formel (I)

Gegenstand der Erfindung ist dementsprechend ein Verfahren zur Herstellung von Verbindungen der Formel (I), in denen X¹ und X² Sauerstoff bedeuten, wobei man 2-Aminobenzylamine der allgemeinen Formel (II), 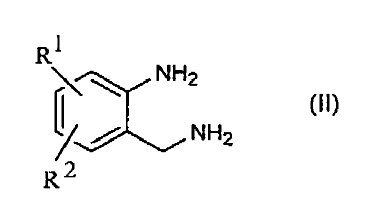 in der R¹ und R² wie oben definiert sind, zunächst mit Pyrrol-2,5-dionen der allgemeinen Formel (III), 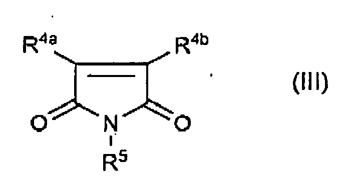 in der R^{4a} und R^{4b} wie oben definiert sind, zu Aminen der allgemeinen Formel (IVa) umsetzt. 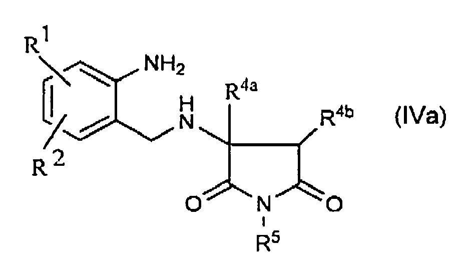 Anstelle der Pyrrol-2,5-dione können auch 3-Brom-pyrrolidin-2,5-dione der allgemeinen Formel (IIIa) 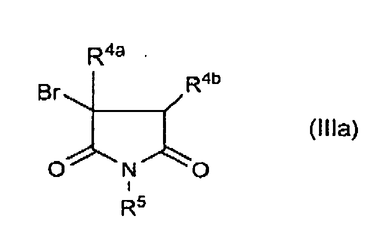 eingesetzt werden.

Verbindungen der allgemeinen Formel (IVb), in denen mindestens ein O aus Formel (IVa) durch S ersetzt wurde 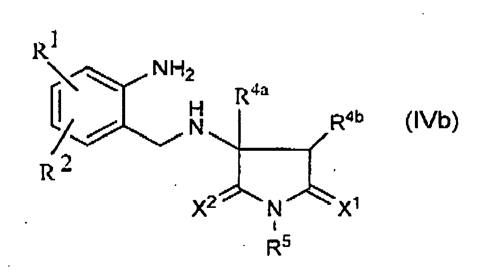 können, vorzugsweise nach Schutz der beiden Stickstoffatome, die nicht mit R⁵ verbunden sind, besonders bevorzugt nach Schutz mit Benzyloxycarbonylgruppen, mit dem Fachmann bekannten Thionierungsreagenzien, aus (IVa) durch Austausch mindestens eines O durch X¹ bzw. X² gleich S und nachfolgender Entschützung erhalten werden. Vorzugsweise erfolgt diese Schwefelung mit Tetraphosphordecasulfid, besonders bevorzugt in einem inerten Lösungsmittel unter Anwendung von Ultraschall bei Temperaturen von weniger als 40°C. Bei Vorhandensein von Benzyloxycarbonyl-Schutzgruppen kann die Entschützung vorzugsweise mit Bromwasserstoff in Essigsäure vorgenommen werden, wobei in diesem Falle die Hydrobromide anfallen.

Durch Reaktion von (IVa) oder (IVb) mit Verbindungen der allgemeinen Formel (V),

R³- C(OR^{y})₃ (V)

in der R³ Wasserstoff oder die Methylgruppe bedeutet und R^{y} für einen geradkettigen oder verzweigten C₁-C₄-Alkylrest steht, oder mit Amidinsalzen der allgemeinen Formel (VI), 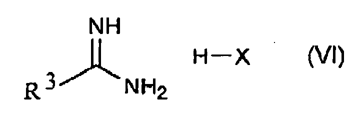 in der R³ wie oben definiert ist und X das Anion einer geeigneten Säure, vorzugsweise Essigsäure, darstellt, werden dann sowohl aus (IVa) als auch aus (IVb) Verbindungen der allgemeinen Formel (I) mit der entsprechenden Bedeutung von R³ erhalten.

Alternativ können hierfür auch Carbonsäureester der allgemeinen Formel (Vb) eingesetzt werden.

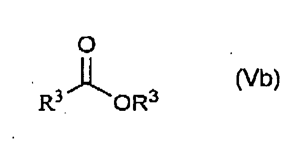

Erfindungsgemäß herzustellende Verbindungen der allgemeinen Formel (I), in der R³ zusammen mit dem C-Atom ein geminal substituiertes C-Atom darstellt, werden durch Reaktion von (IVa) oder (IVb) mit Ketonen der allgemeinen Formel (Vc), 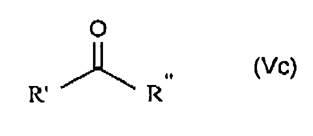 in der R' und R" unabhängig voneinander für einen Alkyl-, Aryl- oder Heteroarylrest stehen, erhalten.

Erfindungsgemäß herzustellende Verbindungen der allgemeinen Formel (I), in der R³ zusammen mit dem C-Atom eine Carbonylgruppe darstellt, werden durch Reaktion von (IVa) oder (IVb) mit C1-Bausteinen der allgemeinen Formel (VII), 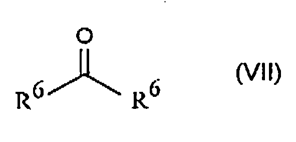 in der R⁶ für Cl, den Imidazol-1-yl-rest, eine C₁-C₄-Alkoxy-, eine Phenyloxy- oder eine mit der Nitrogruppe, Chlor oder Fluor substituierte Phenyloxygruppe oder eine Thiomethylgruppe steht, oder durch Reaktion von (IVa) oder (IVb) mit C₁-C₄-Alkyl-, Phenyl- oder substituierten Phenylestem, vorzugsweise 4-Nitro-, 4-Chlor-oder 4-Fluorphenylestern, der Chlorameisensäure erhalten.

Erfindungsgemäß herzustellende Verbindungen der allgemeinen Formel (I), in der R³ zusammen mit dem C-Atom eine Carbonylgruppe darstellt, werden auch durch Reaktion von (IVa) oder (IVb) mit einem C1-Baustein der allgemeinen Formel VIII,

C(OR⁷)₄ (VIII)

in der R⁷ für den Methyl- oder Ethylrest steht, erhalten.

Bevorzugt erfolgt bei dem erfindungsgemäßen Verfahren die Umsetzung der Aminobenzylamine der allgemeinen Formel (II) mit den Pyrrol-2,5-dionen der allgemeinen Formel (III) in inerten Lösungsmitteln, vorzugsweise Essigsäureethylester, bei Raumtemperatur.

Ebenfalls bevorzugt wird die Reaktion der Verbindungen der allgemeinen Formel (IVa und IVb) mit den Verbindungen der allgemeinen Formel (V) entweder ohne Lösungsmittel oder in einer organischen Carbonsäure, vorzugsweise Essigsäure, in einem Temperaturbereich von 10 bis 150 °C durchgeführt.

Die erfindungsgemäß hergestellten Verbindungen können als reine Enantiomere oder nichtracemische Enantiomerengemische, Racemate, Diastereomere, oder Diastereomerengemische sowohl in Form ihrer freien Basen als auch von Salzen mit physiologisch verträglichen organischen oder anorganischen Säuren erhalten werden.

Die Verbindungen der Formel (I) werden auch dadurch erhalten, dass man zunächst eine Aminoverbindung der allgemeinen Formel (IX) 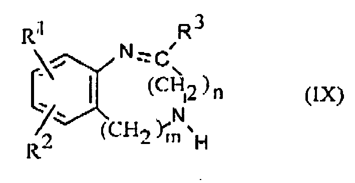 mit einem Pyrrol-2,5-dion der allgemeinen Formel (III) oder einem 3-Brompyrrolidin-2,5-dionderivat der allgemeinen Formel (IIIa) 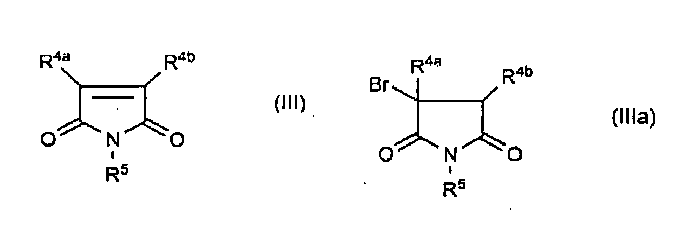 zu einer Verbindung der allgemeinen Formel (Ia) alkyliert, 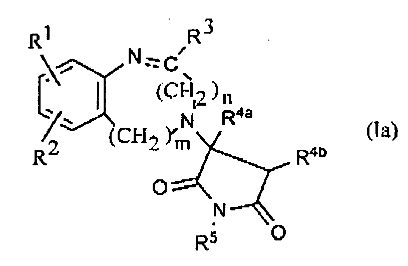 wobei in den Verbindungen (Ia), (II) und (III) die Reste R¹ bis R^{4b} wie oben definiert sind, und anschließend, wenn R⁵ nicht für Wasserstoff stehen soll, diesen Rest durch Umsetzung mit Formaldehyd, gegebenenfalls zusammen mit einem Amin der allgemeinen Formel HNR⁶R⁷, in der R⁶ und R⁷ wie oben definiert sind, einführt, sowie ggf. für X¹ und/oder X² gleich S das oben beschriebene Schwefelungsverfahren anwendet.

Steht in der Verbindung der Formel (Ia) der Rest R^{4a} für Wasserstoff, so lässt sich dieser durch an sich bekannte Alkylierungs- oder Halogenierungsreaktionen gegen die übrigen definitionsgemäßen R^{4a}-Substituenten austauschen.

Stehen in der Verbindung der Formel (Ia) R¹ und/oder R² für eine Nitrogruppe, so lassen sich daraus in an sich bekannter Weise, z.B. durch Reduktion mit katalytisch angeregtem Wasserstoff, Verbindungen (Ia) herstellen, in denen R¹ und/oder R² die Aminogruppe bedeuten.

Stehen in der Verbindung der Formel (Ia) R¹ und/oder R² für eine Aminogruppe, so lassen sich daraus in an sich bekannter Weise durch Acylierungsreaktionen R¹ und/oder R² gleich Acylaminogruppe einführen.

Stehen in der Verbindung der Formel (Ia) R¹ und/oder R² für eine benzylgeschützte Gruppe, z.B. Benzyloxy, so lassen sich daraus in an sich bekannter Weise, z.B. durch Reduktion mit katalytisch angeregtem Wasserstoff, Verbindungen (Ia) herstellen, in denen R¹ und/oder R² die entschützte Gruppe bedeuten, z.B. OH.

Verbindungen der Formel (I) werden auch dadurch erhalten, dass man eine Aminoverbindung der Formel (II) zunächst mit einem 3-Brompyrrolidin-2-onderivat der Formel (X) 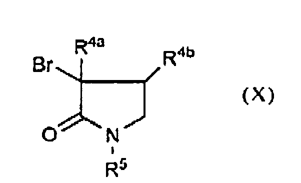 zu einer Verbindung der allgemeinen Formel (Ib) alkyliert, 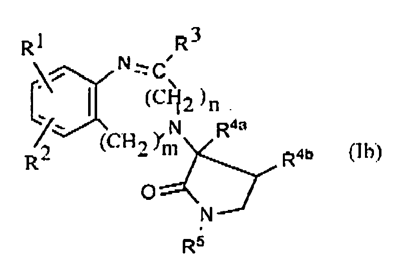 diese, vorzugsweise mit m-Chlor-perbenzoesäure oder Ruthenium (IV)-oxid/Natriumperiodat, zu einer Verbindung der oben genannten Formel (Ia) oxidiert und gegebenenfalls andere Reste R⁴ und/oder den Rest R⁵ einführt.

2-Aminobenzylamine der allgemeinen Formel (II) sind entweder kommerziell verfügbar oder werden nach Literaturangaben oder durch Reduktion entsprechender Anthranilsäureamide der allgemeinen Formel (XI) 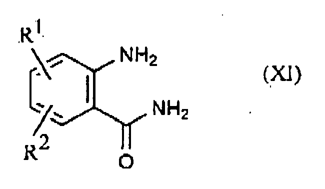 nach dem Fachmann bekannten Methoden hergestellt, z.B. durch Reduktion mit dem Boran-Dimethylsulfid-Komplex oder mit Lithiumaluminiumhydrid, in einem inerten Lösungsmittel, z.B. Tetrahydrofuran, mit oder ohne Erwärmung.

Anthranilsäureamide der allgemeinen Formel (XI) sind entweder kommerziell verfügbar oder werden nach Literaturangaben oder aus entsprechenden Anthranilsäuren (XII) 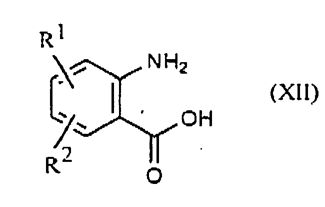 nach dem Fachmann bekannten Methoden hergestellt, z.B. durch Überführung in Anthranilsäure-cyanmethylester (XIII) 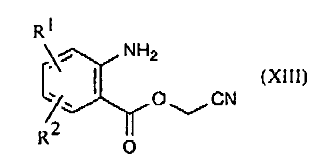 und nachfolgende Umsetzung mit Ammoniak, z.B. in Dioxan/WasserGemisch, ggf. unter Druck und bei erhöhter Temperatur.

Anthranilsäuren der allgemeinen Formel (XII) sind entweder kommerziell verfügbar oder werden nach Literaturangaben oder nach dem Fachmann bekannten Methoden hergestellt, z.B. durch Umsetzung entsprechender lsatine der allgemeinen Formel (XIV) 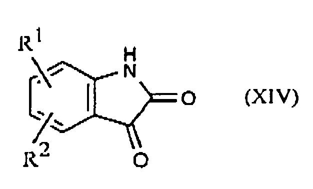 z.B. mit alkalischer Wasserstoffperoxidlösung, und anschließender Zugabe von Säure, z.B. von Ameisensäure.

lsatine der allgemeinen Formel (XIV) sind entweder kommerziell verfügbar oder werden nach Literaturangaben oder nach dem Fachmann bekannten Methoden hergestellt, z.B. durch Umsetzung entsprechender Aniline der allgemeinen Formel (XV) 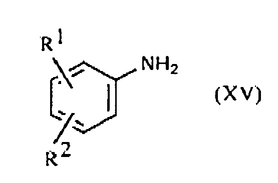 mit Trichloracetaldehyd und Hydroxylamin-Hydrochlorid zu entsprechenden 2-Hydroxyimino-*N*-aryl-acetamiden (XVI) 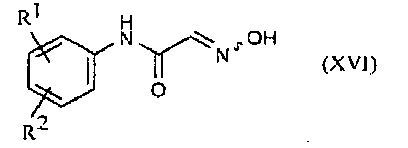 und anschließender saurer Cyclisierung. Im Falle unsymmetrisch substituierter Aniline kann es hierbei zur Bildung regioisomerer Isatine kommen. Eine Auftrennung in die Isomere kann entweder auf dieser Stufe oder in den nachfolgenden Synthesen durch Anwendung von dem Fachmann bekannten Reinigungsmethoden, z.B. Kristallisation oder Chromatographie, erfolgen.

Aniline der allgemeinen Formel (XV) sind entweder kommerziell verfügbar oder werden nach Literaturangaben oder aus entsprechenden Carbonsäuren (XVII) 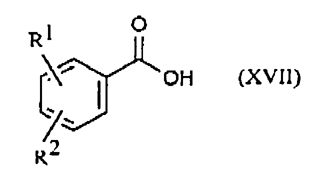 hergestellt, z.B. durch Reaktionen, die nach Art einer Curtius-Umlagerung verlaufen. Vorteilhaft setzt man dabei Reagenzien wie z.B. Azidophosphorsäurediphenylester in Gegenwart von tert.-Butanol und einer

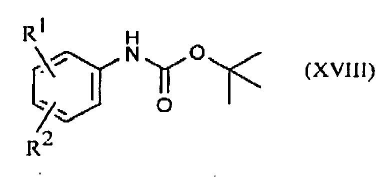

Base ein, z.B. Ethyldiisopropylamin, in einem inerten Lösungsmittel, z.B. Toluol, unter Erhitzen. Die im Falle der Verwendung von tert.-Butanol entstehenden 2-tert.-Butoxycarbonyl-aniline (XVIII)
können nach dem Fachmann bekannten Methoden in die Aniline (XV) oder deren Salze übergeführt werden, z.B. durch Behandlung mit Chlorwasserstoff in Dioxan, ggf. unter Zusatz polarer Lösungsmittel wie z.B. Methanol.

Ein weiterer Weg zur Herstellung der 2-Aminobenzylamine der allgemeinen Formel (II) ist die Reduktion von entsprechenden 2-Aminobenzonitrilen der allgemeinen Formel (XIX) 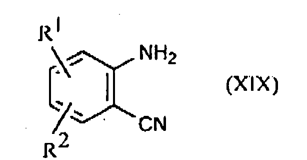 nach dem Fachmann bekannten Methoden, z.B. durch Reduktion mit dem Boran-Dimethylsulfid-Komplex oder mit Lithiumaluminiumhydrid, in einem inerten Lösungsmittel, z.B. Tetrahydrofuran, mit oder ohne Erwärmung.

2-Aminobenzonitrile der allgemeinen Formel (XIX) sind entweder kommerziell verfügbar oder werden nach Literaturangaben oder aus entsprechenden Nitrobenzonitrilen der allgemeinen Formel (XX) 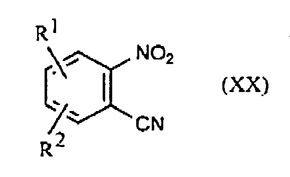 nach dem Fachmann bekannten Methoden hergestellt, z.B. durch Reduktion mit Wasserstoff in Gegenwart eines Katalysators, z.B. Palladium auf Aktivkohle, in z.B. Methanol, oder mit einem Metall in einer geeigneten Oxidationstufe, z.B. elementarem Eisen, in z.B. Essigsäure, oder mit Zinn(II)chlorid in einem protischen Lösungsmittel.

Vorteilhaft kann die Umwandlung von Nitrobenzonitrilen der allgemeinen Formel (XX) in Anthranilsäureamide der allgemeinen Formel (XI) in einem Schritt durch Verwendung von Hydrazin in Gegenwart von Raney-Nickel erfolgen.

Ein weiterer Weg zur Herstellung der 2-Aminobenzylamine der allgemeinen Formel (II) ist die aromatische Substitution geeigneter aktivierender Reste, z.B. die Substitution einer Nitrogruppe, in Dinitrobenzonitrilen der allgemeinen Formel (XXI), 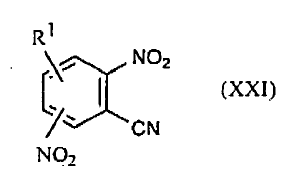 z.B. durch Alkoxide oder Thiolate, unter Gerinnung von Alkoxy- bzw. Alkylthio-2-nitrobenzonitrilen der allgemeinen Formeln (XXIIa) bzw. (XXIIb). 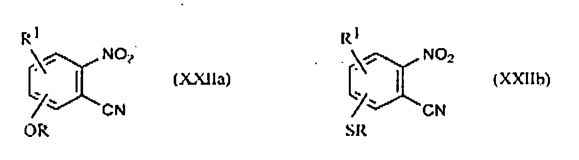 Alkylthio-2-nitrobenzonitrile der allgemeinen Formel (XXIIb) können mit dem Fachmann geläufigen Oxidationsmittel, z.B. Natriummetaperiodat, im Falle von n = 2 z. B. unter Zusatz von Chrom(VI)oxid, in z.B. Acetonitril, in Verbindungen der allgemeinen Formel (XXIIc) überführt werden: 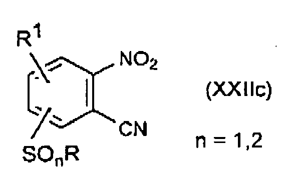 Verbindungen der allgemeinen Formeln (XXIIa) bis (XXIIc) können in der oben für 2-Nitrobenzonitrile beschriebenen Weise in 2-Aminobenzylamine der allgemeinen Formel (II) übergeführt werden.

Bevorzugte Verbindungen der Formel (I), in denen m = 1 und n = 0 bedeuten und R³ für H oder OH steht, können auch analog zu dem in WO 03/053956 A1 beschriebenen Verfahren hergestellt werden, indem ein Formamidderivat der allgemeinen Formel (XXIII), 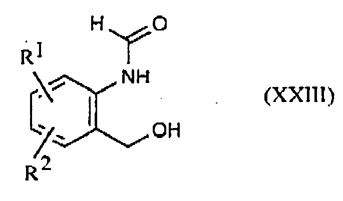 in der R¹ und R² wie oben definiert sind, und das durch selektive *N-*Formylierung des entsprechenden 2-Aminobenzylalkohols oder durch selektive O-Entformylierung des N,0-Bisformylderivats, zum Beispiel enzymatisch mit Hilfe von CAL-B, zugänglich ist, zunächst in an sich bekannter Weise, z.B. mit Pyridiniumdichromat, zu einem Benzaldehyd der allgemeinen Formel (XXIV) oxidiert wird.

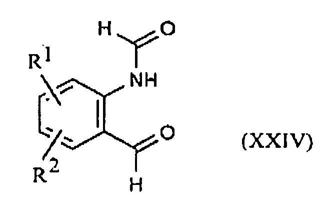

Benzaldehyde der allgemeinen Formel (XXIV) können durch reduktive Aminierung mit Asparagin oder entsprechenden Derivaten mit einem Rest R⁴ ungleich H unter Einsatz von komplexen Borhydriden, wie z.B. Natriumborhydrid, in Verbindungen der allgemeinen Formel (XXV) übergeführt werden.

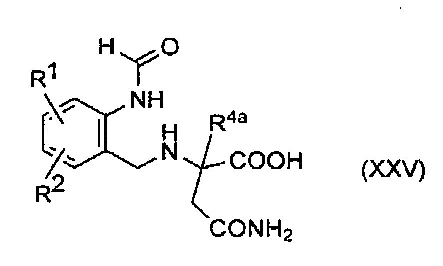

Diese Verbindungen der allgemeinen Formel (XXV) lassen sich dann, z.B. mit N,N'-Carbonyldiimidazol, zu Succinimiden der allgemeinen Formel (XXVI) cyclisieren, vorzugsweise nach vorherigem Schutz der Aminfunktion durch z.B. die Benzyloxycarbonylgruppe, die anschließend, z.B. mit Bromwasserstoff in Eisessig, wieder abgespalten wird.

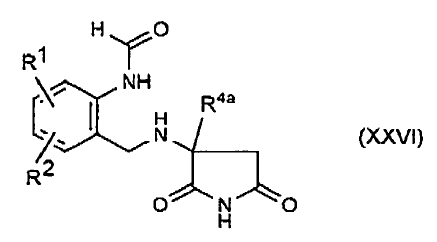

Aus den Verbindungen der allgemeinen Formel (XXVI) werden in protischen Lösungsmitteln wie z.B. Wasser unter Säurekatalyse schließlich Verbindungen der allgemeinen Formel (I) erhalten, in denen R¹, R² und R^{4a} wie oben definiert sind, m für 1, n für 0 steht, eine C=N-Doppelbindung vorliegt und R³ bzw. R⁵ ein Wasserstoffatom darstellen, das sich im Falle von R⁵ wie oben beschrieben gegen die übrigen definitionsgemäßen Substituenten austauschen lässt.

Wird nach reduktiver Aminierung der Verbindungen der allgemeinen Formel (XXIV) das Reaktionsgemisch mit Säuren behandelt, entstehen daraus Verbindungen der allgemeinen Formel (XXVII), in denen R¹, R² und R⁴ wie oben definiert sind, 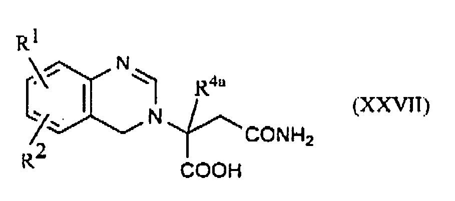 die sich durch Cyclisierung, z.B. mit Essigsäureanhydrid/Acetylchlorid, in Verbindungen der allgemeinen Formel (I), in denen m = 1 und n = 0 bedeuten, eine C=N-Doppelbindung vorliegt, R¹, R² und R⁴ wie oben definiert sind und R³ bzw. R⁵ für Wasserstoff stehen, überführen lassen. Danach werden gegebenenfalls andere definitionsgemäße Reste R⁴ und /oder R⁵ wie oben beschrieben eingeführt.

In analoger Weise lassen sich damit auch Verbindungen der Formel (I) erhalten, in denen m = 2 bedeutet und die übrigen Reste wie oben definiert sind.

Die erfindungsgemäßen Verbindungen besitzen immunmodulatorische Aktivität, sie induzieren eine Reduktion der IL-12 Produktion in LPS-aktivierten Monozyten bei gleichzeitiger Steigerung der 1L-10 Produktion. Aufgrund dieses Wirkprinzips haben diese Verbindungen ein großes therapeutisches Potential bei Krankheiten, wo eine überschießende IL-12 Produktion und ein relativer Mangel an IL-10 für die Pathogenese verantwortlich gemacht wird, das heißt, solche Verbindungen sind zur Behandlung und/oder Prophylaxe von Entzündungs- und Autoimmunerkrankungen zu nutzen. Aufgrund der anti-apoptotischen Wirkung von IL-12 sind die erfindungsgemäßen Verbindungen auch zur Suppression der Bildung von IL-12 bei hämatologisch-onkologischen Erkrankungen geeignet.

Dies unterscheidet die beanspruchten Verbindungen von bekannten Immunmodulatoren wie Corticosteroiden (z.B. Dexamethason), die sowohl die Synthese von IL-12 als auch die von IL-10 durch Monozyten supprimieren.

Überraschenderweise zeigen die erfindungsgemäßen in 3-Position heterocyclisch substituierte Pyrrolidin(thi)one der allgemeinen Formel (I) gegenüber dem Thalidomid-Analogon, in dem das Glutarimid durch ein Succinimid ersetzt wurde (XXVIII), eine gute Wirksamkeit (Vergleichsbeispiel 1), die z. T. der Wirksamkeit des Thalidomids vergleichbar ist.

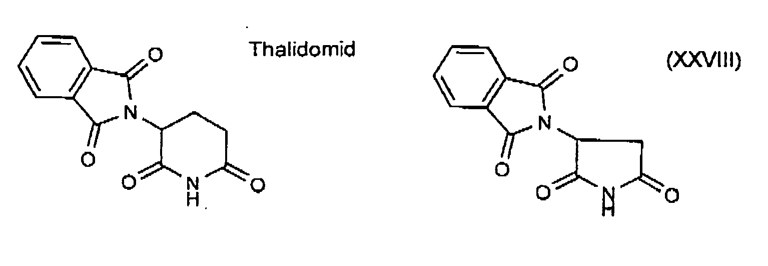

Ferner bewirken intravenöse oder orale Applikationen von bestimmten in 3-Position herterocyclisch substitutierten Pyrrolidonen der allgemeinen Formel (I) überraschenderweise im Vergleich zur Applikation gleicher Dosen analoger Piperidin-2,6-dione hohe Plasmakonzentrationen. Diese sind auf eine starke Verringerung der Verteilungsvolumina gegenüber Thalidomid zurückzuführen. Da sich alle Zielzellen entweder im Blut befinden oder intensiv mit Blut versorgt werden, bedeuten erhöhte Plasmakonzentrationen verbesserte klinische Behandlungsmöglichkeiten.

Die bereits in WO 03/053956 A1 beschriebenen Vorteile gegenüber Thalidomid zeigen sich auch bei den hier beschriebenen neuen Verbindungen, nämlich eine gute Wasserlöslichkeit sowie eine geringere Hydrolyseempfindlichkeit, als auch verbesserte pharmakokinetische Eigenschaften.

Zu den Erkrankungen oben genannter Formenkreise zählen unter anderem Entzündungen der Haut (z.B. atopische Dermatitis, Psoriasis, Ekzeme, Erythema nodosum leprosum), Entzündungen der Atemwege (z.B. Bronchitis, Pneumonie, Asthma bronchiale, ARDS (adult respiratory distress syndrome), Sarkoidose, Silikose/Fibrose), Entzündungen des Gastrointestinaltraktes (z.B. gastroduodenale Ulcera, Morbus Crohn, ulcerative Colitis), ferner Erkrankungen wie Hepatitis, Pankreatitis. Appendizitis, Peritonitis, Nephritis, Aphthosis, Konjunktivitis, Keratitis, Uveitis, Rhinitis.
Die Autoimmunerkrankungen umfassen z.B. Erkrankungen des arthritischen Formenkreises (z.B. rheumatoide Arthritis, HLA-B27 assoziierte Erkrankungen, rheumatoide Spondylitis), ferner Multiple Sklerose, jugendlicher Diabetes oder Lupus erythematosus.
Weitere Indikationen sind Sepsis, septischer Schock, bakterielle Meningitis, mycobakterielle Infektionen, opportunistische Infektionen bei AIDS, Kachexie, Transplantat-Abstoßungs-Reaktionen, Graft-versus-Host Reaktionen sowie chronisches Herzversagen, Herzinsuffizienz, das Reperfusions-Syndrom sowie Atherosklerose.
Darüber hinaus zählen auch chronische Schmerzzustände, Fibromyalgie. Morbus Sudeck (reflex sympathetic dystrophy (RSD)) zu den Indikationen.

Ferner gehören hämatologische Erkrankungen wie multiples Myelom, Myelodisplastisches Syndrom und Leukämien sowie weitere onkologische Erkrankungen wie z.B. Glioblastom, Prostata-, Nierenzell-, Mamma-, Schilddrüsen-, Kopf- und Hals-, Pancreas- und colorectales Carcinom sowie Melanom und Kaposi's Sarcom zu den Krankheitsbildern, bei denen die beschriebenen Immunmodulatoren einzusetzen sind.

Erfindungsgemäße Arzneimittel enthalten neben mindestens einer Verbindung der allgemeinen Formel I Trägermaterialien, Füllstoffe, Lösungsmittel, Verdünnungsmittel. Farbstoffe und/oder Bindemittel. Die Auswahl der Hilfsstoffe sowie die einzusetzenden Mengen hängen davon ab, ob das Arzneimittel oral, rektal, ophthalmisch (intravitreal, intracameral), nasal, topisch (einschließlich buccal und sublingual), vaginal oder parenteral (einschließlich subkutan, intramuskulär, intravenös, intradermal, intratracheal und epidural) verabreicht werden soll.

Für die orale Applikation eignen sich Zubereitungen in Form von Tabletten, Kautabletten, Dragees, Kapseln, Granulaten, Tropfen, Säften oder Sirupen, für die parentale, topische und inhalative Applikation Lösungen, Suspensionen, leicht rekonstituierbare Trockenzubereitungen sowie Sprays. Kutane Applikationsformen sind Salben, Gele, Cremes und Pasten. Ophthalmische Applikationsformen umfassen Tropfen, Salben und Gele. Erfindungsgemäße Verbindungen in einem Depot in gelöster Form, einer Trägerfolie oder einem Pflaster, gegebenenfalls unter Zusatz von die Hautpenetration fördemden Mitteln, sind Beispiele für geeignete perkutane Applikationsformen. Aus oral oder perkutan anwendbaren Zubereitungsformen können die erfindungsgemäßen Verbindungen verzögert freigesetzt werden.

Die an Patienten zu verabreichende Wirkstoffmenge variiert in Abhängigkeit vom Gewicht des Patienten, von der Applikationsart, der Indikation und dem Schweregrad der Erkrankung.

### Beispiele

Die folgenden Beispiele dienen zur näheren Erläuterung der vorliegenden Erfindung.

Als stationäre Phase für die chromatographischen Trennungen wurde Kieselgel 60 (0,040 bis 0.063 mm) der Firma E. Merck. Darmstadt, eingesetzt. Die Mischungsverhältnisse der Elutionsmittel sind stets in Volumen/Volumen angegeben.

Die Substanzen wurden über ihren Schmelzpunkt, das ¹H-NMR- und/oder das ¹³C-NMR-Spektrum charakterisiert. Die Aufnahme der Spektren erfolgte bei 300 MHz mit dem Gerät Gemini 300 der Firma Varian. Die chemischen Verschiebungen sind in ppm angegeben (8-Skala). Als interner Standard wurde Tetramethylsilan (TMS) verwendet.

Die Beispielverbindungen können ggf. wechselnde geringe Rückstände an Essigsäure enthalten, die in der Regel zwischen 0 und 3 Moläquivalenten Essigsäure, bezogen auf die jeweilige Titelverbindung, entsprechen.

### Vergleichsbeispiel 1

### 2-(2,5-Dioxo-pyrrolidin-3-yl)-isoindol-1.3-dion

Die Herstellung der Titelverbindung kann nach Literatur (D. Misiti et al. J. Med. Chem. 1963, 6 464) erfolgen.

Siehe auch Patentschrift GB 1185273.

### Beispiel 1

### 3-(5-Chlor-7-fluor-4H-chinazolin-3-yl)-pyrrolidin-2,5-dion

### Stufe 1:

### (3-Chlor-5-fluorphenyl)-carbaminsäure-tert-butyl ester

Eine Lösung von 3-Chlor-5-fluorbenzoesäure (2,44 g, 14,0 mmol), Diisopropylethylamin (2,8 mL, 2,20 g, 17 mmol) und Azidophosphorsäurediphenylester (3,7 mL, 4,70 g. 17 mmol) in Toluol (10 mL) und tert-Butanol (10 mL) wurde 16 h unter Rückfluss erhitzt. Das Reaktionsgemisch wurde anschließend im Vakuum eingeengt. Der Rückstand wurde in Wasser (20 mL) aufgenommen und mit Essigsäureethylester / Cyclohexan 1:4 (4 x 10 mL) extrahiert. Die vereinigten organischen Phasen wurden mit Natriumchlorid-Lösung gewaschen, mit Magnesiumsulfat getrocknet und eingeengt. Das Rohprodukt (4,1 g) wurde durch Flashchromatographie mit Essigsäureethylester / Cyclohexan (1:6) gereinigt.
Ausbeute: 2,79 g (81 %), weißer Feststoff.
Schmelzpunkt: 55-58 °C

### Stufe 2:

### 3-Chlor-5-fluoranilin Hydrochlorid

Zum Produkt aus Stufe 1 (27,2 g, 0,11 mmol) in einem Gemisch aus 1,4-Dioxan (40 mL) und Methanol (80 mL) wurde eine 30%ige Lösung von Chlorwasserstoff in 1,4-Dioxan (45 mL) gegeben und 20 h bei Raumtemperatur gerührt. Das Reaktionsgemisch wurde im Vakuum aufkonzentriert und mit Diethylether (200 mL) versetzt. Der ausgefallene Niederschlag wurde abfiltriert, mit Diethylether gewaschen und im Vakuum getrocknet.
Ausbeute: 18,2 g (91 %), weißer Feststoff.
Schmelzpunkt: 156-159 °C

### Stufe 3:

### N-(3-Chlor-5-fluorphenyl)-2-hydroxyimino-acetamid

Zu einer Lösung von Chloralhydrat (17,4 g, 105 mmol) und Natriumsulfat (113 g) in Wasser (380 mL) wurde eine Suspension des Produkts aus Stufe 2 (17,9 g, 98 mmol) in Wasser (10 mL) gegeben. Diese Mischung wurde mit einer Lösung von Hydroxylamin-Hydrochlorid (21,7 g, 313 mmol) in Wasser (100 mL) versetzt. Das Reaktionsgemisch wurde 40 min unter Rückfluss erhitzt. Anschließend wurde noch 16 h bei Raumtemperatur gerührt, das ausgefallene Produkt abfiltriert, mit Wasser gewaschen und im Vakuum über Phosphorpentoxid getrocknet.
Ausbeute: 20,7 g (97 %), weißer Feststoff.
Schmelzpunkt: 179 °C

### Stufe 4:

### 4-Chlor-6-fluor-1H-indol-2,3-dion (Hauptisomer) und 6-Chlor-4-fluor-1H-indol-2,3-dion (Minderisomer)

Das Produkt aus Stufe 3 (20,6 g. 95 mmol) wurde innerhalb von 15 min zu 50-60 °C warmer konzentrierter Schwefelsäure (104 mL) gegeben. Die Mischung wurde anschließend auf 100 °C erwärmt, 30 min gerührt und nach dem Abkühlen auf Raumtemperatur auf Eis (1 kg) gegossen. Der ausgefallene Feststoff wurde abfiltriert, mit Wasser neutral gewaschen und im Vakuum über Phosphorpentoxid getrocknet.
Ausbeute: 15,5 g (82 %) des Gemisches der Regioisomeren im Verhältnis 9:1, gelber Feststoff.
Hauptisomer:
¹H-NMR (DMSO-d₆): 6.72 (0.9 H, dd, J = 8.8 und 2.0 Hz); 6.77 (0.1 H, d, J = 2.0 Hz); 7.05 (0.9 H, dd, J = 9.2 und 2.0 Hz); 7.08 (0.1 H, dd, J = 9.2 und 4.0 Hz); 11.36 (1 H, s).

### Stufe 5:

### 2-Amino-6-chlor-4-fluor-benzoesäure (Hauptisomer) und 2-Amino-4-chlor-6-fluor-benzoesäure (Minderisomer)

Zu einer Suspension des Produktgemisches aus Stufe 4 (8,00 g, 40 mmol) in Wasser (200 mL) wurde bei 0 °C zuerst Natriumhydroxid (10,7 g, 267 mmol) und dann 30 % Wasserstoffperoxid (10.7 mL) in Wasser (96 mL) gegeben. Die Mischung wurde über Nacht bei Raumtemperatur gerührt und dann mit Ameisensäure (ca. 20 mL, starkes Schäumen) auf pH 3,3 gebracht. Die ausgefallene Titelverbindung wurde abfiltriert und im Vakuum über Phosphorpentoxid getrocknet. Die wässrige Lösung wurde mit Essigsäureethylester (2 x 150 mL) extrahiert. Der pH der wässrigen Phase wurde auf 3,3 korrigiert und erneut mit Essigsäureethylester (150 mL) extrahiert. Die vereinigten organischen Phasen wurden mit Natriumsulfat getrocknet, eingeengt und der Rückstand im Vakuum getrocknet. Ausbeute: 7,30 g (95 %) des Gemisches der Regioisomeren im Verhältnis 9:1, brauner Feststoff.

Hauptisomer:
¹³C-NMR (DMSO-d₆): 100.2 (d, J = 24 Hz); 104.5 (d, J = 26 Hz); 112.0; 133.7 (d, J = 15 Hz); 151.0 (d, J = 13 Hz); 162.9 (d, J = 244 Hz); 167.1 .

### Stufe 6:

### 2-Amino-6-chlor-4-fluor-benzoesäurecyanmethylester (Hauptisomer) und 2-Amino-4-chlor-6-fluor-benzoesäurecyanmethylester (Minderisomer)

Eine Lösung des Produktgemisches aus Stufe 5 (7,00 g, 37 mmol) in Aceton (128 mL) wurde mit Triethylamin (7,6 mL, 55 mmol) und mit einer Lösung von Chloracetonitril (3,9 mL, 60 mmol) in Aceton (25 mL) versetzt und 76 h bei Raumtemperatur gerührt. Anschließend wurde filtriert und das Filtrat im Vakuum eingeengt. Der Rückstand (12,8 g) wurde in Essigsäureethylester gelöst, die Lösung mit Wasser gewaschen, die organische Phase mit Natriumsulfat getrocknet und im Vakuum eingeengt. Ausbeute: 6,00 g (70 %) des Gemisches der Regioisomeren im Verhältnis 9:1, braunes Öl.

Hauptisomer :
¹H-NMR (DMSO-d₆): 5.17(2 H, s); 6.37 (2 H, s); 6.40-6.60 (2 H, m).

### Stufe 7:

### 2-Amino-6-chlor-4-fluor-benzamid (Hauptisomer) und 2-Amino-4-chlor-6-fluor-benzamid (Regioisomer)

Eine Lösung des rohen Produktgemisches aus Stufe 6 (1,80 g) in 1,4-Dioxan (3 mL) wurde mit 25 % wässrigem Ammoniak (7 mL) versetzt und im Stahlautoklaven über Nacht bei 100 °C gerührt. Nach Abkühlen der Reaktionslösung wurde diese im Vakuum eingeengt. Das Rohprodukt (1 g) wurde durch Flashchromatographie mit Essigsäureethylester / Cyclohexan (1:1) gereinigt.

Minderisomer:
Ausbeute: 55 mg (5 %), gelber Feststoff
Schmelzpunkt: 142-145 °C (Zersetzung)
¹H-NMR (DMSO-d₆): 6.41 (2 H, s); 6.45 (1 H, dd, J = 10.8 und 2.0 Hz); 6.59 (1 H, t, J = 2.0 Hz); 7.55 (1 H, s); 7.59 (1 H, s).
13C-NMR (DMSO-d₆): 102.0 (d, J = 28 Hz); 104.8 (d, J = 18 Hz); 110.7; 135.0 (d, J = 16 Hz); 151.7 ( d, J = 8 Hz); 160.8 (d, J = 245 Hz); 165.7.

Hauptisomer:
Ausbeute: 500 mg (47 %), weißer Feststoff.
Schmelzpunkt: 101-104 °C ¹H-NMR (DMSO-d₆); 5.56 (2 H, m); 6.40-6.50 (2 H, m); 7.60 (1 H, s); 7.82 (1 H. s).
¹³C-NMR (DMSO-d₆): 99.6 (d, J = 23 Hz); 103.1 (d, J = 26 Hz); 118.3; 131.1 (d, J = 14 Hz); 148.7 (d, J =13dz): 162.1 (d. J = 244 Hz); 166.9.

### Stufe 8:

### 2-Aminomethyl-3-chlor-5-fluor-phenylamin

Eine Lösung des Hauptisomers aus Stufe 7 (4,8 g, 25 mmol) in wasserfreiem Tetrahydrofuran (85 mL) wurde mit 2 M Boran-Dimethylsulfid-Komplex (40 mL, 80 mmol) in Tetrahydrofuran versetzt und 6 h unter Rückfluss erhitzt. Die abgekühlte Reaktionslösung wurde vorsichtig mit Wasser (6.5 mL) versetzt und dann im Vakuum eingeengt. Der Rückstand wurde wiederholt (3 x) in Methanol gelöst und jeweils wieder eingeengt. Das Rohprodukt (9 g) wurde durch Flashchromatographie mit Chloroform / Methanol (4:1) und 1 % Triethylamin gereinigt.
Ausbeute: 2,97 g (68 %), gelblicher Feststoff.
Schmelzpunkt: 71-74 °C

### Stufe 9:

### 3-(2-Amino-6-chlor-4-fluor-benzylamino)-pyrrolidin-2,5-dion

Eine Lösung des Produkts aus Stufe 8 (600 mg, 3.4 mmol) und Maleinimid (500 mg, 5.1 mmol) in Essigsäureethylester (9 mL) wurde bei Raumtemperatur 22 h gerührt. Das ausgefallene Produkt wurde abfiltriert und im Vakuum getrocknet.
Ausbeute: 820 mg (88 %), weißer Feststoff.
Schmelzpunkt: 143-144 °C

### Stufe 10:

### 3-(5-Chlor-7-fluor-4H-chinazolin-3-yl)-pyrrolidin-2,5-dion

Eine Lösung des Produkt aus Stufe 9 (308 mg, 1.1 mmol) und Orthoartieisensäuretriethylester (326 mg, 361 µL, 2,2 mmol) in Essigsäure (10 mL) wurde 6 h bei Raumtemperatur gerührt. Das Reaktionsgemisch wurde im Vakuum eingeengt, der Rückstand mit Toluol versetzt und erneut eingeengt.
Ausbeute: 400 mg (100%) der Titelverbindung, weißer Feststoff, der 1 Moläquivalent Essigsäure enthält.
Schmelzpunkt: 203-204 °C

### Beispiel 2

### 3-(7-Chlor-5-fluor-4H-chinazolin-3-yl)-pyrrolidin-2,5-dion

Bei Ersatz des in Beispiel 1, Stufe 8, verwendeten Hauptisomers durch das Minderisomer und Anwendung der in den Stufen 8 bis 10 beschriebenen Verfahrensweise erhielt man die Titelverbindung in Form eines weißen Feststoffs.
Schmelzpunkt: 179-181 °C

### Beispiel 3

### 3-(7-Fluor-4H-chinazolin-3-yl)-pyrrolidin-2,5-dion

### Stufe 1:

### 2-Amino-4-fluorbenzoesäurecyanmethylester

Eine Lösung von 2-Amino-4-fluorbenzoesäure (3,93 g, 25,3 mmol) in Aceton (90 mL) wurde zuerst mit Triethylamin (5,03 mL, 36,1 mmol), dann mit einer Lösung von Chloracetonitril (2,44 mL, 38,7 mmol) in Aceton (15 mL) versetzt und anschließend 2 d bei Raumtemperatur gerührt. Das Reaktionsgemisch wurde filtriert, der feste Rückstand mit Aceton gewaschen und das Filtrat im Vakuum eingeengt. Der Rückstand wurde in Ethylacetat gelöst und mit halbgesättigter Natriumchloridlösung gewaschen. Die organische Phase wurde mit Natriumsulfat getrocknet und im Vakuum eingeengt.
Ausbeute: 4,22 g (86 %), weißer Feststoff.
Schmelzpunkt: 69-70 °C

### Stufe 2 :

### 2-Amino-4-fluorbenzamid

Eine Lösung des Produkt aus Stufe 1 (3,50 g, 18 mmol) in 1,4-Dioxan (10 mL) wurde mit 33 % wässriger Ammoniaklösung (40 mL) versetzt und 20 h bei 100 °C Badtemperatur im verschlossenen 80 mL-Stahlautoklaven gerührt. Anschließend wurde die auf Raumtemperatur abgekühlte Reaktionslösung im Vakuum eingeengt und der Rückstand durch Flashchromatographie mit Chloroform / Methanol (95:5) gereinigt. Ausbeute: 2,45 g (88 %), weißer Feststoff.
Schmelzpunkt: 118-123 °C
¹H-NMR (DMSO-d₆): 6.27 (1 H, dt, J = 8.6 und 3.1 Hz); 6.43 (1 H, dd, J = 11.7 und 3.1 Hz); 6.87 (2 H, b s); 7.04 (1 H br s); 7.59 (1 H, dd J = 8.6 und 6.3 Hz); 7.67 (1 H br s).

### Stufe 3:

### 2-Aminomethyl-5-fluorphenylamin

Eine Lösung des Produkts aus Stufe 2 (2,45 g, 16 mmol) in wasserfreiem Tetrahydrofuran (50 mL) wurde mit 2 M Boran-Dimethylsulfid-Komplex (40 mL, 80 mmol) in Tetrahydrofuran versetzt und 9 h unter Rückfluss erhitzt. Die abgekühlte Reaktionslösung wurde vorsichtig mit Wasser (4 mL) versetzt und dann im Vakuum eingeengt. Der Rückstand wurde mit Toluol (2×) versetzt und jeweils wieder eingeengt. Anschließend wurde der Rückstand wiederholt in Methanol (3×) gelöst und jeweils eingeengt. Das Rohprodukt wurde durch Flashchromatographie mit Chloroform / Methanol (7:3) und 1 % Triethylamin gereinigt.
Ausbeute: 1,37 g (61 %), weißer Feststoff.
Schmelzpunkt: 49-51 °C

### Stufe 4:

### 3-(2-Amino-4-tluorbenzylamino)-pyrrolidin-2,5-dion

Eine Lösung des Produkts aus Stufe 3 (280 mg, 2 mmol) und Maleinimid (194 mg, 2 mmol) in Essigsäureethylester (5 mL) wurde 20 h bei Raumtemperatur gerührt. Anschließend wurde 3 h bei 50 °C gerührt. Da die Reaktion nicht vollständig war, wurde weiteres Maleinimid (49 mg, 0.5 mmol) zugesetzt und erneut 18 h bei 50 °C gerührt. Das Reaktionsgemisch wurde im Vakuum eingeengt und der Rückstand durch Flashchromatographie mit Chloroform / Methanol (95:5) gereinigt.
Ausbeute: 358 mg (75 %).

¹H-NMR (DMSO-d₆): 2.42 (1 H, dd, J = 17.6 und 5.9 Hz); 2.64 (1 H, br s); 2.75 (1 H, dd, J = 17.6 und 8.8 Hz); 3.57-3.74 (3 H, m); 5.47 (2 H, s); 6.24 (1 H, dt, J = 8.8 und 2.9 Hz); 6.37 (1 H, dd, J = 11.7 und 2.0 Hz); 6.97 (1 H. dd, J = 7.8 und 7.8 Hz); 11.13 (1 H, s).

### Stufe 5:

### 3-(7-Fluor-4H-chinazolin-3-yl)-pyrrolidin-2,5-dion

Eine Lösung des Produkts aus Stufe 4 (311 mg. 1,3 mmol) in Essigsäure (10 mL) und Orthoameisensäuretriethylester (428 µL, 2.6 mmol) wurde 24 h bei Raumtemperatur gerührt. Das Reaktionsgemisch wurde im Vakuum eingeengt, der Rückstand in Toluol aufgenommen, erneut eingeengt und durch Flashchromatographie mit Essigsäureethylester / Methanol (4:1) gereinigt.
Ausbeute: 243 mg (76 %), gelblicher Feststoff.
Schmelzpunkt: 191 °C

### Stufe 6:

### 3-(7-Fluor-4H-chinazolin-3-yl)-pyrrolidin-2,5-dion Hydrochlorid

Eine Lösung des Produktes aus Stufe 5 (230 mg, 0,93 mmol) in Butan-2-on (3 ml) wurde unter Rühren mit Wasser (0,02 ml) und anschließend mit Chlortrimethylsilan (0,12 ml) versetzt und für 30 Minuten bei Raumtemperatur gerührt. Der entstandene Feststoff wurde abgesaugt, mit Diethylether gewaschen und bei 70°C im Trockenschrank für 15 Stunden getrocknet.
Ausbeute: 258 mg (98 %), beigefarbener Feststoff.
Zersetzung ab 214° C

### Beispiel 4

### 3-(5,7-Difluor-4H-chinazolin-3-yl)-pyrrolidin-2,5-dion

### Stufe 1:

### N-(3,5-Difluorphenyl)-2-hydroxyiminoacetamid

Zu einer Lösung von Chloralhydrat (20,5 g. 124 mmol) und Natriumsulfat (133,8 g) in Wasser (450 mL) wurde eine Suspension von 3,5-Difluoranilin (15,0 g, 116 mmol) in konzentrierter Salzsäure (10 mL) gegeben. Diese Mischung wurde mit einer Lösung von Hydroxylamin-Hydrochlorid (25,7 g, 370 mmol) in Wasser (120 mL) versetzt. Die Mischung wurde 30 min unter Rückfluss erhitzt, wobei eine klare Lösung entstand, aus der dann Feststoff ausfiel. Anschließend wurde noch 3,5 h bei Raumtemperatur gerührt, das ausgefallene Produkt abfiltriert, mit viel Wasser gewaschen und im Exsikkator über Phosphorpentoxid getrocknet.
Ausbeute: 21,1 g (91 %), weißer Feststoff.
Schmelzpunkt: 190-192 °C

### Stufe 2:

### 4,6-Difluor-1H-indol-2,3-dion

Das Produkt aus Stufe 1 (21,1 g, 105 mmol) wurde innerhalb von 15 min zu 50-60 °C warmer konzentrierter Schwefelsäure (115 mL) gegeben. Die Mischung wurde anschließend auf 100 °C erwärmt, 30 min gerührt und nach dem Abkühlen auf Raumtemperatur langsam auf Eis (1,2 kg) gegossen. Der ausgefallene Feststoff wurde abfiltriert, mit Wasser neutral gewaschen und im Exsikkator über Phosphorpentoxid getrocknet. Ausbeute: 18,4 g (95 %), gelber Feststoff.

¹H-NMR (DMSO-d6): 6.60 (1 H, dd, J = 8.8 und 2.0 Hz); 6.84 (1 H, dt, J = 9.8 und 2.0 Hz); 11.20 (1 H, br s).

### Stufe 3:

### 2-Amino-4,6-difluorbenzoesäure

Zu einer Suspension des Produkts aus Stufe 2 (4,58 g, 25 mmol) in Wasser (125 mL) wurde bei 0 °C zuerst Natriumhydroxid (6,70 g, 167 mmol) und dann 30 % Wasserstoffperoxid (6.7 mL) in Wasser (60 mL) gegeben. Die Mischung wurde 20 h bei Raumtemperatur gerührt und dann mit Ameisensäure (ca. 11 mL, starkes Schäumen) auf pH 3 gebracht, wobei die Titelverbindung ausfiel. Anschließend wurde filtriert und im Exsikkator über Phosphorpentoxid getrocknet.
Ausbeute: 3,72 g (86 %), weißer Feststoff.
Schmelzpunkt: 198-202 °C

### Stufe 4:

### 2-Amino-4,6-difluorbenzoesäurecyanmethylester

Eine Lösung des Produkts aus Stufe 3 (1,00 g, 5,8 mmol) in Aceton (20 mL) wurde zuerst mit Triethylamin (580 mg, 1,2 mL, 8,7 mmol) und dann mit einer Lösung von Chloracetonitril (0,6 mL, 9,5 mmol) in Aceton (4 mL) versetzt und über Nacht bei Raumtemperatur gerührt. Anschließend wurde filtriert, das Filtrat im Vakuum eingeengt, der Rückstand in Essigsäureethylester gelöst, vom ungelösten Feststoff abfiltriert und erneut eingeengt. Der Rückstand wurde durch Flashchromatographie mit Cyclohexan / Essigsäureethylester (4:1) gereinigt.
Ausbeute: 1,01 g (82 %), weißer Feststoff.
Schmelzpunkt: 71-72 °C

### Stufe 5:

### 2-Amino-4,6-difluorbenzamid

Eine Lösung des Produkts aus Stufe 4 (6,88 g, 32,5 mmol) in 1,4-dioxan (10 mL) wurde mit 25 % wässrigem Ammoniak versetzt und im Stahlautoklaven übers Wochenende bei 100 °C gerührt. Nach Abkühlen der Reaktionslösung wurde im Vakuum eingeengt.
Ein zweiter Reaktionsansatz wurde parallel genauso durchgeführt. Die Rohprodukte wurden vereinigt und zusammen durch Flashchromatographie mit Chloroform / Methanol (95:5) gereinigt. Ausbeute: 9,04 g (81 %), weißer Feststoff.
Schmelzpunkt: 113-114 °C

### Stufe 6:

### 2-Aminomethyl-3,5-ditluorphenylamin

Eine Lösung des Produkts aus Stufe 5 (5,00 g, 29 mmol) in wasserfreiem Tetrahydrofuran (100 mL) wurde mit 2 M Boran-Dimethylsulfid-Komplex (46,5 mL, 93 mmol) in Tetrahydrofuran versetzt und 8 h unter Rückfluss erhitzt. Die abgekühlte Reaktionslösung wurde vorsichtig mit Wasser (7,5 mL) versetzt und dann im Vakuum eingeengt. Der Rückstand wurde mit Toluol (2x) versetzt und jeweils wieder eingeengt. Anschließend wurde der Rückstand wiederholt in Methanol (3x) gelöst und jeweils eingeengt. Das Rohprodukt wurde durch Flashchromatographie mit Chloroform / Methanol (7:3) und 1 % Triethylamin gereinigt.
Ausbeute: 3,16 g (69 %), bräunliches Öl.

¹H-NMR (DMSO-d₆): 1.73 (2 H, sehr br s); 3.65 (2 H, s); 5.87 (2 H; br s); 6.10-6.30 (2 H, m).

### Stufe 7:

### 3-(2-Amino-4,6-difluorbenzylamino)-pyrrolidin-2,5-dion

Eine Lösung des Produkts aus Stufe 6 (1,00 g, 6,32 mmol) und Maleinimid (776 mg, 8 mmol) in Essigsäureethylester (15 mL) wurde 70 h bei 50 °C gerührt. Anschließend wurde das ausgefallene Produkt abfiltriert und getrocknet.
Ausbeute: 1,20 g (75 %), farbloser Feststoff.
Schmelzpunkt: 178 °C

### Stufe 8:

### 3-(5,7-Difluor-4H-chinazolin-3-yl)-pyrrolidin-2,5-dion

Eine Lösung des Produkts aus Stufe 7 (300 mg, 1,18 mmol) in Essigsäure (10 mL) und Orthoameisensäuretriethylester (351 mg, 390 µL, 2,37 mmol) wurde 24 h bei Raumtemperatur gerührt. Da die Umsetzung noch nicht vollständig war, wurde weitere 3,5 h bei 50 °C gerührt, dann mit weiterem Orthoameisensäuretriethylester (390 µL) versetzt und 2 h bei Raumtemperatur gerührt. Das Reaktionsgemisch wurde im Vakuum eingeengt, der Rückstand in Toluol aufgenommen, das Gemisch erneut eingeengt und das Rohprodukt durch Flashchromatographie mit
Chloroform / Methanol (9:1) gereinigt.
Ausbeute: 247 mg (75 %), weißer Feststoff.
Schmelzpunkt: 198 °C

### Stufe 9:

### 3-(5,7-Difluor-4H-chinazolin-3-yl)-pyrrolidin-2,5-dion hydrochlorid

Eine Suspension des Produktes aus Stufe 8 (220 mg, 0,84 mmol) in Butan-2-on (3 ml) wurde unter Rühren mit Wasser (0,02 ml) und anschließend mit Chlortrimethylsilan (0,11 ml) versetzt und für 30 Minuten im Eisbad bei 0° C gerührt. Der entstandene Feststoff wurde abgesaugt, mit Diethylether gewaschen und bei 70°C im Trockenschrank für 15 Stunden getrocknet. Ausbeute: 245 mg (98 %), weißer Feststoff.
Zersetzung ab 290° C

### Beispiel 5

### 3-(5,7-Dichlor-4H-chinazolin-3-yl)pyrrolidin-2,5-dion

Bei Ersatz der in Beispiel 4, Stufe 1, verwendeten Anilins durch das entsprechende 3,5-Dichlorisomer und Anwendung der in Stufen 1 bis 8 beschriebenen Verfahrensweise erhielt man die Titelverbindung in Form eines gelblicher Feststoffs:
Schmelzpunkt: 215-217 °C

### Beispiel 6

### 3-(5-Brom-4H-chinazolin-3-yl)pyrrolidin-2,5-dion

Bei Ersatz der in Beispiel 4, Stufe 2, verwendeten 1-*H*-Indol-2,3-dions durch das entsprechende 4-Bromisomer und Anwendung der in Stufen 2 bis 8 beschriebenen Verfahrensweise erhielt man die Titelverbindung in Form eines gelben Feststoffs.
Schmelzpunkt: 214-217 °C

### Beispiel 7

### 3-(7-Trifluormethyl-4H-chinazolin-3-yl)-pyrrolidin-2,5-dion

Bei Ersatz der in Beispiel 4, Stufe 3, verwendeten 2-Aminobenzoesäure durch das entsprechende Ausgangsmaterial und Anwendung der in Stufen 3 bis 8 beschriebenen Verfahrensweise erhielt man die Titelverbindung.
Schmelzpunkt: 213-215 °C

Die in diesem Beispiel als Ausgangsmaterial verwendete 2-Aminobenzoesäure ließ sich in einem Schritt aus der entsprechenden 2-Nitrobenzosäure herstellen:
2-Amino-4-trifluormethylbenzoesäure

Ein Lösung von 2-Nitro-4-trifluormethylbenzoesäure (10,0 g, 42,6 mmol) in Methanol (20 mL) wurde mit 10 % Palladium auf Aktivkohle (1.00 g) versetzt und bei Raumtemperatur unter 3 bar Wasserstoffatmosphäre hydriert bis 128 mmol Wasserstoff aufgenommen waren. Das Gemisch wurde über Kieselgur filtriert und das Filtrat im Vakuum eingeengt. Ausbeute: 8,48 g (97 %), weißer Feststoff.
Schmelzpunkt: 174-178 °C

### Beispiel 8

Bei Ersatz der in Beispiel 4, Stufe 3, verwendeten 2-Aminobenzoesäure durch das entsprechende Ausgangsmaterial und Anwendung der in Stufen 3 bis 8 beschriebenen Verfahrensweise erhielt man die nachfolgenden Verbindungen:
a) 3-(5,8-Dichlor-4*H*-chinazolin-3-yl)-pyrrolidin-2,5-dion ¹H-NMR (DMSO-d₆): 1.91 (3 H, s, HOAc): 2.86 (1 H, dd, J = 18.6 und 9.8 Hz), 3.11 (1H, dd, J = 18.6 und 5.9 Hz); 4.14 (1 H, d, J = 14.7 Hz); 4.67 (1 H, d, J = 15.7 Hz); 4.94 (1 H, dd, J = 8.8 und 6.8 Hz); 7.06 (1 H, d, J = 8.8 Hz); 7.23-7.34 (2 H, s und d überlagert); 11.65 (1 H, brs); 11.97 (1 H, br s, HOAc).
b) 3-(5-Methyl-4*H*-chinazolin-3-yl)-pyrrolidin-2,5-dion Schmelzpunkt: 221-224 °C
c) 3-(4*H*-Benzo[g]chinazolin-3-yl)-pyrrolidin-2,5-dion Schmelzpunkt: 100-102 °C
d) 3-(6,7-Difluor-4*H*-chinazolin-3-yl)-pyrrolidin-2,5-dion
e) 3-(6,8-Dichlor-4*H*-chinazolin-3-yl)-pyrrolidin-2,5-dion Schmelzpunkt: 230-232 °C
f) 3-(6-Nitro-4*H*-chinazolin-3-yl)-pyrrolidn-2,5-dion Schmelzpunkt: 168-172 °C
g) 3-(7-Chlor-4*H*-chinazolin-3-yl)pyrrolidin-2,5-dion Schmelzpunkt: 194 °C
h) 3-(7-Nitro-4*H*-chinazolin-3-yl)-pyrrolidin-2,5-dion Schmelzpunkt: 212-225 °C
i) 3-(8-Brom-6-methyl-4*H*-chinazolin-3-yl)-pyrrolidin-2,5-dion Schmelzpunkt: 240-241 °C
j) 3-(8-Chlor-4*H*-chinazolin-3-yl)-pyrrolidin-2,5-dion Schmelzpunkt: 194 °C
k) 3-(8-Methoxy-4*H*-chinazolin-3-yl)-pyrrolidin-2,5-dion Schmelzpunkt: 195-200 °C
l) 3-(6-Benzyloxy-4*H*-chinazolin-3yl)-pyrrolidin-2,5-dion Schmelzpunkt: 171-173 °C

Bei der Herstellung der eingesetzten 2-Amino-5-benzyloxybenzoesäure wurde mit Ausnahme des letzten Schritt wie in der Literatur (A. Witt, J. Bergman J. Org. Chem. 2001, 66, 2784) beschrieben vorgegangen.

Überraschenderweise wurde im letzten Schritt festgestellt, dass bei Behandlung mit Salzsäure nicht nur der Methylester und die N-Acetylgruppe wie erforderlich verseift wurden, sondern auch die Benzylgruppe entfernt wurde. Daher wurde die Verseifung des Methylesters durch längeres Erhitzen unter Rückfluss in Gegenwart von Natriumhydroxid in einem Wasser-Dioxan-Gemisch wie folgt durchgeführt:
Zu einer Suspension von 2-Acetylamino-5-benzyloxybenzoesäuremethylester (13,9 g, 46,4 mmol) in Wasser (120 mL) und 1,4-Dioxan (120 mL) wurde Natriumhydroxid (4,64 g, 116 mmol) gegeben. Das Gemisch wurde 5 h unter Rückfluss erhitzt, wobei eine Lösung entstand. Es wurde anschließend über Nacht weiter bei 80 °C gerührt, das Gemisch im Vakuum eingeengt, der Rückstand in Wasser aufgenommen und die Lösung mit Ameisensäure auf pH 3-4 gestellt. Der ausgefallene Feststoff wurde abfiltriert und getrocknet. Das entstandene Gemisch wurde erneut in Wasser (250 mL) gelöst, mit Natriumhydroxid (4,64 g, 116 mmol) versetzt, 8 h unter Rückfluss und über Nacht bei 80 °C gerührt. Das Reaktionsgemisch wurde abgekühlt und die Lösung mit Ameisensäure auf pH 3-4 gestellt. Das ausgefallene Produkt wurde abfiltriert und im Vakuum über Phosphorpentoxid getrocknet.
Ausbeute: 10,2 g (90 %) 2-Amino-5-benzyloxybenzoesäute, grauer Feststoff.
1 H-NMR (DMSO-d₆): 4.97 (2 H, s); 6.70 (1 H, d, J = 9.8 Hz); 7.01 (1 H, dd, J = 8.8 und 2.9 Hz); 7.28-7.43 (6 H, m); 8.40 (3 H, sehr br s).

### Beispiel 9

Bei Ersatz des in Beispiel 4, Stufe 5, verwendeten 2-Aminobenzamids durch das entsprechende Ausgangsmaterial und Anwendung der in Stufen 5 bis 8 beschriebenen Verfahrensweise erhielt man die nachfolgenden Verbindungen:

### a) 3-(5,6-Dichlor-4H-chinazolin-3-yl)pyrrolidin-2,5-dion Schmelzpunkt: 174-178 °C

Das in diesem Beispiel als Ausgangsmaterial verwendete 2-Aminobenzamid ließ sich in einem Schritt aus dem entsprechenden 2-Nitrobenzonitril herstellen:
6-Amino-2,3-dichlorbenzamid

Eine Lösung von 2,3-Dichlor-6-nitrobenzonitril (2,17 g, 10 mmol) in Ethanol (45 mL) wurde bei 45 °C mit 85 %igem Hydrazinhydrat (0,6 mL, 40 mmol) versetzt. Zu dieser Lösung wurde portionsweise wässrige 50 % Raney-Nickel-Suspension gegeben, bis keine Gasentwicklung mehr zu beobachten war. Anschließend wurde 2,5 h unter Rückfluss gekocht. Nach Abkühlen auf Raumtemperatur wurde das Reaktionsgemisch filtriert, der Filterrückstand mit heißem Ethanol gewaschen und das Filtrat im Vakuum eingeengt. Der Rückstand wurde wiederholt in Ethanol (2×) aufgenommen, jeweils im Vakuum eingeengt und anschließend durch Flashchromatographie mit Chloroform / Methanol (95:5) gereinigt.
Ausbeute: 1,48 g (72 %), weißer Feststoff.
Schmelzpunkt: 110-112 °C

### b) 3-(7-Methoxy-4H-chinazolin-3-yl)-pyrrolidin-2,5-dion

Schmelzpunkt: 174-184 °C

Das in diesem Beispiel verwendeten 2-Aminobenzamid ließ sich analog zum vorhergehenden Beispiel aus dem entsprechenden 2-Nitrobenzonitril herstellen:
2-Amino-4-methoxybenzamid
Schmelzpunkt: 152-155 °C

### Beispiel 10

Bei Ersatz des in Beispiel 4, Stufe 6, verwendeten 2-Aminobenzylamins durch das entsprechende Ausgangsmaterial und Anwendung der in Stufen 6 bis 8 beschriebenen Verfahrensweise erhielt man die nachfolgenden Verbindungen:

### a) 3-(5-Chlor-4H-chinazolin-3-yl)-pyrrolidin-2,5-dion

Schmelzpunkt: 218 °C

Das in diesem Beispiel als Ausgangsmaterial verwendete 2-Aminobenzylamin ließ sich in einem Schritt aus dem entsprechenden 2-Aminobenzonitril herstellen:
Zu 10,0 g (253 mmol) Lithiumaluminiumhydrid in wasserfreiem Tetrahydrofuran (520 mL) wurde eine Lösung von 2-Amino-6-chlor-benzonitril (19,0 g, 125 mmol) in wasserfreiem Tetrahydrofuran (150 mL) bei 20 - 30 °C zugetropft und anschließend 1 h unter Rückfluss gerührt. Bei 0 - 10°C wurde überschüssiges Lithiumaluminiumhydrid vorsichtig mit 125 mL THFNVasser (2:1) zerstört. Die erhaltene Suspension wurde über Kieselgur abgesaugt und mit Tetrahydrofuran gewaschen. Das Filtrat wurde im Vakuum eingeengt. Der Rückstand wurde durch Säulenchromatographie mit Dichlormethan / Methanol (4:1) gereinigt.
Ausbeute: 17,3 g (88.4 %), gelber Feststoff.
¹H-NMR (DMSO-d₆, 300 MHz): 1.76 (2 H, br s); 3.81 (2 H, s); 5.52 (2 H, br s); 6.59 (2 H, dd, J = 7.9 und 7.9 Hz); 6.91 (1 H, t, J = 7.9 Hz).

### b) 3-(6,7-Dimethoxy-4H-chinazolin-3-yl)-pyrrolidin-2,5-dion

Schmelzpunkt: 189-200 °C

Das in diesem Beispiel als Ausgangsmaterial verwendete 2-Aminobenzylamin ließ sich in einem Schritt aus dem entsprechenden 2-Aminobenzonitril herstellen:
2-Aminomethyl-4,5-dimethoxyphenylamin

Eine Lösung von 2-Amino-4,5-dimethoxybenzonitril (2,00 g, 11,2 mmol) in Tetrahydrofuran (50 mL) und 2 M Boran-Dimethylsulfid-Komplex in Tetrahydrofuran (28 mL, 56 mmol) wurde 7 h unter Rückfluss gekocht. Die abgekühlte Reaktionslösung wurde vorsichtig mit Wasser (5 mL) versetzt und dann im Vakuum eingeengt. Der Rückstand wurde mehrmals in Toluol und dann in Methanol aufgenommen und jeweils wieder eingeengt. Der Rückstand wurde durch Flashchromatographie mit Chloroform / Methanol (9:1 und 1 % Triethylamin) gereinigt.
Ausbeute: 1,51 g (74 %), bräunlicher Feststoff.
Schmelzpunkt: 86-87 °C

### c) 3-(6-Chlor4H-chinazolin-3-yl)=pyrrolidin-2,5-dion

Schmelzpunkt: 166 °C

Das in diesem Beispiel als Ausgangsmaterial verwendete 2-Aminobenzylamin ließ sich analog zu dem Ausgangsmaterial in Beispiel 10b in einem Schritt aus dem entsprechenden 2-Aminobenzonitril herstellen:
2-Aminomethyl-5-chlorphenylamin

¹H-NMR (DMSO-d₆): 1.82 (2 H, s); 3.58 (2 H, s); 5.21 (2 H, br s); 6.57 (1 H, d, J = 8.8 Hz); 6.92 (1 H, dd, J = 7.8 und 2.0 Hz); 7.07 (1 H, d, J = 2.0 Hz).

### Beispiel 11

Bei Ersatz des in Beispiel 4, Stufe 6, verwendeten 2-Aminobenzylamins durch das entsprechende Ausgangsmaterial und Anwendung der in Stufen 6 bis 8 beschriebenen Verfahrensweise erhielt man die nachfolgenden Verbindungen:

### a) 3-(4H-Chinazolin-3-yl)-pyrrolidin-2,5-dion

Schmelzpunkt: 168-174 °C

1 H-NMR (DMSO-d6): 2.87 (1 H, dd, J = 18.6 und 8,8 Hz); 3.00 (1 H, dd, J = 18.6 und 5.6 Hz); 4.13 (1 H, d, J = 13.7 Hz); 4.61 (1 H, d, J = 13.7 Hz); 4.84 (1 H, dd, J = 8.8 und 5.9 Hz); 6.90 (2 H, dd, J = 8.8 und 8.8 Hz); 6.98 (1 H, dd, J = 7.8 und 7.8 Hz); 7.11 (1 H, d, J = 7.8 Hz); 7.13 (1 H, s); 11.60 (1 H, br s).

### b) 3-(5-Fluor-4H-chinazolin-3-yl)-pyrrolidin-2,5-dion

Schmelzpunkt: 184-191 °C

### c) 3-(8-Trifluormethyl-4H-chinazolin-3-yl)-pyrrolidin-2,5-dion Schmelzpunkt: 188-190 °C

Bei Ersatz des in Beispiel 11a verwendeten Maleinimids durch N-Methylmaleinimid erhielt man die nachfolgende Verbindung:

### d) 1-Methyl-3-(4H-chinazolin-3-yl)-pyrrolidin-2,5-dion Schmelzpunkt: 163-164 °C

Bei Ersatz des in Beispiel 11a verwendeten Maleinimids durch N-Methylmaleinimid sowie Ersatz des 2-Aminomethylbenzylamins durch 2-Aminomethyl-5-fluorbenzylamin erhielt man die nachfolgende Verbindung:

### e) 7-Fluor-1-methyl-3-(4H-chinazolin-3-yl)-pyrrolidin-2,5-dion Schmelzpunkt: 191-193 °C

### Beispiel 12

### a) 3-(5-Methoxy-4H-chinazolin-3-yl)-pyrrolidin-2,5-dion

Bei Ersatz des in Beispiel 4, Stufe 6, verwendeten 2-Aminobenzylamins durch 6-Methoxy-2-aminobenzylamin und Anwendung der in Stufen 6 bis 8 beschriebenen Verfahrensweise erhielt man die Zielverbindung. Ausbeute: 131 mg (100 %), gelblicher Feststoff.
Schmelzpunkt: 222 °C (Zersetzung)

Das für die Herstellung von Beispiel 12a benötigte 6-Methoxy-2-aminobenzylamin wurde wie in der Literatur (J. H. Sellstedt et al. J. Med. Chem. 1975, 18, 926-933) beschrieben hergestellt, von 2,6-Dinitrobenzonitril ausgehend. Dabei erfolgt in der 1. Stufe Umsetzung mit Natriummethanolat in wasserfreiem Methanol.

2,6-Dinitrobenzonitril kann entweder kommerziell erworben oder nach Literatur (J. R. Beck, J. Org. Chem. 1972, 37, 3224-3226) hergestellt werden.

Bei Ersatz des in Beispiel 12a, Stufe 1 verwendeten Natriummethanolates in wasserfreiem Methanol durch entsprechende Natriumalkoholate im entsprechenden wasserfreien Alkohol und Anwendung der oben beschriebenen Verfahrensweise erhielt man die folgenden Zielverbindungen:

### b) 3-(5-Ethoxy-4H-chinazolin-3-yl)-pyrrolidin-2,5-dion

Schmelzpunkt: 203-205 °C

### c) 3-(5-Pentyloxy-4H-chinazolin-3yl)-pyrrolidin-2,5-dion

Schmelzpunkt: 154-160 °C

### d) 3-(5-Benzyloxy-4H-chinazolin-3-yl)-pyrrolidin-2,5-dion

Schmelzpunkt: 100-108 °C

### e) 3-(5-Isopropoxy-4H-chinazolin-3-yl)-pyrrolidin2,5-dion

Schmelzpunkt: 191-192 °C

### f) 3-(5-(2-Methoxyethoxy)-4-H-chinazolin-3-yl)-pyrrolidin -2,5-dion

Schmelzpunkt: 288-290 °C

### Beispiel 13:

### a) 3-(5-Ethansulfonyl-4H-chinazolin-3-yl)-pyrrolidin-2,5-dion

### Stufe 1:

### 2-Ethylsulfanyl-6-nitrobenzonitril

Eine Lösung von 2,6-Diaminobenzonitril (4,72 g, 24,4 mmol) und Triethylamin (2,60 g, 3,55 mL, 25,7 mmol) in N,N-Dimethylformamid (50 mL) wurde bei 0 °C mit einer Lösung von Ethanthiol (1,59 g, 1,9 mL, 25,7 mmol) in N,N-Dimethylformamid (10 mL) versetzt. Anschließend wurde 4 h bei Raumtemperatur gerührt. Nach Zugabe von Wasser (100 mL) wurde das ausgefallene Produkt abfiltriert, mit Wasser gewaschen und über Phosphorpentoxid getrocknet.
Ausbeute: 4,50 g (88 %), gelber Feststoff.

Schmelzpunkt: 126-127 °C

### Stufe 2:

### 2-Ethansulfonyl-6-nitrabenzonitril

Eine Suspension von Periodsäure (8,76 g, 38,4 mmol) in Acetonitril (160 mL) wurde bei Raumtemperatur 30 min kräftig gerührt und dann mit Chrom(VI)-oxid (19,2 mg, 0,19 mmol) versetzt. Die Mischung wurde 5 min bei Raumtemperatur gerührt, wobei sie sich gelborange färbte. Zu dieser Mischung wurde eine Lösung des Produkts aus Stufe 1 (2,00 g, 9,6 mmol) in Acetonitril (40 mL) getropft, wobei ein weißer Niederschlag ausfiel. Das Reaktionsgemisch wurde über Nacht gerührt, der ausgefallene Niederschlag abfiltriert und das Filtrat im Vakuum eingeengt. Der Rückstand wurde in Essigsäureethylester (150 mL) aufgenommen und mit gesättigter Natriumsulfitlösung (dreimal 50 mL) und gesättigter Natriumchloridlösung (60 mL) gewaschen. Die organische Phase wurde mit Natriumsulfat getrocknet und im Vakuum eingeengt.
Ausbeute: 2,19 g (95 %), weißer Feststoff.

Schmelzpunkt: 162-163 °C

### Stufe 3:

### 2-Amino-6-ethansulfonylbenzamid

Eine Lösung des Produkts aus Stufe 2 (2,10 g, 8,74 mmol) in Ethanol (200 mL) wurde bei 70 °C mit 99%igem Hydrazinhydrat (500 mg, 486 µL, 10 mmol) versetzt. Zu dieser Lösung wurde portionsweise wässrige 50%ige Raney-Nickel-Suspension gegeben, bis keine GasentwiCklung mehr zu beobachten war. Anschließend wurde 2 h unter Rückfluss gerührt. Es wurde nochmals Hydrazinhydrat (500 mg, 486 µL, 10 mmol) und Raney-Nickel-Suspension zugegeben sowie weitere 2 h unter Rückfluss erhitzt. Nach Abkühlen auf Raumtemperatur wurde der Katalysator abfiltriert, der Filterrückstand mit heißem Ethanol und Ethylacetat gewaschen und das Filtrat im Vakuum eingeengt. Der erhaltene Rückstand wurde in Essigsäureethylester (150 mL) aufgenommen, mit Natriumsulfat getrocknet und erneut im Vakuum eingeengt.
Ausbeute: 1,88 g (94 %), cremefarbener Feststoff.

Schmelzpunkt: 128-134 °C

### Stufe 4:

### 2-Aminomethyl-3-ethansulfonylphenylamin

Eine Lösung des Produkts aus Stufe 3 (1,88 g, 8,2 mmol) in absolutem Tetrahydrofuran (100 mL) wurde mit einer 2 M Lösung des Boran-Dimethylsulfid-Komplexes in Tetrahydrofuran (12,3 mL, 24,6 mmol) versetzt und 8 h unter Rückfluss erhitzt. Anschließend wurde Wasser (3 mL) zugegeben, die Reaktionslösung im Vakuum eingeengt, der Rückstand mit Toluol und Methanol (je zweimal) versetzt und jeweils wieder eingeengt. Das Rohprodukt (2,12 g) wurde durch Flashchromatographie mit Chloroform und 1 % Triethylamin und später mit Chloroform / Methanol (9:1) und 1 % Triethylamin gereinigt.
Ausbeute: 1,41 g (80 %), gelbliches Öl.
1 H-NMR (DMSO-d6): 1.10 (3 H, t, J = 7.3 Hz); 3.30 (2 H, q, J = 6.8 Hz); 3.98 (2 H, s); 5.71 (2 H, s, austauschbar); 6.99 (1 H, d, J = 7.8 Hz); 7.08 (1 H, d, J = 7.8 Hz); 7.19 (1 H, t, J = 7.8 Hz). 2 austauschbare Signale liegen unter dem HDO-Signal.

### Stufe 5:

### 3-(2-Amino-6-ethansulfonylbenzylamino)-pyrrolidin-2,5-dion

Eine Lösung des Produkts aus Stufe 4 (220 mg, 1 mmol) in Tetrahydrofuran (50 mL) wurde mit Maleinimid (146 mg, 1,5 mmol) versetzt und 24 h bei Raumtemperatur gerührt. Das Lösungsmittel wurde im Vakuum entfernt und der Rückstand durch Flashchromatographie mit Chloroform / Methanol (9:1) gereinigt.

Ausbeute: 109 mg (35 %), weißer Feststoff.
1 H-NMR (DMSO-d6): 1.12 (3 H, t, J = 6.8 Hz); 2.46-2.49 (1 H, m); 2.51-2.58 (1 H, m); 2.85 (1 H, dd, J = 17.6 und 8.8 Hz); 3.22-3.32 (2 H, m); 3.79-3.96 (2 H, m); 4.17 (1 H, dd, J = 12.9 und 5.7 Hz); 5.12 (2 H, s); 7.00 (1 H, d, J = 7.8 Hz); 7.11 (1 H, d, J = 6.8 Hz); 7.22 (1 H, t, J = 7.8 Hz); 11.20 (1 H, s).

### Stufe 6:

### 3-(5-Ethansulfonyl-4H-chinazolin-3-yl)-pyrrolidin-2,5-dion

Eine Lösung des Produkts aus Stufe 5 (100 mg, 0,32 mmol) in Eisessig (5 mL) wurde mit Orthoameisensäuretriethylester (95 mg, 105 µL, 0.64 mmol) versetzt und 24 h bei Raumtemperatur gerührt. Das Lösungsmittel wurde im Vakuum entfernt und der Rückstand wiederholt mit Toluol versetzt und jeweils wieder eingeengt.
Ausbeute: 92 mg (88 %), weißer Feststoff.

Schmelzpunkt: 210-212 °C

### b) 3-(5-Ethansulfinyl-4H-chinazolin-3-yl)-pyrrolidin-2,5-dion

Bei Verwendung von Natriummetaperiodat anstelle von Periodsäure und Chrom(VI)oxid in Beispiel 13, Stufe 2, und Anwendung der in Beispiel 13, Stufe 1 sowie in Stufe 3 bis 6 beschriebenen Verfahren erhält man die Titelverbindung in Form eines Gemisches der Diastereomeren in einem Verhältnis von ca. 1 : 1.
¹H-NMR (DMSO-d₆): 1.02-1.11 (3 H, m); 2.50-3.14 (4 H, m); 4.17 (0.5 H, d, J = 14.7 Hz); 4.29 (0.5 H, d, J = 14.7 Hz); 4.57 (0.5 H, d, J = 14.7 H); 4.74 (0.5 H, d, J = 13.7 Hz); 4.85-4.95 (1 H, m); 6.99-7.08 (1H, m); 7.21 (1 H, d, J = 6.8 Dz); 7.35-7.44 (2 H, m); 11.57, br s)

### c) 3-(5-Ethylthio-4H-chinazolin-3-yl)-pyrrolidin-2,5-dion

Bei Auslassung der Stufe 2 in Beispiel 13a, und Anwendung der in Beispiel 13a, Stufe 1 sowie in Stufe 3 bis 6 beschriebenen Verfahren erhielt man die Titelverbindung.

### Beispiel 14:

### 3-(5-Nitro-4H-chinazolin-3yl)-pyrrolidin-2,5-dion

Bei Ersatz der in Beispiel 4, Stufe 3, verwendeten 2-Aminobenzoesäure durch 2-Amino-6-nitrobenzoesäure und Anwendung der in Stufen 3 bis 8 beschriebenen Verfahrensweise erhielt man die Zielverbindung. Schmelzpunkt: 125-129 °C

Die Herstellung der 2-Amino-6-nitrobenzoesäure erfolgte nach Literatur (R. Kahn, Chem. Ber. 1902, 35, 3857-3884; W. S. Saari, J. E. Schwering, J. Heterocyct. Chem. 1986, 23, 1253-1255) über die regioselektive Öffnung von 3-Nitrophthalimid mit wässrigem Kaliumhydroxid zur Phthalamidsäure. und anschließendem Hofmann-Abbau mit Natriumhypobromit zur 2-Amino-6-nitrobenzoesäure.

### Beispiel 15

Bei Ersatz des in Beispiel 3, Stufe 5, verwendeten Orthoameisensäuretriethylester durch Orthoessigsäuretriethylester sowie Einsatz entsprechender Ausgangsverbindungen erhielt man analog die nachfolgenden Verbindungen:

### a) 1-Methyl-3-(2-methyl-4H-chinazolin-3-yl)-pyrrolidin-2,5-dion

¹H-NMR (DMSO-d₆): 2.14 (3 H, s); 2.90 (3 H, s); 2.99 (2 H, m); 4.01 (1 H, m); 4.48 (1 H, d); 5.22 (1 H, m); 6.88 (3 H, m); 7.11 (1 H, m).

### b) 3-(2-Methyl-4H-chinazolin-3-yl)-pyrrolidin-2,5-dion

Schmelzpunkt: 72-75 °C
1H-NMR (DMSO-d6): 2.12 (3 H, s); 2.91 (1 H, dd, J = 17.6 und 8.8 Hz); 2.97 (1 H, dd, J = 17.6 und 5.9 Hz); 4.02 (1 H, d, J = 13.7 Hz); 4.52 (1 H, d, J = 13.7 Hz); 5.22 (1 H, dd, J = 8.8 und 5.9 Hz); 6.88 (1 H, d, J = 7.8 Hz); 6.94 (2 H, d, J = 4.9 Hz); 7.06 -7.13 (1 H, m); 11.33 (1 H, br s).

### c) 3-(5,6-Dichlor-2-methyl-4H-chinazolin-3-yl)-pyrrolidin-2,5-dion

Schmelzpunkt: 161-166 °C

### d) 3-(5,7-Dichlor-2-methyl-4H-chinazolin-3-yl)-pyrrolidin-2,5-dion

Schmelzpunkt: 199-201 °C

### e) 3-(5,7-Difluor-2-methyl-4H-chinazolin-3-yl)-3-pyrrolidin-2,5-dion

Freie Base:
Schmelzpunkt: 176 - 178 °C

Das Hydrochlorid der Titelverbindung wurde durch Fällung aus 2-Butanon mit Chlortrimethylsilan und Wasser im Eisbad hergestellt:
3-(5,7-Difluor-2-methyl-4*H*-chinazolin-3-yl)-pyrrolidin-2,5-dion Hydrochlorid ¹H-NMR (DMSO-d₆): 13.6 (1 H, s); 11.8 (1 H, s); 7.24 (1 H, dd, J = 9.8 und 9.0 Hz); 7.3 (1 H, d, J = 9.0 Hz); 5.6 (1 H, t, J = 8.0 Hz); 4.91 (1 H, d, J = 15.1 Hz); 4.53 (1 H, d, J = 15.1 Hz); 3.24 (1 H, dd, J = 18.1 und 6.8 Hz); 3.01 (1 H, dd, J = 18.1 und 9.0 Hz); 2.57 (3 H, s).

### f) 3-(5,8-Dichlor-2-methyl-4H-chinazolin-3-yl)-pyrrolidin-2,5-dion

Schmelzpunkt: 186-191 °C

### g) 3-(5-Benzyloxy-2-methyl-4H-chinazolin-3-yl)-pyrrolidin-2,5-dion

¹H-NMR (DMSO-d₆): 1.90 (4 H, s, HOAc); 2.10 (3 H, s); 2.92 (1 H, dd, J = 17.6 und 8.8 Hz); 3.05 (1 H, dd, J = 18.6 und 5.9 Hz); 4.09 (1 H, d, J = 13.7 Hz); 4.43 (1 H, d, J = 14.7 Hz); 5.11 (2 H, s); 5.25 (1 H, dd, J = 8.8 und 5.9 Hz); 6.49 (1 H, d, J = 7.8 Hz); 6.67 (1 H, d, J = 7.8 Hz); 7.02 (1 H, t, J = 7.8 Hz); 7.09-7.44 (5 H, m); ca. 11.7 (1 H, sehr br s).

### h) 3-(5-Brom-2-methyl-4H-chinazolin-3-yl)-pyrrolidin-2,5-dion

¹H-NMR (DMSO-d₆): 2.11 (3 H, s); 2.94 (1 H, dd, J = 18.0 und 9.3 Hz); 3.10 (1 H, dd, J = 17.6 und 5.9 Hz); 4.07 (1 H, d, J = 13.7 Hz); 4.48 (1 H, d, J = 13.7 Hz); 5.27 (1 H, dd, J = 8.8 und 5.9 Hz); 6.86 (1 H, t, J = 8.8 Hz); 7.07 (1 H, t, J = 8.3 Hz); 7.20 (1 H, d, J = 7.8 Hz); 11.92 (1 H, s).

### i) 3-(5-Chlor-2-methyl-4H-chinazolin-3-yl)-pyrrolidin-2,5-dion

Schmelzpunkt: 163-170 °C

### j) 3-(5-Chlor-7-fluor-2-methyl-4H-chinazolin-3-yl)-pyrrolidin-2,5-dion

Freie Base:
Schmelzpunkt: 179-181 °C

Das Hydrochlorid der Titelverbindung wurde auf übliche Weise erhalten:
3-5-Chlor-7-fluor-2-methyl-4*H*-chinazolin-3-yl)-pyrrolidin-2,5-dion Hydrochlorid

### k) 3-(5-Ethoxy-2-methyl-4H-chinazolin-3-yl)-pyrrolidin-2,5-dion

Schmelzpunkt: 130-133 °C

### l) 3-(5-Fluor-2-methyl-4H-chinazolin-3-yl)-pyrrolidin-2,5-dion

Schmelzpunkt: 185 °C

### m) 3-(5-Isopropoxy-2-methyl-4H-chinazolin-3-yl)-pyrrolidin-2,5-dion

Schmelzpunkt: 116-119 °C

### n) 3-(2,5-Dimethyl-4H-chinazolin-3-yl)-pyrrolidin-2,5-dion

Schmelzpunkt: 200-201 °C

### o) 3-(5-Methoxy-2-methyl-4H-chinazolin-3-yl)-pyrrolidin-2,5-dion

Schmelzpunkt: 122 °C

### p) 3-(2-Methyl-5-nitro-4H-chinazolin-3-yl)-pyrrolidin-2,5-dion

Schmelzpunkt: > 260 °C Zersetzung

### q) 3-(2-Methyl-5-pentyloxy-4H-chinazolin-3-yl)-pyrroldin-2,5-dion

Schmelzpunkt: 154-156 °C

### r) 3-(5-Ethylthio-2-Methyl-4H-chinazolin-3-yl)-pyrrolidin-2,5-dion

### s) 3-(5-Ethansulfonyl-2-methyl-4H-chinazolin-3-yl)-pyrrolidin-2,5-dion

1 H-NMR (DMSO-d6): 1.08 (3 H, t, J = 7.3 Hz); 2.15 (3 H, s); 3.00 (2 H, d, J = 6.8 Hz); 3.16-3.30 (2 H, m); 4.48 (1 H, d, J = 14.7 Hz); 4.67 (1 H, d, J = 14.7 Hz); 5.26 (1 H, t, J = 7.3 Hz); 7.23 (1 H, d, J = 7.8 Hz); 7.43 (1 H, t, J = 7.8 Hz); 7.49 (1 H, d, J = 7.8 Hz).

### t) 3-(2-Methyl-4H-benzo[g]chinazolin-3-yl)-pyrrolidin-2,5-dion

Schmelzpunkt: 106-110 °C

### u) 3-(6,7-Difluor-2-methyl-4H-chinazolin-3-yl)-pyrrolidin-2,5-dion

Schmelzpunkt: 183-185 °C

### v) 3-(6,7-Dimethoxv-2-methyl-4H-chinazolin-3-yl)-pyrrolidin-2,5-dion

Schmelzpunkt: 200-202 °C

### w) 3-(6,8-Dichlor-2-methyl-4H-chinazolin-3-yl)-pyrrolidin-2,5-dion

Schmelzpunkt: 180-182 °C

### x) 3-(6-Benzyloxy-2-methyl-4H-chinazolin-3yl)-pyrrolidin-2,5-dion

Schmelzpunkt: 132-143 °C

### y) 3-(6-Chlor-2-methyl-4H-chinazolin-3-yl)-pyrrolidin-2,5-dion

Schmelzpunkt: 195-203 °C

### z) 3-(2-Methyl-6-nitro-4H-chinazolin-3-yl)-pyrrolidin-2,5-dion

Schmelzpunkt: 170-175 °C

### aa)3-(2-Methyl-7-trifluormethyl-4H-chinazolin-3-yl)-pyrrolidin-2,5-dion

Schmelzpunkt: 205-206 °C

### bb)3-(7-Chlor-2-methyl-4H-chinazolin-3-yl)-pyrrolidin-2,5-dion

Schmelzpunkt: 153-155 °C

### cc) 3-(7-Fluor-2-methyl-4H-chinazolin-3-yl)-pyrrolidin-2,5-dion

¹H-NMR (DMSO-d₆) 2.12 (3 H, s); 2.90 (1 H, dd, J = 17.6 und 8.8 Hz); 3.00 (1 H, dd, J = 18.0 und 6.0 Hz); 4.02 (1 H, d, J = 13.7 Hz); 4.51 (1 H, d, J = 13.7 Hz); 5.25 (1 H, dd, J = 8.8 und 6.8 Hz); 6.65 (1 H, d, J = 10.8 Hz); 6.77 (1 H, dt, J = 8.8 und 2.0 Hz); 6.98 (1 H, t, J = 6.8 Hz); ca. 11.8 (1 H, sehr br s).

### dd) 3-(7-Methoxy-2-methyl-4H-chinazolin-3-yl)-pyrrolidin-2,5-dion

¹H-NMR (DMSO-d₆): 2.12 (3 H, s); 2.90 (1 H, dd, J = 18.0 und 9.0 Hz); 2.98 (1 H, dd, J = 18.0 und 6.0 Hz); 3.69 (3 H, s); 3.98 (1 H, d, J = 13.7 Hz); 4.45 (1 H, d, J = 13.7 Hz); 5.23 (1 H, dd, J = 8.8 und 6.8 Hz); 6.46 (1 H, d, J = 2.0 Hz); 6.55 (1 H, dd, J = 7.8 und 2.0 Hz); 6.86 (1 H, d, J = 7.8 Hz); 11-12 (1 H, sehr br s).

### ee) 3-(2-Methyl-7-nitro-4H-chinazolin-3-yl)-pyrrolidin-2,5-dion

Schmelzpunkt: 203-210 °C

### ff) 3-(8-Brom-2,6-dimethyl-4H-chinazolin-3-yl)-pyrrolidin-2,5-dion

Schmelzpunkt: 145-150 °C

### gg) 3-(2-Methyl-8-trifluormethyl-4H-chinazolin-3-yl)-pyrrolidin-2,5-dion

¹H-NMR (DMSO-d₆): 2.16 (3 H, s); 2.91 (1 H, dd, J = 18.6 und 8.8 Hz); 3.00 (1 H, dd, J = 18.6 und 6.8 Hz); 4.10 (1 H, d, J = 13.7 Hz); 4.59 (1 H, d, J = 13.7 Hz); 5.26 (1H, dd, J = 8.6 und 6.8 Hz); 7.07 (1 H, t, J = 7.8 Hz); 7.21 (1 H, d, J = 7.8 Hz); 7.42 (1 H, d, J = 7.8 Hz); 11.60 (1 H, br s).

### hh)3-(8-Chlor-2-methyl-4H-chinazolin-3-yl)-pyrrolidin-2,5-dion

Schmelzpunkt: 176-178 °C

### ii) 3-(8-Methoxy-2-methyl-4H-chinazolin-3-yl)-pyrrolidin-2,5-dion

¹H-NMR (DMSO-d₆): 1.90 (6 H, s, HOAc); 2.13 (3 H, s); 2.90 (1 H, dd, J =18.6 und 8.8 Hz); 2.96 (1 H, dd, J = 18.6 und 6.8 Hz); 3.72 (3 H, s); 3.98 (1 H, d, J = 13.7 Hz); 4.46 (1 H, d, J = 13.7 Hz); 5.22 (1 H, dd, J = 8.8 und 6.8 H); 6.53 (1 H, d, J = 7.8 Hz); 6.77 (1 H, d, J = 8.8 Hz); 6.92 (1 H, t, J = 7.8 Hz).

### jj) 3-(5-(2-Methoxyethoxy)-2-methyl-4H-chinazolin-3-yl) pyrrolidin -2,5-dion

1 H-NMR (DMSO-d6): 2.10 (3 H, s); 2.94-3.00 (2 H, m); 3.28 (3 H, s); 3.59-3.62 (2 H, m); 4.01 (1 H, d, J = 13.7 Hz); 4.05-4.08 (2 H, m); 4.37 (1 H, d, J = 13.7 Hz); 5.24 (1 H, dd, J = 8.8 und 6.8 Hz); 6.50 (1 H, d, J = 7.8 Hz); 6.64 (1 H, d, J = 7.8 Hz); 7.05 (1 H, t, J = 7.8 Hz); 11.52 (1 H br s).

### Beispiel 16

### a) 3-(7-Amino-4H-chinazolin-3-yl)-pyrrolidin-2,5-dion-Hydrochlorid

### Stufe 1:

### 3-(7-Amino-4H-chinazolin-3-yl)-pyrrolidin-2,5-dion

Eine Lösung der Nitroverbindung aus Beispiel 14 (93 mg, 0,34 mmol) in Essigsäure (25 mL) wurde mit Palladium auf Aktivkohle (20 mg, 10 %) 20 min bei 3 bar Wasserstoffatmosphäre und Raumtemperatur hydriert. Anschließend wurde das Reaktionsgemisch filtriert und im Vakuum eingeengt.
Ausbeute: 80 mg (99 %), bräunliches Öl.
¹H-NMR (DMSO-d₆): 2.90 (1 H, dd, J = 16.6 und 9.7 Hz); 3.48 (1 H, t, J = 5.3 Hz); 3.96 (1 H, d, J = 12.7 Hz); 4.42 (1 H, d, J = 12.7 Hz); 4.82 (1 H, m); 6.22 (1 H, m); 6.58 (1 H, m); 7.06-7.30 (2 H, m).

### Stufe 2:

### 3-(7-Amino-4H-chinazolin-3-yl)-pyrrolidin-2,5-dion-Hydrochlorid

Eine Lösung der Aminoverbindung aus Stufe 1 (93 mg, 0,34 mmol) in Methanol (3 mL) wurde unter Rühren mit einer Lösung von Chlorwasserstoff in Diethylether (1 mL) versetzt, wobei sofort ein heller Niederschlag ausfiel. Nach Zugabe von Diethylether wurde dekantiert, erneut mit Diethylether versetzt und dekantiert. Der Rückstand wurde im Vakuum getrocknet.
Ausbeute: 50 mg (53 %), gelber Feststoff.

¹³C-NMR (DMSO-d₆): 32.3; 43.2; 62.1; 100.9; 106.9; 111.8; 118.1; 127.9; 130.0; 151.8; 173.6; 174.8.

Bei Ersatz des in Beispiel 15a, Stufe 1, verwendeten Ausgangsmaterials durch entsprechende Ausgangsverbindungen und Anwendung der in Stufen 1 bis 2 beschriebenen Verfahrensweise erhielt man analog die nachfolgenden Verbindungen:

### b) 3-(7-Amino-2-methyl-4H-chinazolin-3-yl)-pyrrolidin-2,5-dionHydrochlorid

¹H-NMR (DMSO-d₆): 2.57 (3 H, s); 2.98-3.10 (2 H, m); 4.47 (1 H, d, J = 14.7 Hz); 4.90 (1 H, d, J = 14.7 Hz); 5.53 (1 H, t, J = 7.8 Hz); 7.10 (1 H, d, J 7.8 Hz); 7.19 (2 H, d, J = 6.8 Hz); 11.92 (1 H, s); 13.34 (1 H, s).

### c) 3-(6-Amino-4H-chinazolin-3-yl)-pyrrolidin-2,5-dion Hydrochlorid

¹H-NMR (DMSO-d₆): 2.98 (1 H, dd, J = 18.0 und 9.0 Hz); 3.18 (1 H, dd, J = 17.6 und 5.9 Hz); 4.56 (1 H, d, J = 15.6 Hz); 4.99 (1 H, d, J = 8.8 Hz); 5.25 (1 H, dd, J = 9.8 und 5.8 Hz); 7.02 (1 H, s); 7.19 (1 H, d, J = 8.8 Hz); 7.24 (1 H, d, J = 8.8 Hz); 8.69 (1 H, s); 11.86 (1 H, s); 13.10 (1 H, sehr br s).

### d) 3-(6-Amino-2-methyl-4H-chinazolin-3-yl)-pyrrolidin-2,5-dion-Hydrochlorid

¹H-NMR (DMSO-d₆): 2.56 (3 H, s); 2.99 (1 H, dd, J = 18. 0 und 9.0 Hz); 3.18(1 H, dd, J = 18.6 und 5.9 Hz); 4.51 (1 H, d, J = 15.6 Hz); 4.95 (1 H, d, J = 15.6 Hz); 5.53 (1 H, dd, J = 8.9 und 6.8 Hz); 7.07 (1 H, s); 7.22 (1 H, d, J = 7.8 Hz); 7.32 (1 H, d, J = 7.8 Hz); 11.90 (1 H, s); 13.3 (1 H, s).

### e) 3-(5-Amino4H-chinazolin-3-yl)-pyrrolidin-2,5-dion Hydrochlorid

¹H-NMR (DMSD-d6): 2.96 (1 H, dd, J = 17.6 und 9.0 Hz), 3.20 (1 H, dd, J = 17.6 und 5.9 Hz); 4.27 (1 H, d, J = 14.7 Hz); 4.87 (1 H, d, J = 15.6 Hz); 5.27 (1 H, dd, J = 9.0 und 6.0 Hz); 6.61 (1 H, d, J = 7.8 Hz); 6.78 (1 H, d, J = 7.8 Hz); 6.84 (2 H, sehr br s); 7.13 (1 H, t, J = 7.8 Hz); 11.92 (1 H, s); 13.03 (1 H, s).

### f) 3-(5-Amino-2-methyl-4H-chinazolin-3-yl)pyrrolidin-2,5-dionHydrochlorid

¹H-NMR (DMSO-d6): 2.33 (3 H, s); 2.99 (1 H, dd, J = 18.0 und 9.0 Hz); 3.26 (1 H, dd, J = 18.0 und 6.0 Hz), 4.30 (1 H, d, J = 14.7 Hz); 4.83 (1 H, d, J = 14.7 Hz); 5.55 (1 H, dd, J = 9.0 und 6.8 Hz); 6.49-7.04 (2 H, sehr br s); 6.73 (1 H, d, J = 7.8 Hz); 6.79 (1 H, d, J = 7.8 Hz); 7.14 (1 H, t, J = 7.8 Hz); 11.90 (1 H, s); 13.02 (1 H, s).

### Beispiel 17

### a) 3-(5-Hydroxy-4H-chinazolin-3-yl)-pyrrolidin-2,5-dion Hydrochlorid

### Stufe 1:

### 3-(5-Hydroxy-4H-chinazolin-3-yl)-pyrrolidin-2,5-dion

Eine Lösung des Produkts aus Beispiel 12e (550 mg, 1,64 mmol) in Eisessig (50 mL) wurde unter Argon mit 10 % Palladium auf Aktivkohle (144 mg) versetzt und anschließend 3 h bei Raumtemperatur und 3 bar Wasserstoff hydriert. Die Reaktionslösung wurde filtriert und im Vakuum eingeengt.
Ausbeute: 606 mg, braunes Öl.
¹H-NMR (DMSO-d₆): 2.83 (1 H, dd, J = 18.6 und 8.8 Hz); 3.02 (1 H, dd, J = 17.6 und 5.9 Hz); 3.98 (1 H, d, J = 13.7 Hz); 4.45 (1 H, d, J = 14.7 Hz); 4.85 (1 H, dd, J = 9.8 und 5-9 Hz); 6.35 (1 H, d, J = 7.8 Hz); 6.48 (1 H, d, J = 7.8 Hz); 6.91 (1 H, t, J = 7.8 Hz); 7.09 (1 H, s).

### Stufe 2:

### 3-(5-Hydroxy-4H-chinazolin-3-yl)-pyrrolidin-2,5-dion-Hydrochlorid

Eine Lösung des Produkts aus Stufe 1 (650 mg, 2,01 mmol) in Eisessig (1 mL) wurde mit einer Lösung von Chlorwasserstoff in Diethylether (5 mL) versetzt. Der Überstand wurde dekantiert, der Feststoff wiederholt mit Diethylether versetzt und jeweils wieder dekantiert. Das feste Produkt wurde über Kaliumhydroxid im Exsikkator getrocknet.
Ausbeute: 373 mg (61 %), weißer Feststoff.

Schmelzpunkt: 182-188 °C

Bei Ersatz des Produkts aus Beispiel 12e in Beispiel 17a durch die Produkte aus Beispiel 9b, 15f und 15v und Anwendung der in Beispiel 17a, Stufen 1 bis 2, beschriebenen Verfahrensweise, erhielt man analog die nachfolgenden Verbindungen:

### b) 3-(67Hydroxy-2-methyl-4H-chinazolin-3-yl)-pyrrolidin-2,5-dion Hydrochlorid

¹H-NMR (DMSO-d₆): ca. 2.5 (3 H, s); 3.01 (1 H, dd, J = 18.0 und 9.4 Hz); 3.16 (1 H, dd, J = 18.4 und 6.7 Hz); 4.39 (1 H, d, J = 14.9 Hz); 4.84 (1 H, d, J = 15.6 Hz); 5.47 (1 H, dd, J = 9.4 und 6.3 Hz); 6.60 (1 H, d, J = 2.3 Hz); 6.75 (1 H, dd, J = 8.6 und 3.1 Hz); 7.05 (1 H, dd, J = 5.8 und 2.8 Hz); 9.92 (1 H, s); 11.84 (1 H, s); ca. 12.6 (1 H sehr br s).

### c) 3-(6-Hydroxy-2-methyl-4H-chinazolin-3-yl)-pyrrolidin-2,5-dion Hydrochlorid

¹H-NMR (DMSO-d₆): ca. 2.5 (3 H, s, überlagert von DMSO-Signal); 3.01 (1 H, dd, J = 18.0 und 9.4 Hz); 3.16 (1 H, dd, J = 18.4 und 6.7 Hz); 4.39 (1 H, d, J = 14.9 H); 4.84 (1 H, d, J = 15.6 Hz); 5.47 (1 H, dd, J = 9.4 und 6.3 Hz); 6.60 (1 H, d, J = 2.3 Hz); 6.75 (1 H, dd, J = 8.6 und 3.1 Hz); 7.05 (1 H, dd, J = 5.8 und 2.8 Hz); 9.92 (1 H, s); 11.84 (1 H, s); ca. 12.6 (1 H sehr br s).

### d) 3-(5-Hydroxy-2-methyl-4H-chinazolin-3-yl)-pyrrolidin-2,5-dion Hydrochlorid

1H-NMR (DMSO-d6): 1.90 (3 H, s, HOAc); 2.53 (3 H, s); 3.02 (1 H, dd, J = 18.6 und 8.8 Hz); 3.25 (1 H, dd, J = 18.6 und 5.9 Hz); 4.31 (1 H, d, J = 14.7 Hz); 4.70 (1 H, d, J = 15.7); 5.51 (1 H, dd, J = 8.8 und 6.8 Hz); 6.69 (1 H, d, J = 7.8 Hz); 6.77 (1 H, d, J = 7.8 Hz); 7.15 (1 H, t, J = 7.8 Hz); 10.48 (1 H, s); 11.91 (1 H, s); 12.00 (1 H, br s); 12.93 (1 H, s).

### Beispiel 18

Die Position der Thiocarbonylgruppe ist bisher nicht bewiesen. Sie folgt als Analogieschluss aus den Untersuchungen zur Thionierung von 2-Benzyloxycarbonyl-Aminoglutarimid, wobei die Carbonylgruppe am schnellsten reagierte, die sterisch am wenigsten gehindert war.

### a) 3-(7-Fluor-4H-chinazolin-3-yl)-5-thioxopyrrolidin-2-on-Hydrobromid

### Stufe 1:

### (2-{[Benzyloxycarbonyl-(2,5-dioxopyrrolidin-3-yl)-amino]-methyl} 5-fluorphenyl)-carbamidsäurebenzylester

Eine Lösung des Produkts aus Beispiel 3, Stufe 4 (9,83 g, 41 mmol), Kohlensäurebenzylester-2,5-dioxopyrrolidin-1-yl-ester (22,0 g, 88 mmol) und 4-N,N-Dimethylaminopyridin (100 mg) in wasserfreiem Tetrahydrofuran (50 mL) wurde 24 h bei Raumtemperatur gerührt. Das Reaktionsgemisch wurde im Vakuum eingeengt, der Rückstand in Essigsäureethylester gelöst und die organische Phase zweimal mit Wasser gewaschen. Die organische Phase wurde mit Natriumsulfat getrocknet und im Vakuum eingeengt. Die Hälfte des Rückstandes wurde durch Flashchromatographie mit Essigsäureethylester / Cyclohexan (2:3) gereinigt, wodurch 5,54 g eines Gemisches aus mono- und di-Benzyloxycarbonyl-substituiertem Produkt im Verhältnis von ca. 1:-1 erhalten wurden. Wiederholte Umsetzung mit Kohlensäurebenzylester-2,5-dioxopyrrolidin-1-yl-ester und anschließende wiederholte Flashchromatographie der Reaktionsgemische und Mischfraktionen mit Chloroform / Isopropanol (99:1) lieferte die Titelverbindung.
Ausbeute: 6,16 g (29 %), farbloser Schaum.
¹H-NMR (DMSO-d₆): 2.61 (1 H, dd, J = 17.6 und 5.9 Hz); 2.74 (1 H, dd, J = 17.6 und 9.8 Hz); 4.20-4.62 (3 H, m); 5.00-5.20 (4 H, m); 6.99 (1 H, dt, J = 8.8 und 2.9 Hz); 7.18-7.52 (12 H, m); 9.27 und 9.30 (1 H, 2 s); 11.25 und 11.28 (1 H, 2 s).

### Stufe 2:

### (2-{[Benzyloxycarbonyl-(2-oxo-5-thioxopyrrolidin-3-yl)amino]methyl}-5-fluorphenyl)-carbamoylsäurebenzylester

Ein Mischung aus dem Produkt aus Stufe 1 (2,1 g, 4,5 mmol) und Tetraphosphordecasulfid (4,0 g, 18 mmol, bezogen auf P₂S₅) in Tetrahydrofuran (60 mL) wurde unter Argon-Atmosphäre 40 min bei 90 °C in der Mikrowelle behandelt. Der ausgefallene Niederschlag wurde abfiltriert und mit heißem Chloroform gewaschen. Die vereinigten Filtrate wurden mit Kieselgel 60 (0,2-0,5 mm) versetzt, die Suspension im Vakuum eingeengt und der Rückstand durch Flashchromatographie mit Essigsäureethylester / Cyclohexan (1:2) gereinigt.
Ausbeute: 1,44 g (62 %), gelber Feststoff.

¹H-NMR (DMSO-d₆): 2.88-3.24 (2 H, m); 4.20-4.68 (3 H, m); 4.92-5.70 (4 H, m); 6.92-7.52 (13 H, m); 9.27 (1 H, s); 12.78 und 12.84 (1 H, 2 s).
Das 13C-NMR-Spektrum (DMSO-d₆) zeigt charakteristische Signale bei 211.3 ppm (C=S) und bei 177.7 ppm (C=O).

### Stufe 3:

### 3-(2-Amino-4-fluorbenzylamino)-5-thioxopyrrolidin-2-on-Dihydrobromid

Zu einer Suspension des Produkts aus Stufe 2 (770 mg, 1,5 mmol) in Essigsäure (2 mL) und Dichlormethan (3 mL) wurde eine 33 % Lösung von Bromwasserstoff in Essigsäure (3 mL) gegeben. Das Reaktionsgemisch wurde 3 h bei Raumtemperatur gerührt, anschließend mit Diethylether (ca. 20 mL) versetzt und über Nacht stehen gelassen. Der ausgefallene Feststoff wurde abfiltriert, mit Diethylether gewaschen und im Vakuum getrocknet.
Ausbeute: 570 mg (93 %), beigefarbene Feststoff.

Schmelzpunkt: 150-153 °C

### Stufe 4:

### 3-(7-Fluor-4H-chinazolin-3-yl)-5-thioxopyrrolidin-2-on-Hydrobromid

Eine Mischung des Produkts aus Stufe 3 (200 mg, 0,48 mmol) in Essigsäure (5 mL), Dichlormethan (5 mL) und Orthoameisensäuretriethylester (142 mg, 157 µL, 0,96 mmol) wurde bei Raumtemperatur gerührt. Nach 3 h wurde zum Reaktionsgemisch weiterer Orthoameisensäuretriethylester (71 mg, 80 µL, 0,48 mmol) zugefügt, 2 h bei Raumtemperatur gerührt, das Gemisch im Vakuum eingeengt und der Rückstand im Vakuum getrocknet.
Ausbeute: 175 mg (90 %), gelblicher Feststoff.

Schmelzpunkt: 202-208°C (Zersetzung)

### b) 3-(4H-Chinazolin-3-yl)-5-thioxopyrrolidin-2-on-Hydrobromid

### Stufe 1:

### (2-{[Benzyloxycarbonyl-(2,5-dioxopyrrolidin-3-yl)amino]methyl}phenyl)-carbamoyl-säurebenzylester

Eine Lösung von 3-(2-Amino-benzylamino)-pyrrolidin-2,5-dion (4,38 g, 20 mmol) und 4-*N,N*-Dimethylaminopyridin (Spatelspitze) in Tetrahydrofuran (40 mL) wurde mit einer Lösung von Kohlensäurebenzylester-2,5-dioxopyrrolidin-1-yl-ester (10,2 g, 41 mmol) in Tetrahydrofuran (10 mL) versetzt. Anschließend wurde die Lösung bei Raumtemperatur über Nacht gerührt und dann im Vakuum eingeengt. Der Rückstand wurde in Chloroform gelöst und mit Wasser (3x100 mL) gewaschen. Die organische Phase wurde mit Natriumsulfat getrocknet und im Vakuum eingeengt. Der Rückstand (10,7 g) wurde durch Flashchromatographie mit Essigsäureethylester / Cyclohexan (1:1) gereinigt.
Ausbeute: 6,45 g (66 %), weißer Feststoff.

### Schmelzpunkt: 61-64°C

### Stufe 2 bis 4:

Durch Ersatz von (2-{[Benzyloxycarbonyl-(2,5-dioxopyrrolidin-3-yl)-amino]-methyl}-5-fluorphenyl)-carbamidsäurebenzylester in Beispiel 16a, Stufe 2, durch (2-{[Benzyloxycarbonyl-(2,5-dioxopyrrolidin-3-yl)amino]methy}-phenyl)carbamoyl-säurebenzylester und Anwendung der im Beispiel 16a, Stufe 2 bis 4, verwendeten Verfahren wurde die Zielverbindung erhalten:
Schmelzpunkt: 215-217 °C

### Beispiel 19

### a) 3-Methyl-3-(4H-chinazolin-3-yl)-pyrrolidin-2,5-dion

### Stufe 1:

### 3-Methylpyrrol-2,5-dion

Zu einer Lösung von Citraconsäureanhydrid (5,60 g, 4,49 mL, 50 mmol) in N,N-Dimethylformamid (170 mL) wurde bei 0 °C eine Lösung von Hexamethyldisilazan (80,7 g, 104,8 mL, 500 mmol) in Methanol getropft. Die Reaktionslösung wurde 20 h bei Raumtemperatur gerührt, danach mit Methanol (100 mL) versetzt und weitere 20 min gerührt. Das Lösungsmittel wurde im Vakuum eingeengt und der Rückstand in einem Gemisch von Essigsäureethylester / Wasser (150 mL:50 ml) aufgenommen. Die organische Phase wurde abgetrennt, mit Wasser (2 × 50 mL), Natriumhydrogencarbonat-Lösung (50 mL) und erneut mit Wasser (50 mL) gewaschen und dann mit Natriumsulfat getrocknet. Das Lösungsmittel wurde eingeengt und der Rückstand im Vakuum getrocknet.
Ausbeute: 2,30 g (40 %), weißer Feststoff
Schmelzpunkt: 100-102 °C [Lit. 101-104°C (K. R. Shah, C. DeWitt Blanton, J. Org. Chem. 1982, 47, 502)].

### Stufe 2:

### 3-(2-Aminobenzylamino)-3-methylpyrrolidin-2,5-dion

Eine Lösung des Produkts aus Stufe 1 (1,94 g, 17,5 mmol) und 2-Aminobenzylamin (1,49 g, 12,2 mmol) in Essigsäureethylester (44 mL) wurden 48 h bei 50° C gerührt und danach im Vakuum eingeengt. Der Rückstand (3,8 g) wurde durch Flashchromatographie mit Chloroform / Methanol (9:1) fraktioniert.
Ausbeute: 1,07 g (37 %), weißer Feststoff.
Schmelzpunkt: 129-134 °C

### Stufe 3:

### 3-Methyl-3-(4H-chinazolin-3-yl)-pyrrolidin-2,5-dion

Eine Lösung des Produkts aus Stufe 2 (233 mg, 1.0 mmol) in Essigsäure (10 mL) wurde mit Orthoameisensäuretriethylester (296 mg, 328 µL, 2 mmol) versetzt und 5 h bei Raumtemperatur gerührt. Das Reaktionsgemisch wurde eingeengt und im Vakuum getrocknet. Der Rückstand wurde mit Diethylether versetzt, das Gemisch 20 h bei 4°C stehengelassen, der ausgefallene Feststoff abfiltriert, mit Diethylether gewaschen und im Vakuum getrocknet.
Ausbeute: 206 mg (68 %), weißer Feststoff.
Schmelzpunkt: 112-125°C

### b) 3-(5-Brom-4H-chinazolin-3-yl)-3-methyl-pyrrolidin-2,5-dion

Bei Ersatz des 2-Aminobenzylamins in Beispiel 19a, Stufe 1, durch 2-Aminomethyl-3-brom-phenylamin, und Anwendung der in Beispiel 19a, Stufe 2 und 3 beschriebenen Verfahren, wurde die Zielverbindung erhalten:
Schmelzpunkt: 124-125°C

### Beispiel 20

### a) 3-(2-Oxo-1,4-dihydro-2H-chinazolin-3-yl)-pyrrolidin-2,5-dion

### Stufe 1:

### 3-(2-Amino-benzylamino)-pyrrolidin-2,5-dion

Ein Gemisch aus Maleinimid (485 mg, 5 mmol) und 2-Aminobenzylamin (671 mg, 5.5 mmol) in Essigsäureethylester (7 mL) wurde 20 h bei Raumtemperatur gerührt. Die Lösung wurde im Vakuum eingeengt und der Rückstand durch Flashchromatographie mit Chloroform / Methanol (97:3) gereinigt.
Ausbeute: 1,09 g (100 %), zähes Öl
¹H-NMR (DMSC)-d₆): 2.40 (1 H, dd, J = 17.6 und 4.9); 2.65 (1 H, br s); 2.75 (1 H, dd, J = 17.6 und 7.8 Hz); 3.60-3.80 (3 H , m); 5.15 (2 H, s); 6.49 (1 H, dd, J = 7.8 und 7.8 Hz); 6.59 (1 H, d, J = 7.8 Hz); 6.93-6.98 (2 H, m); 11.10 (1 H, s).

### Stufe 2:

### 3-(2-Oxo-1,4-dihydro-2H-chinazolin-3-yl)-pyrrolidin-2,5-dion

Eine Lösung des Produkts aus Stufe 1 (300 mg, 1,37 mmol) und *N,N*'-Carbonyldiimidazol (447 mg, 2,74 mmol) in Tetrahydrofuran (40 mL) wurde 3 h unter Rückfluss gekocht, über Nacht bei Raumtemperatur gerührt., erneut 5 h unter Rückfluss gekocht und nochmals Carbonyldiimidazol zugegeben (447 mg, 2,74 mmol) und weitere 3 Stunden unter Rückfluss gekocht. Das Reaktionsgemisch wurde im Vakuum eingeengt und der Rückstand durch Flashchromatographie mit Chloroform / Isopropanol (95:5) gereinigt.
Ausbeute: 264 mg (79 %), weißer Feststoff.

Schmelzpunkt: 251 °C

### b) 3-(7-Fluor-2-oxo-1,4-dihydro-2H-chinazolin-3-yl)-pyrrolidin-2,5-dion

Bei Ersatz des 2-Aminobenzylamins durch 2-(Aminomethyl)-5-fluorbenzylamin in Beispiel 20a, Stufe 1, sowie Anwendung der in Beispiel 20a, Stufe 1 bis 2, beschriebenen Verfahren, erhielt man die Titelverbindung.

Schmelzpunkt: 283 -285 °C

### c) 3-(5,7-Difluor-2-oxo-1,4-dihydro-2H-chinazolin-3-yl)-pyrrolidin-2,5-dion

Bei Ersatz des 2-Aminobenzylamins durch 2-(Aminomethyl)-3,5-difluorbenzylamin in Beispiel 20a, Stufe 1, sowie Anwendung der in Beispiel 20a, Stufe 1 bis 2, beschriebenen Verfahren, erhielt man die Titelverbindung.

### d) 3-(2-oxo-1,4-dihydro-2H-chinazolin-3-yl)-4-phenylpyrrolidin-2,5-dion

### Stufe 1:

### 3-Phenylpyrrol-2,5-dion

Eine Lösung von Anilin (4,66 g, 50 mmol) in konzentrierter Salzsäure (15 mL) und Wasser (10 mL) wurde unter kräftigem Rühren auf 0 °C abgekühlt und mit Eis (10 g) versetzt. Zur gebildeten Emulsion wurde eine Lösung von Natriumnitrit 3,45 g (50 mmol) in Wasser (15 mL) so zugetropft, dass die Innentemperatur 5 °C nicht überstieg. Die kalte Suspension wurde in einer Portion zu einer auf 0 °C gekühlten Suspension von Maleinimid (5,82 g. 60 mmol) in Aceton (15 mL) gegeben. Der pH-Wert dieser Mischung wurde durch Zugabe von festem Natriumacetat auf 3 eingestellt. Anschließend wurde Kupfer(II)-chlorid (1.0 g) und danach soviel Aceton zugegeben, bis eine klare Lösung entstanden war. Nach 30 min Rühren bei 0 °C wurde auf 40 °C erwärmt, wobei Stickstoffentwicklung begann. Bei dieser Temperatur wurde über Nacht gerührt, bevor Aceton im Vakuum bei einer Badtemperatur von maximal 20 °C abgezogen wurde. Der Rückstand wurde mit Essigsäureethylester (dreimal 50 moL) extrahiert. Die vereinigten organischen Extrakte wurden mit Natriumsulfat getrocknet, im Vakuum eingeengt und der Rückstand durch Flashchromatographie mit Cyclohexan 1 Essigsäureethylester (4:1) gereinigt.
Ausbeute: 1,48 g (17 %), gelber Feststoff.

Schmelzpunkt: 160-164 °C

### Stufe 2:

### 3-(2-Aminobenzylamino)-4-phenylpyrrolidin-2,5-dion

Zu einer Lösung des Produkts aus Stufe 1 (1,20 g, 6,9 mmol) in Essigsäureethylester (20 mL) wurde 2-Aminobenzylamin (843 mg, 6.9 mmol) gegeben und 24 h bei Raumtemperatur gerührt. Der ausgefallene Feststoff wurde abfiltriert und im Vakuum getrocknet. Ausbeute: 403 mg (32 %), weißer Feststoff.

Schmelzpunkt: 167-169 °C

### Stufe 3:

### 3-(2-4Oxo-1,4-dihydro-2H-chinazolin-3-yl)-4-phenylpyrrolidin-2,5-dion

Zu einer unter Rückfluss kochenden Lösung von *N,N*'-Carbonyldiimidazol (1,21 g, 7,44 mmol) in Tetrahydrofuran (100 mL) wurde portionsweise das Produkt aus Stufe 2 (1,10 g, 3,72 mmol) gegeben. Das Reaktionsgemisch wurde anschließend 6 h unter Rückfluss erhitzt und nach dem Abkühlen auf Raumtemperatur im Vakuum eingeengt. Der Rückstand wurde mittels Flashchromatographie mit Chloroform / Methanol (9:1) und anschließende Flashchromatographie mit Essigsäureethylester / Cyclohexan (2:1) gereinigt.
Ausbeute: 230 mg (19 %), gelber Feststoff, der an der Luft zerfließt.

Schmelzpunkt: 226-236 °C

### e) 3-(2-Thio-1,4-dihydro-2H-chinazolin-3-yl)-pyrrolidin-2,5-dion

Bei Ersatz des Carbonyldiimidazols in Beispiel 20a, Stufe 2, durch *N,N*'-Thiocarbonyldiimidazol, sowie Anwendung der in Beispiel 20a, Stufe 1 bis 2, beschriebenen Verfahren, erhielt man die Titelverbindung.

Schmelzpunkt: 235-239 °C

### Beispiel 21

### 3-(2-(Methylthio)-4H-chinazolin-3-yl)pyrrolidin-2,5-dion Hydroiodid

Eine Lösung des Produkts aus Beispiel 20 d (194 mg, 0,74 mmol) in absolutem Tetrahydrofuran (20 mL) wurde mit Methyliodid (527 mg, 0,231 mL, 3.7 mmol) versetzt und 7 d bei Raumtemperatur gerührt. Die Reaktionslösung wurde anschließend im Vakuum eingeengt.
Ausbeute: 300 mg (100 %), brauner Feststoff
¹H-NMR (DMSO-d₆): 2.60 (3 H, s); 2.80-3.05 (2 H, m); 4.18-4.30 (1 H, m); 4.58-4.75 (1 H, m); 5.50 (1 H, br s); 8.95-7.18 (3 H, m); 7.20-7.36 (1 H, m); 11.69 (1 H, s).

### Beispiel 22

### 3-(2-(Dimethylamino)-4-chinazolin-3-yl)pyrrolidin-2,5-dion Hydroiodid

Eine Lösung des Produkts aus Beispiel 21 (138 mg, 0,34 mmol) in absolutem Tetrahydrofuran (10 mL) wurde mit 2 M Dimethylamin-Lösung in Tetrahydrofuran (0,17 mL, 0,34 mmol) versetzt und 2 d bei Raumtemperatur gerührt. Die Reaktionslösung wurde anschließend im Vakuum eingeengt.
Ausbeute: 121 mg (89 %), brauner Feststoff.

¹H-NMR (DMS-d₆): 2.45 (3 H, s); 2.54 (3 H, s); 2.90 (2 H, dd, J = 11.7 und 5.9 Hz); 4.05 (1 H, d, J = 13.7 Hz); 4.53 (1 H, dd, J,= 13.7 Hz); 5.34 (1 H, dd, J = 8.8 und 5.9 Hz); 6.80-7.05 (3 H, m); 7.12-7.20 (1 H, m); 8.01-8.30 (1 H, br s); 11.58 (1 H, s).

### Beispiel 23

### 3-(4H-Chinazolin-3-yl)-pyrrolidin-2-on

### Stufe 1:

### 3-Aminopyrrolidin-2-on

Die Synthese erfolgte nach Literatur (R. Pellegata et al., Synthesis, 1978, 614-616).

### Stufe 2:

### 3-(4H-Chinazolin-3-yl)-pyrrolidin-2-on

Eine Lösung des Produkts aus Stufe 1 (1.00 g, 10mmol) in absolutem 1,2-Dichlorethan (150 mL) wurde mit 2-Formylamino-benzaldehyd (1,49 g, 10 mmol, Verfahren zur Herstellung siehe WO 03/053956 A1) versetzt und 15 min bei Raumtemperatur gerührt. Die Mischung wurde mit Essigsäure (601 mg, 572 µL, 10 mmol) und Natriumtriacetoxyborhydrid (3,04 g, 14,3 mmol) versetzt und über Nacht bei Raumtemperatur gerührt. Das Reaktionsgemisch wurde mit 2 N Natronlauge auf pH-9 eingestellt. Nach Phasentrennung wurde die wässrige Phase mit Dichlormethan (zweimal 20 mL) extrahiert. Die vereinigten organischen Phasen wurden mit Natriumsulfat getrocknet und im Vakuum eingeengt. Der Rückstand wurde durch Flashchromatographie mit Chloroform / Methanol (9:1) und Methanol gereinigt. Die durch Methanol eluierte Fraktion (123 mg) wurde nochmals mittels Flashchromatographie mit Chloroform / Methanol (3:7) gereinigt.
Ausbeute: 78 mg (3,6 %), gelblicher Feststoff.
Schmelzpunkt: 222-225 °C

### Beispiel 24

### a) 3-(4H-Chinazolin-3-yl)-pyrrolidin-2-on

Eine Lösung von 3,4-Dihydrochinazolin (505 mg, 2 mmol), Maleinimid (292 mg, 3 mmol) und Triethylamin (607 mg, 832 µL, 6 mmol) in *N*,*N*-Dimethylformamid (10 mL) wurde über Nacht bei Raumtemperatur gerührt. Anschließend wurde das Gemisch im Vakuum eingeengt, der Rückstand in Toluol aufgenommen, erneut eingeengt und durch Flashchromatographie mit Essigsäureethylester / Methanol (2:1) gereinigt.
Ausbeute: 341 mg (74 %), beigefarbener Schaum.

Das ¹H-NMR-Spektrum war mit dem des Beispiels 11a identisch.

### b) 3-(2-Methyl-4H-chinazolin-3-yl)-pyrrolidin-2,5-dion

Bei Ersatz des 3,4-Dihydrochinazolin durch 2-Methyl-3,4-dihydrochinazolin in Beispiel 24a, sowie Anwendung der dort beschriebenen Verfahren, erhielt man die Titelverbindung.

Das ¹H-NMR-Spektrum war mit dem des Beispiels 15b identisch.

### Untersuchung der immunmodulatorischen Wirksamkeit

Stimulation humaner Monozyten mit Lipopolysaccharid zur Sekretion von IL-10 und IL-12:
Humane Monozyten wurden aus peripheren Blut-mononucleären Zellen (PBMC), die mittels einer Ficoll-Dichtegradientenzentrifugation von heparinisiertem Vollblut gewonnen wurden, isoliert. Dazu wurden die PBMC mit einem monoklonalen Antikörper inkubiert, der gegen das Monozyten-spezifische Oberflächenmolekül CD14 gerichtet ist und an den superparamagnetische Microbeads (Miltenyi Biotech. Bergisch Gladbach) gekoppelt sind. Zur positiven Selektion der markierten Monozyten aus dem Zellgemisch der PBMC wurde die Gesamtzellsuspension auf eine Säule mit ferromagnetischer Trägermatrix aufgebracht und diese in ein Magnetfeld gestellt. Dadurch wurden die Zellen, die mit Microbeads beladen waren, an die Trägermatrix gebunden, unmarkierte Zellen passierten die Säule und wurden verworfen. Nach Herausnehmen der Matrix aus dem Magnetfeld wurden die Antikörper-beladenen Zellen durch Spülen der nun entmagnetisierten Säule mit Puffer eluiert. Die Reinheit dieser so erhaltenen CD14-positiven Monozytenpopulation beträgt etwa 95-98%. Diese Monozyten wurden in einer Dichte von 10⁶ Zellen/ml Kulturmedium (RPMI, supplementiert mit 10% foetalem Kälberserum) mit den in DMSO-gelösten Prüfsubstanzen für eine Stunde bei 37°C und 5% CO₂ inkubiert. Anschließend wurde 10 µg/mt LPS aus E. coli zugegeben. Nach 24 Stunden wurden zellfreie Kulturüberstände genommen und auf den Gehalt an IL-10 sowie IL-12 getestet.

Die Konzentration von IL-12 und IL-10 in den Zellkulturüberständen wurde mittels Sandwich-ELISAs unter Verwendung zweier anti-IL-12 bzw. IL-10 monoklonaler Antikörper (Biosource Europe, Fleurus, Belgien) bestimmt. Eine Referenzstandardkurve mit humanem IL-10 bzw. IL-12 wurde eingeschlossen. Das Detektionslimit der ELISAs betrug 15 pg/ml.

**Tabelle 1: Einfluss der Prüfsubstanzen im Vergleich zu Thalidomid und die Verbindung XXVIII (V1 → Vergleichsbeispiel 1) auf die IL-12 und IL-10 Produktion von LPS-aktivierten Monozyten**

| Beispiel-Nr. | Hemmung der IL-12 Produktion IC₅₀ (ng/ml) | Steigerung der IL-10 Produktion EC₂₀₀ (ng/ml) |
|---|---|---|
| 1 | 1500 | 4000 |
| 2 | 1400 | 1000 |
| 3 | 130 | 400 |
| 4 | 107 | 620 |
| 8b | 7300 | > 50000 |
| 8c | 4000 | > 50000 |
| 8d | 715 > | 50000 |
| 8g | 620 | 1000 |
| 9b | 15000 | 16000 |
| 10a | 303 | 16000 |
| 10c | 1172 | > 50000 |
| 11a | 600 | 5000 |
| 11b | 514 | 5600 |
| 12a | 1800 | > 50000 |
| 14 | 5706 | 20000 |
| 15b | 4004 > | 50000 |
| 15bb | 419 | 5600 |
| 15cc | 6000 | 20000 |
| 15e | 520 | 3000 |
| 15h | 4000 | > 50000 |
| 15i | 884 | 15000 |
| 15j | 521 | 5000 |
| 151 | 2000 | 5600 |
| 15u | 1800 | > 50000 |
| XXVIII = V1 | > 50000 | Keine Steigerung |
| Thalidomid | 70 | 20000 |

Die in 3-Position heterocyclisch substituierten Pyrrolidin(thi)one der in Formel I beschriebenen Grundstruktur supprimierten in potenter Weise die IL-12 Produktion LPS-aktivierter Monozyten in einer konzentrationsabhängigen Weise. Interessanterweise wurde die IL-10 Produktion gesteigert. Die maximale IL-12 Hemmung sowie die IC₅₀ Werte liegen deutlich über denen von Verbindung XXVIII (Vergleichsbeispiel 1). Die wirksamsten Verbindungen sind die, deren aromatischer Ring eine Fluor-Substitution in Position 7 aufweist bzw. Fluor-Substitutionen in den Positionen 5 und 7 aufweist. Die Wirkstärke ist der von Thalidomid vergleichbar.

### Untersuchungen zur Pharmakokinetik

### Abbildung Plasmakonzentrations-Zeitverläufe von Beispiel 3 und dessen Piperidin-2,6-dion Analogon nach intravenöser Gabe von je 10 mg/kg an Ratten

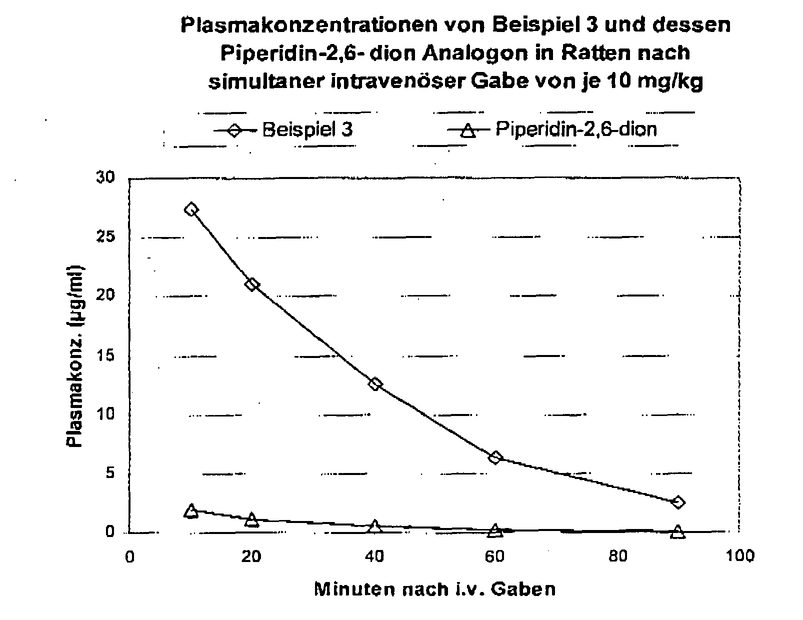

Beispiel 3 und dessen Piperidin-2,6-dion Analogon wurden zusammen in einer Konzentration von je 5 mg/mL in physiologischer Kochsalzlösung gelöst. Volumina von 2 mL/kg wurden Ratten durch die Schwanzvene intravenös verabreicht. Nach definierten Zeiten wurden Blutproben aus dem retro-orbitalen Plexus genommen und Plasma gewonnen. Plasmakonzentrationen von beiden Verbindungen wurden mit validen bioanalytischen Methoden bestimmt. Das Verteilungsvolumen von Beispiel 3 wurde als 0.26 Ukg, das des Piperidin-2,6-dion Analogons als 1.0 L/kg geschätzt.

## Patentansprüche

1. In 3-Position heterocyclisch substituierte Pyrrolidin(thi)one der allgemeinen Formel (I), 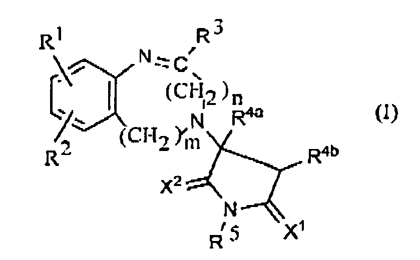 in denen
R¹ und R² gleich oder verschieden voneinander, H, Br, Cl, F, I, CF₃, OH, NO₂, NR^{5'}R^{6'}, Alkyl, Alkoxy, Alkylthio, Alkylsulfinyl, Alkylsulfonyl, Aryl, Heteroaryl, jeweils verzweigt oder unverzweigt sein können oder zusammengenommen einen ankondensierten Benzolring bedeuten, wobei die Ringe gegebenenfalls mit R¹ und/oder R² substituiert und R¹ und R² wie oben definiert sind, und R^{5'} und R^{6'} ausgewählt sind aus H, Alkyl- oder Acylresten
R³ H, die Methylgruppe oder, für den Fall, dass eine C-N-Einfachbindung vorliegt, zusammen mit dem C-Atom eine Carbonyl-Gruppe darstellt,
R^{4a} und/oder R^{4b} H, Alkyl, Aryl oder Heteroaryl bedeutet,
R⁵ für H, Aryl, Heteroaryl, Alkyl, eine CH₂-OH- Gruppe oder einen Rest CH₂-NR⁶R⁷, in dem R⁶ und R⁷ gleich oder verschieden sind und eine Alkylgruppe mit 1-6 C-Atomen (geradkettig oder verzweigt) oder zusammen mit dem N-Atom einen Pyrrolidin-, Piperidin-, Hexamethylenimin- oder Morpholinring bedeuten, steht,
X¹ und/oder X² für O oder S stehen und die verbleibenden X¹ oder X² H₂ bedeuten,
n 0 oder 1 und
m 1 oder 2 bedeutet,
und deren reine Enantiomere oder nichtracemische Enantiomerengemische Racemate, Diasteremere oder Diastereomerengemische sowohl in Form ihrer freien Basen als auch von Salzen mit physiologisch verträglichen organischen oder anorganischen Säuren.

2. Verbindungen nach Anspruch 1, **dadurch gekennzeichnet, dass** sie eine C=N-Doppelbindung aufweisen und R¹ und R², gleich oder verschieden voneinander, H, Br, Cl, F, CF₃, NO₂, NH₂, C₁₋₃-Alkyl, C₁₋₃-Alkoxy, oder zusammengenommen einen ankondensierten Benzolring bedeuten, R³ für H oder eine Methylgruppe , R^{4a} für H oder eine Methylgruppe, R^{4b} für H oder eine Phenylgruppe und R⁵ für H oder Methyl steht, X¹ und X² für O stehen und n = 0 und m = 1 ist.

3. Verbindungen nach einem der Ansprüche 1 oder 2, **dadurch gekennzeichnet, dass** sie eine C=N-Doppelbindung aufweisen und R¹ und R², gleich oder verschieden voneinander, H, Cl, oder F bedeuten, R³, R^{4a}, R^{4b} und R⁵ Wasserstoff bedeuten, X¹ und X² für O stehen und n = 0 und m = 1 ist.

4. Verbindungen nach Anspruch 1:
3-(5-Chlor-7-fluor-4*H-*chinazolin-3-yl)pyrrolidin-2,5-dion **(1)**
3-(7-Chlor-5-fluor-4*H-*chinazolin-3-yl)-pyrrolidin-2,5-dion **(2)**
3-(7-Fluor-4*H-*chinazolin-3-yl)-pyrrolidin-2,5-dion und dessen Hydrochlorid **(3)**
3-(5,7-Difluor-4*H-*chinazolin-3-yl)-pyrrolidin-2,5-dion und dessen Hydrochlorid **(4)**
3-(5,7-Dichlor-4*H-*chinazolin-3-yl)-pyrrolidin-2,5-dion **(5)**
3-(5-Brom-4*H-*chinazolin-3-yl)-pyrrolidin-2,5-dion **(6)**
3-(7-Trifluormethyl-4*H-*chinazolin-3-yl)pyrrolidin-2,5-dion **(7)**
3-(5,8-Dichlor-4*H-*chinazolin-3-yl)-pyrrolidin-2,5-dion **(8a)**
3-(5-Methyl-4*H-*chinazolin-3-yl)-pyrrolidin-2,5-dion **(8b)**
3-(4*H-*Benzo[g]chinazolin-3-yl)-pyrrolidin-2,5-dion **(8c)**
3-(6,7-Difluor-4*H-*chinazolin-3-yl)-pyrrolidin-2,5-dion **(8d)**
3-(6,8-Dichlor-4*H*-chinazolin-3-yl)-pyrrolidin-2,5-dion **(8e)**
3-(6-Nitro-4*H*-chinazolin-3-yl)-pyrrolidin-2,5-dion **(8f)**
3-(7-Chlor-4*H*-chinazolin-3-yl)-pyrrolidin-2,5-dion **(8g)**
3-(7-Nitro-4*H*-chinazolin-3-yl)-pyrrolidin-2,5-dion **(8h)**
3-(8-Brom-6-methyl-4*H-*chinazolin-3-yl)-pyrrolidin-2,5-dion **(8i)**
3-(8-Chlor-4*H*-chinazolin-3-yl)-pyrrolidin-2,5-dion **(8j)**
3-(8-Methoxy-4*H*-chinazolin-3-yl)-pyrrolidin-2,5-dion **(8k)**
3-(6-Benzyloxy-4*H*-chinazolin-3-yl)-pyrrolidin-2,5-dion **(8l)**
3-(5,6-Dichlor-4*H*-chinazolin-3-yl)-pyrrolidin-2,5-dion **(9a)**
3-(7-Methoxy-4*H*-chinazolin-3-yl)-pyrrolidin-2,5-dion **(9b)**
3-(5-Chlor-4*H*-chinazolin-3-yl)-pyrrolidin-2,5-dion **(10a)**
3-(6,7-Dimethoxy-4*H*-chinazolin-3-yl)-pyrrolidin-2,5-dion **(10b)**
3-(6-Chlor-4*H*-chinazolin-3-yl)-pyrrolidin-2,5-dion **(10c)**
3-(4*H*-Chinazolin-3-yl)-pyrrolidin-2,5-dion **(11a)**
3-(5-Fluor-4*H*-chinazolin-3-yl)-pyrrolidin-2,5-dion **(11b)**
3-(8-Trifluormethyl-4*H-*chinazolin-3-yl)-pyrrolidin-2,5-dion **(11c)**
1-Methyl-3-(4*H-*chinazolin-3-yl)-pyrrolidin-2,5-dion **(11d)**
7-Fluor-1-methyl-3-(4*H-*chinazolin-3-yl)-pyrrolidin-2,5-dion **(11e)**
3-(5-Methoxy-4*H-*chinazolin-3-yl)-pyrrolidin-2,5-dion **(12a)**
3-(5-Ethoxy-4*H-*chinazolin-3-yl)-pyrrolidin-2,5-dion **(12b)**
3-(5-Pentyloxy-4*H-*chinazolin-3-yl)-pyrrolidin-2,5-dion **(12c)**
3-(5-Benzyloxy-4*H-*chinazolin-3-yl)-pyrrolidin -2,5-dion **(12d)**
3-(5-Isopropoxy-4*H-*chinazolin-3-yl)-pyrrolidin-2,5-dion **(12e)**
3-(5-(2-Methoxyethoxy)-4*H-*chinazolin-3-yl)-pyrrolidin -2,5-dion **(12f)**
3-(5-Ethansulfonyl-4*H-*chinazolin-3-yl)-pyrrolidin-2,5-dion **(13a)**
3-(5-Ethansulfinyl-4*H-*chinazolin-3-yl)-pyrrolidin-2,5-dion **(13c)**
3-(5-Ethylthio-4*H-*chinazolin-3-yl)-pyrrolidin-2,5-dion **(13b)**
3-(5-Nitro-4*H-*chinazolin-3-yl)-pyrrolidin-2,5-dion **(14)**
1-Methyl-3-(2-methyl-4*H-*chinazolin-3-yl)-pyrrolidin-2,5-dion **(15a)**
3-(2-Methyl-4*H-*chinazolin-3-yl)-pyrrolidin-2,5-dion **(15b)**
3-(5,6-Dichlor-2-methyl-4*H-*chinazolin-3-yl)-pyrrolidin-2,5-dion **(15c)**
3-(5,7-Dichlor-2-methyl-4*H-*chinazolin-3-yl)-pyrrolidin-2,5-dion **(15d)**
3-(5,7-Difluor-2-methyl-4H-chinazolin-3-yl)-pyrrolidin-2,5-dion und dessen Hydrochlorid **(15e)**
3-(5,8-Dichlor-2-methyl-4*H-*chinazolin-3-yl)-pyrrolidin-2,5-dion **(15f)**
3-(5-Benzyloxy-2-methyl-4*H-*chinazolin-3-yl)-pyrrolidin-2,5-dion **(15g)**
3-(5-Brom-2-methyl-4*H-*chinazolin-3-yl)-pyrrolidin-2,5-dion **(15h)**
3-(5-Chlor-2-methyl-4*H-*chinazolin-3-yl)-pyrrolidin-2,5-dion **(15i)**
3-(5-Chlor-7-fluor-2-methyl-4*H-*chinazolin-3-yl)-pyrrolidin-2,5-dion und dessen Hydrochlorid **(15j)**
3-(5-Ethoxy-2-methyl-4*H*-chinazolin-3-yl)-pyrrolidin-2,5-dion**(15k)**
3-(5-Fluor-2-methyl-4*H*-chinazolin-3-yl)-pyrrolidin-2,5-dion**(15l)**
3-(5-Isopropoxy-2-methyl-4*H*-chinazolin-3-yl)-pyrrolidin-2,5-dion **(15m)**
3-(2,5-Dimethyl-4*H*-chinazolin-3-yl)-pyrrolidin-2,5-dion **(15n)**
3-(5-Methoxy-2-methyl-4*H*-chinazolin-3-yl)-pyrrolidin-2,5-dion **(15o)**
3-(2-Methyl-5-nitro-4*H-*chinazolin-3-yl)-pyrrolidin-2,5-dion **(15p)**
3-(2-Methyl-5-pentyloxy-4*H*-chinazolin-3-yl)-pyrrolidin-2,5-dion **(15q)**
3-(5-Ethylthio-2-Methyl-4*H*-chinazolin-3-yl)-pyrrolidin-2,5-dion **(15r)**
3-(5-Ethansulfonyl-2-methyl-4*H*-chinazolin-3-yl)-pyrrolidin-2,5-dion **(15s)**
3-(2-Methyl-4*H*-benzo[g]chinazolin-3-yl)-pyrrolidin-2,5-dion **(15t)** 3-(6,7-Difluor-2-methyl-4*H*-chinazolin-3-yl)-pyrrolidin-2,5-dion **(15u)**
3-(6,7-Dimethoxy-2-methyl-4*H*-chinazolin-3-yl)-pyrrolidin-2,5-dion **(15v)**
3-(6,8-Dichlor-2-methyl-4*H*-chinazolin-3-yl)-pyrrolidin-2.5-dion **(15w)**
3-(6-Benzyloxy-2-methyl-4*H*-chinazolin-3-yl)-pyrrolidin-2,5-dion **(15x)**
3-(6-Chlor-2-methyl-4*H*-chinazolin-3-yl)-pyrrolidin-2,5-dion **(15y)**
3-(2-Methyl-6-nitro-4*H*-chinazolin-3-yl)-pyrrolidin-2,5-dion **(15z)**
3-(2-Methyl-7-trifluormethyl-4*H*-chinazolin-3-yl)-pyrrolidin-2,5-dion **(15aa)**
3-(7-Chlor-2-methyl-4*H*-chinazolin-3-yl)-pyrrolidin-2,5-dion **(15bb)**
3-(7-Fluor-2-methyl-4*H*-chinazolin-3-yl)-pyrrolidin-2,5-dion **(15cc)**
3-(7-Methoxy-2-methyl-4*H*-chinazolin-3-yl)-pyrrolidin-2,5-dion **(15dd)**
3-(2-Methyl-7-nitro-4*H*-chinazolin-3-yl)-pyrrolidin-2,5-dion **(15ee)**
3-(8-Brom-2,6-dimethyl-4*H*-chinazolin-3-yl)-pyrrolidin-2,5-dion **(15ff)** 3-(2-Methyl-8-trifluormethyl-4*H*-chinazolin-3-yl)-pyrrolidin-2,5-dion **(15gg)**
3-(8-Chlor-2-methyl-4*H*-chinazolin-3-yl)-pyrrolidin-2,5-dion **(15hh)**
3-(8-Methoxy-2-methyl-4*H-*chinazolin-3-yl)-pyrrolidin-2,5-dion **(15ii)**
3-(5-(2-Methoxyethoxy)-2-methyl-4*H-*chinazolin-3-yl) pyrrolidin-2,5-dion **(15jj)**
3-(7-Amino-4*H*-chinazolin-3-yl)-pyrrolidin-2,5-dion-Hydrochlorid **(16a)**
3-(7-Amino-2-methyl-4*H*-chinazolin-3-yl)-pyrrolidin-2,5-dion Hydrochlorid **(16b)**
3-(6-Amino-4*H*-chinazolin-3-yl)-pyrrolidin-2,5-dion Hydrochlorid **(16c)**
3-(6-Amino-2-methyl-4*H*-chinazolin-3-yl)-pyrrolidin-2,5-dion-Hydrochlorid **(16d)**
3-(5-Amino-4*H-*chinazolin-3-yl)-pyrrolidin-2,5-dion Hydrochlorid **(16e)**
3-(5-Amino-2-methyl-4*H-*chinazolin-3-yl)-pyrrolidin-2,5-dion Hydrochlorid **(16f)**
3-(5-Hydroxy-4*H-*chinazolin-3-yl)-pyrrolidin-2,5-dion Hydrochlorid **(17a)**
3-(6-Hydroxy-2-methyl-4*H-*chinazolin-3-yl)-pyrrolidin-2,5-dion Hydrochlorid **(17b)**
3-(5-Hydroxy-2-methyl-4*H*-chinazolin-3-yl)-pyrrolidin-2,5-dion Hydrochlorid **(17c)**
3-(5-Hydroxy-2-methyl-4*H*-chinazolin-3yl)-pyrrolidin-2,5-dion Hydrochlorid **(17d)**
3-(7-Fluor-4*H*-chinazolin-3-yl)-5-thioxopyrrolidin-2-on-Hydrobromid **(18a)**
3-(4*H-*Chinazolin-3-yl)-5-thioxopyrrolidin-2-on-Hydrobromid **(18b)**
3-Methyl-3-(4*H*-chinazolin-3-yl)-pyrrolidin-2,5-dion **(19a)**
3-(5-Brom-4*H*-chinazolin-3-yl)-3-methyl-pyrrolidin-2,5-dion **(19b)**
3-(2-Oxo-1,4-dihydro-2*H*-chinazolin-3-yl)-pyrrolidin-2,5-dion **(20a)**
3-(7-Fluor-2-oxo-1,4-dihydro-2*H*-chinazolin-3-yl)-pyrrolidin-2,5-dion **(20b)**
3-(2-Oxo-1,4-dihydro-2*H*-chinazolin-3-yl)-4-phenylpyrrolidin-2,5-dion **(20c)**
3-(2-Thio-1,4-dihydro-2*H-*chinazolin-3-yl)-pyrrolidin-2,5-dion **(20d)**
3-(2-(Methylthio)-4*H-*chinazolin-3-yl)pyrrolidin-2,5-dion Hydroiodid **(21)**
3-(2-(Dimethylamino)-4-chinazolin-3-yl)pyrrolidin-2,5-dion Hydroiodid **(22)**
3-(4*H-*Chinazolin-3-yl)-pyrrolidin-2-on **(23).**

5. Verfahren zur Herstellung von in 3-Position heterocyclisch substituierten Pyrrolidin(thi)onen der allgemeinen Formel (I) nach Anspruch 1, wobei man
a) für die Herstellung der Verbindungen mit x¹ und x² gleich Sauerstoff 2-Aminobenzylamine der allgemeinen Formel (II), 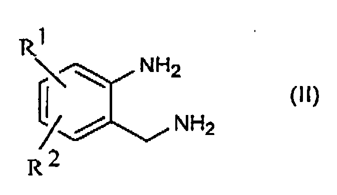 in der R¹ und R² wie oben definiert sind, zunächst mit Pyrrol-2,5-dionen der allgemeinen Formel (III), 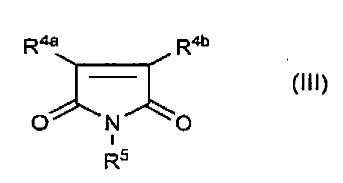 in der R^{4a} und R^{4b} wie oben definiert sind, zu Aminen der allgemeinen Formel (IVa) umsetzt, 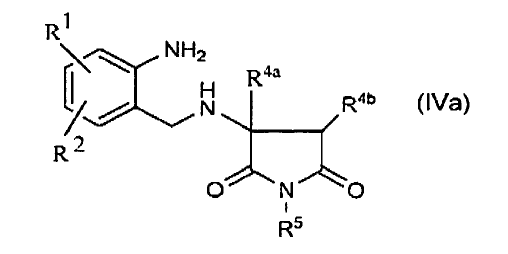 wobei gegebenenfalls anstelle der Pyrrol-2,5-dione 3-Brompyrrolidin-2,5-dione der allgemeinen Formel (IIIa) 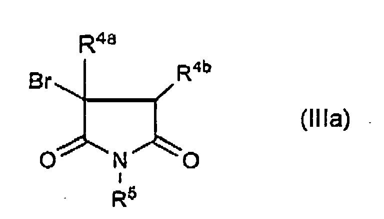 eingesetzt werden, und
b) für die Herstellung der Verbindungen, in denen mindestens einer der Reste X¹ und X² Schwefel bedeutet,
Verbindungen der allgemeinen Formel (IVb), in denen mindestens ein O aus Formel (IVa) durch S ersetzt ist 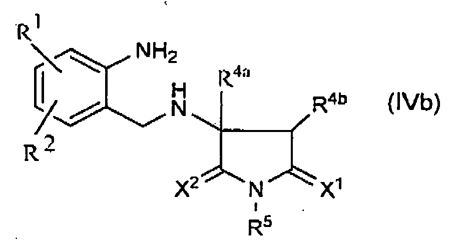 herstellt, vorzugsweise nach Schutz der beiden Stickstoffatome, die nicht mit R⁵ verbunden sind, mit dem Fachmann bekannten Thionierungsreagenzien aus (IVa) durch Austausch mindestens eines O durch X¹ bzw. X² gleich S und nachfolgender Entschützung,
wobei durch anschließend Reaktion von (IVa) oder (IVb) mit Verbindungen der allgemeinen Formel (V),
R³ - C(OR^{y})₃ (V)
in der R³ Wasserstoff oder die Methylgruppe bedeutet und R^{y} für einen geradkettigen oder verzweigten C₁-C₄-Alkylrest steht, oder mit Amidinsalzen der allgemeinen Formel (VI), 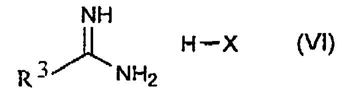 in der R³ wie oben definiert ist und X das Anion einer geeigneten Säure darsteilt, oder mit Carbonsäureestern der allgemeinen Formel (Vb) 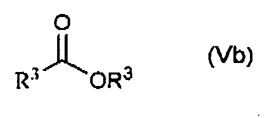 dann sowohl aus (IVa) als auch aus (IVb) Verbindungen der allgemeinen Formel (I) mit der entsprechenden Bedeutung von R³ hergestellt werden.

6. Verfahren nach Anspruch 5, **dadurch gekennzeichnet, dass** Verbindungen der allgemeinen Formel (I), in der R³ zusammen mit dem C-Atom ein geminal substituiertes C-Atom darstellt, durch Reaktion von (IVa) oder (IVb) mit Ketonen der allgemeinen Formel (Vc), 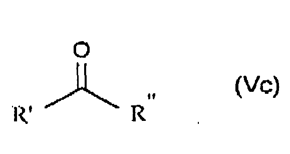 in der R' und R" unabhängig voneinander für einen Alkyl-, Aryl- oder Heteroarylrest stehen, hergestellt werden.

7. Verfahren nach Anspruch 5, **dadurch gekennzeichnet, dass** Verbindungen der allgemeinen Formel (I), in der R³ zusammen mit dem C-Atom eine Carbonylgruppe darstellt, durch Reaktion von (IVa) oder (IVb) mit C1-Bausteinen der allgemeinen Formel (VII), 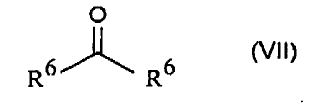 in der R⁶ für Cl, den Imidazol-1-yl-rest, eine C₁-C₄-Alkoxy-, eine Phenyloxy- oder eine mit der Nitrogruppe, Chlor oder Fluor substituierte Phenyloxygruppe oder eine Thiomethylgruppe steht, oder durch Reaktion von (IVa) oder (IVb) mit C₁-C₄-Alkyl-, Phenyl- oder substituierten Phenylestern, vorzugsweise 4-Nitro-, 4-Chlor-oder 4-Fluorphenylestern, der Chlorameisensäure hergestellt werden.

8. Verfahren nach Anspruch 5, **dadurch gekennzeichnet, dass** Verbindungen der allgemeinen Formel (I), in der R³ zusammen mit dem C-Atom eine Carbonylgruppe darstellt, durch Reaktion von (IVa) oder (IVb) mit einem C1-Baustein der allgemeinen Formel VIII,
C(OR⁷)₄ (VIII)
in der R⁷ für den Methyl- oder Ethylrest steht, hergestellt werden.

9. Verfahren nach Anspruch 5, **dadurch gekennzeichnet, dass** die Umsetzung der Aminobenzylamine der allgemeinen Formel (II) mit den Pyrrol-2,5-dionen der allgemeinen Formel (III) in inerten Lösungsmitteln, vorzugsweise Essigsäureethylester, bei Raumtemperatur erfolgt.

10. Verfahren nach Anspruch 5, **dadurch gekennzeichnet, dass** die Reaktion der Verbindungen der allgemeinen Formel (IVa und IVb) mit den Verbindungen der allgemeinen Formel (V) entweder ohne Lösungsmittel oder in einer organischen Carbonsäure, vorzugsweise Essigsäure, in einem Temperaturbereich von 10 bis 150 °C durchgeführt wird.

11. Verfahren zur Herstellung von in 3-Position heterocyclisch substituierten Pyrrolidin(thi)onen der allgemeinen Formel (I) nach Anspruch 1, wobei man zunächst eine Aminoverbindung der allgemeinen Formel (IX) 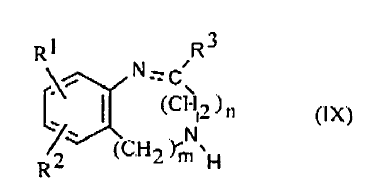 mit einem Pyrrol-2,5-dion der allgemeinen Formel (III) oder einem 3-Brompyrrolidin-2,5-dionderivat der allgemeinen Formel (IIIa) 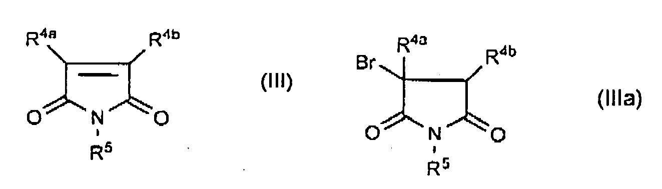 zu einer Verbindung der allgemeinen Formel (IA) alkyliert, 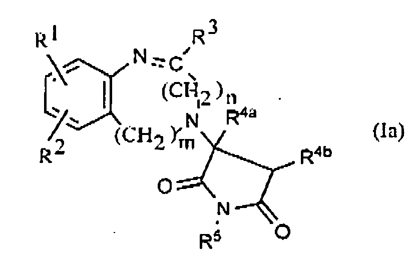 wobei in den Verbindungen (Ia), (II) und (III) die Reste R¹ bis R^{4b} wie oben definiert sind, und anschließend, wenn R⁵ nicht für Wasserstoff stehen soll, diesen Rest durch Umsetzung mit Formaldehyd, gegebenenfalls zusammen mit einem Amin der allgemeinen Formel HNR⁵R⁷, in der R⁶ und R⁷ wie oben definiert sind, einführt, sowie gegebenenfalls für X¹ und/oder X² gleich S das oben beschriebene Schwefelungsverfahren anwendet.

12. Verfahren zur Herstellung von in 3-Position heterocyclisch substituierten Pyrrolidin(thi)onen der allgemeinen Formel (I) nach Anspruch 1, wobei man eine Aminoverbindung der Formel (II) zunächst mit einem 3-Brompyrrolidin-2-onderivat der Formel (X) 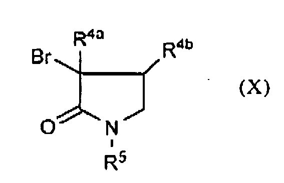 zu einer Verbindung der allgemeinen Formel (Ib) alkyliert, 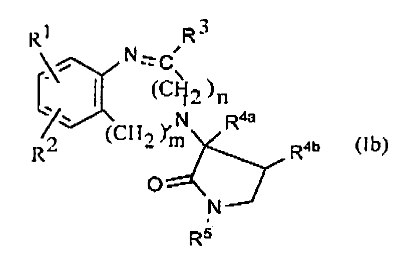 diese zu einer Verbindung der oben genannten Formel (Ia) oxidiert und gegebenenfalls andere Reste R⁴ und/oder den Rest R⁵ einführt.

13. Verfahren zur Herstellung von in 3-Position heterocyclisch substituierten Pyrrolidin(thi)onen der allgemeinen Formel (I), in denen m = 1 und n = 0 bedeuten und R³ für H oder OH steht, indem ein Formamidderivat der allgemeinen Formel (XXIII), 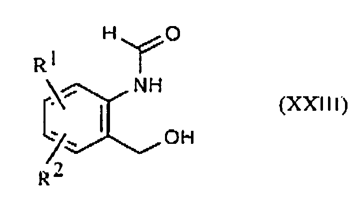 in der R¹ und R² wie oben definiert sind, zunächst in an sich bekannter Weise zu einem Benzaldehyd der allgemeinen Formel (XXIV) oxidiert wird, 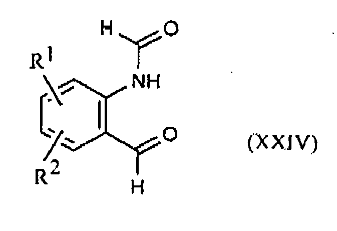 welches durch reduktive Aminierung mit Asparagin oder entsprechenden Derivaten mit einem Rest R⁴ ungleich H unter Einsatz von komplexen Borhydriden in eine Verbindung der allgemeinen Formel (XXV) übergeführt wird, 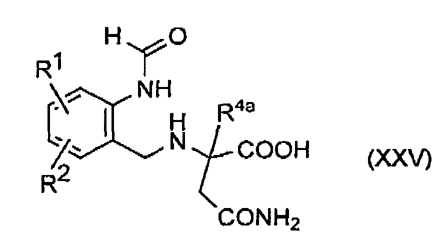 die zu einem Succinimid der allgemeinen Formel (XXVI) cyclisiert wird, vorzugsweise nach vorherigem Schutz der Aminfunktion, die anschließend wieder abgespalten wird, 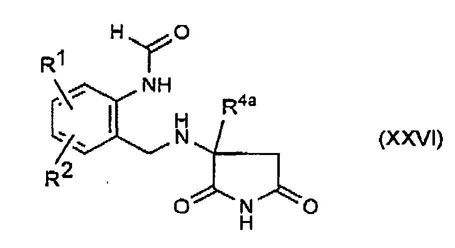 woraus in protischen Lösungsmitteln unter Säurekatalyse schließlich Verbindungen der allgemeinen Formel (I) erhalten werden, in denen R¹, R² und R^{4a} wie oben definiert sind, m für 1, n für 0 steht, eine C=N-Doppelbindung vorliegt und R³ bzw. R⁵ ein Wasserstoffatom darstellen, das im Falle von R⁵ gegebenenfalls gegen die übrigen definitionsgemäßen Substituenten ausgetauscht wird,
und wobei
wenn nach dem Schritt der reduktiven Aminierung der Verbindung der allgemeinen Formel (XXIV) das Reaktionsgemisch mit Säuren behandelt wird, daraus Verbindungen der allgemeinen Formel (XXVII), in denen R¹, R² und R⁴ wie oben definiert sind, hergestellt werden 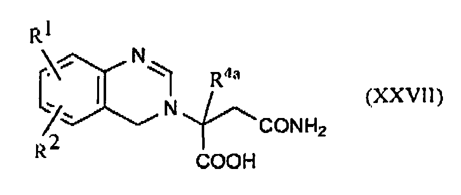 die durch Cyclisierung in Verbindungen der allgemeinen Formel (I), in denen m = 1 und n = 0 bedeuten, eine C=N-Doppelbindung vorliegt, R¹, R² und R⁴ wie oben definiert sind und R³ bzw. R⁵ für Wasserstoff stehen, übergeführt werden,
wonach gegebenenfalls andere definitionsgemäße Reste R⁴ und /oder R⁵ eingeführt werden.

14. Arzneimittel enthaltend als Wirkstoff wenigstens eine Verbindung der allgemeinen Formel (I) nach Anspruch 1 und/oder deren Enantiomere, Diastereomere, Basen, oder Salze von physiologisch verträglichen organischen oder anorganischen Säuren.

15. Arzneimittel nach Anspruch 14 mit immunmodulatorischer Wirkung zur Behandlung und/oder Prophylaxe von Entzündungs- sowie Autoimmunerkrankungen und/oder hämatologisch-onkologischen Erkrankungen.

16. Verwendung wenigstens einer Verbindung der allgemeinen Formel (I) nach Anspruch 1 und/oder deren Enantiomeren, Diastereomeren, Basen oder Salzen von physiologisch verträglichen organischen oder anorganischen Säuren zur Herstellung eines Arzneimittels zur Behandlung und/oder Prophylaxe von Entzündungs- und Autoimmunerkrankungen.

## Claims

1. Pyrrolidine(thi)ones substituted by heterocyclic substituents in the 3-position of the general formula (I) 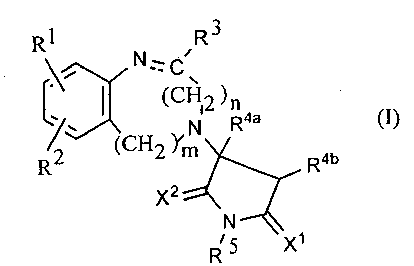 in which
R¹ and R² are identical or different from one another and can be H, Br, Cl, F, I, CF₃, OH, NO₂, NR^{5'}R^{6'} alkyl, alkoxy, alkylthio, alkylsulfinyl, alkylsulfonyl, aryl, heteroaryl, in each case branched or unbranched, or together denote a fused-on benzene ring, wherein the rings are optionally substituted by R¹ and/or R² and R¹ and R² are as defined above, and R^{5'} and R^{6'} are chosen from H, or alkyl or acyl radicals,
R³ represents H, the methyl group or, in the case where a C-N single bond is present, together with the C atom represents a carbonyl group,
R^{4a} and/or R^{4b} denotes H, alkyl, aryl or heteroaryl,
R⁵ represents H, aryl, heteroaryl, alkyl, a CH₂-OH group or a radical CH₂-NR⁶R⁷, in which R⁶ and R⁷ are identical or different and denote an alkyl group having 1-6 C atoms (straight-chain or branched) or together with the N atom denote a pyrrolidine, piperidine, hexamthyleneimine or morpholine ring,
X¹ and/or X² represent O or S and the remaining X¹ or X² denotes H₂,
n denotes 0 or 1 and
m denotes 1 or 2
and pure enantiomers or non-racemic enantiomer mixtures, racemates, diastereomers or diastereomer mixtures thereof, in the form both of their free bases and of salts with physiologically acceptable organic or inorganic acids.

2. Compounds according to claim 1, **characterized in that** they have a C=N double bond and R¹ and R² are identical or different from one another and denote H, Br, Cl, F, CF₃, NO₂, NH₂, C₁₋₃-alkyl, C₁₋₃-alkoxy, or together a fused-on benzene ring, R³ represents H or a methyl group, R^{4a} represents H or a methyl group, R^{4b} represents H or a phenyl group and R⁵ represents H or methyl, X¹ and X² represent O and n = 0 and m = 1.

3. Compounds according to one of claims 1 or 2, **characterized in that** they have a C=N double bond and R¹ and R² are identical or different from one another and denote H, Cl or F, R³, R^{4a}, R^{4b} and R⁵ denote hydrogen, X¹ and X² represent O and n = 0 and m = 1.

4. Compounds according to claim 1:
3-(5-chloro-7-fluoro-4*H-*quinazolin-3-yl)-pyrrolidine-2,5-dione **(1)**
3-(7-chloro-5-fluoro-4*H-*quinazolin-3-yl)-pyrrolidine-2,5-dione **(2)**
3-(7-fluoro-4*H-*quinazolin-3-yl)-pyrrolidine-2,5-dione and the hydrochloride thereof **(3)**
3-(5,7-difluoro-4*H-*quinazolin-3-yl)-pyrrolidine-2,5-dione and the hydrochloride thereof **(4)**
3-(5,7-dichloro-4*H-*quinazolin-3-yl)-pyrrolidine-2,5-dione **(5)**
3-(5-bromo-4*H-*quinazolin-3-yl)-pyrrolidine-2,5-dione **(6)**
3-(7-trifluoromethyl-4*H-*quinazolin-3-yl)pyrrolidine-2,5-dione **(7)**
3-(5,8-dichloro-4*H-*quinazolin-3-yl)-pyrrolidine-2,5-dione **(8a)**
3-(5-methyl-4*H-*quinazolin-3-yl)-pyrrolidine-2,5-dione **(8b)**
3-(4*H-*benzo[g]quinazolin-3-yl)-pyrrolidine-2,5-dione **(8c)**
3-(6,7-difluoro-4*H-*quinazolin-3-yl)-pyrrolidine-2,5-dione **(8d)**
3-(6,8-dichloro-4*H-*quinazolin-3-yl)-pyrrolidine-2,5-dione **(8e)**
3-(6-nitro-4*H-*quinazolin-3-yl)-pyrrolidine-2,5-dione **(8f)**
3-(7-chloro-4*H-*quinazolin-3-yl)-pyrrolidine-2,5-dione **(8g)**
3-(7-nitro-4*H-*quinazolin-3-yl)-pyrrolidine-2,5-dione**(8h)**
3-(8-bromo-6-methyl-4*H-*quinazolin-3-yl)-pyrrolidine-2,5-dione **(8i)**
3-(8-chloro-4*H-*quinazolin-3-yl)-pyrrolidine-2,5-dione **(8j)**
3-(8-methoxy-4*H-*quinazolin-3-yl)-pyrrolidine-2,5-dione **(8k)**
3-(6-benzyloxy-4*H-*quinazolin-3-yl)-pyrrolidine-2,5-dione **(8l)**
3-(5,6-dichloro-4*H-*quinazolin-3-yl)-pyrrolidine-2,5-dione **(9a)**
3-(7-methoxy-4*H-*quinazolin-3-yl)-pyrrolidine-2,5-dione **(9b)**
3-(5-chloro-4*H-*quinazolin-3-yl)-pyrrolidine-2,5-dione **(10a)**
3-(6,7-dimethoxy-4*H-*quinazolin-3-yl)-pyrrolidine-2,5-dione **(10b)**
3-(6-chloro-4*H-*quinazolin-3-yl)-pyrrolidine-2,5-dione **(10c)**
3-(4*H-*quinazolin-3-yl)-pyrrolidine-2,5-dione **(11a)**
3-(5-fluoro-4*H-*quinazolin-3-yl)-pyrrolidine-2,5-dione **(11b)**
3-(8-trifluoromethyl-4*H-*quinazolin-3-yl)-pyrrolidine-2,5-dione **(11c)**
1-methyl-3-(4*H-*quinazolin-3-yl)-pyrrolidine-2,5-dione **(11d)**
7-fluoro-1-methyl-3-(4*H-*quinazolin-3-yl)-pyrrolidine-2,5-dione **(11e)**
3-(5-methoxy-4*H-*quinazolin-3-yl)-pyrrolidine-2,5-dione **(12a)**
3-(5-ethoxy-4*H-*quinazolin-3-yl)-pyrrolidine-2,5-dione **(12b)**
3-(5-pentyloxy-4*H-*quinazolin-3-yl)-pyrrolidine-2,5-dione **(12c)**
3-(5-benzyloxy-4*H*-quinazolin-3-yl)-pyrrolidine-2,5-dione **(12d)**
3-(5-isopropoxy-4*H-*quinazolin-3-yl)-pyrrolidine-2,5-dione **(12e)**
3-(5-(2-methoxyethoxy)-4*H-*quinazolin-3-yl)-pyrrolidine-2,5-dione **(12f)**
3-(5-ethanesulfonyl-4*H*-quinazolin-3-yl)-pyrrolidine-2,5-dione **(13a)**
3-(5-ethanesulfinyl-4*H-*quinazolin-3-yl)-pyrrolidine-2,5-dione **(13c)**
3-(5-ethylthio-4*H-*quinazolin-3-yl)-pyrrolidine-2,5-dione **(13b)**
3-(5-nitro-4*H-*quinazolin-3-yl)-pyrrolidine-2,5-dione **(14)**
1-methyl-3-(2-methyl-4*H-*quinazolin-3-yl)-pyrrolidine-2,5-dione **(15a)**
3-(2-methyl-4*H-*quinazolin-3-yl)-pyrrolidine-2,5-dione **(15b)**
3-(5,6-dichloro-2-methyl-4*H-*quinazolin-3-yl)-pyrrolidine-2,5-dione **(15c)**
3-(5,7-dichloro-2-methyl-4*H-*quinazolin-3-yl)-pyrrolidine-2,5-dione **(15d)**
3-(5,7-difluoro-2-methyl-4*H-*quinazolin-3-yl)-pyrrolidine-2,5-dione and the hydrochloride thereof **(15e)**
3-(5,8-dichloro-2-methyl-4*H-*quinazolin-3-yl)-pyrrolidine-2,5-dione **(15f)**
3-(5-benzyloxy-2-methyl-4*H-*quinazolin-3-yl)-pyrrolidine-2,5-dione **(15g)**
3-(5-bromo-2-methyl-4*H-*quinazolin-3-yl)-pyrrolidine-2,5-dione **(15h)**
3-(5-chloro-2-methyl-4*H-*quinazolin-3-yl)-pyrrolidine-2,5-dione **(15i)**
3-(5-chloro-7-fluoro-2-methyl-4*H-*quinazolin-3-yl)-pyrrolidine-2,5-dione and the hydrochloride thereof **(15j)**
3-(5-ethoxy-2-methyl-4*H-*quinazolin-3-yl)-pyrrolidine-2,5-dione **(15k)**
3-(5-fluoro-2-methyl-4*H*-quinazolin-3-yl)-pyrrolidine-2,5-dione **(15l)**
3-(5-isopropoxy-2-methyl-4*H*-quinazolin-3-yl)-pyrrolidine-2,5-dione **(15m)**
3-(2,5-dimethyl-4*H*-quinazolin-3-yl)-pyrrolidine-2,5-dione **(15n)**
3-(5-methoxy-2-methyl-4*H*-quinazolin-3-yl)-pyrrolidine-2,5-dione **(15o)**
3-(2-methyl-5-nitro-4*H*-quinazolin-3-yl)-pyrrolidine-2,5-dione **(15p)**
3-(2-methyl-5-pentyloxy-4*H-*quinazolin-3-yl)-pyrrolidine-2,5-dione **(15q)**
3-(5-ethylthio-2-methyl-4*H-*quinazolin-3-yl)-pyrrolidine-2,5-dione **(15r)**
3-(5-ethanesulfonyl-2-methyl-4*H*-quinazolin-3-yl)-pyrrolidine-2,5-dione **(15s)**
3-(2-methyl-4*H-*benzo[g]quinazolin-3-yl)-pyrrolidine-2,5-dione **(15t)**
3-(6,7-difluoro-2-methyl-4*H-*quinazolin-3-yl)-pyrrolidine-2,5-dione **(15u)**
3-(6,7-dimethoxy-2-methyl-4*H*-quinazolin-3-yl)-pyrrolidine-2,5-dione **(15v)**
3-(6,8-dichloro-2-methyl-4H*-*quinazolin-3-yl)-pyrrolidine-2,5-dione (15w)
3-(6-benzyloxy-2-methyl-4H*-*quinazolin-3-yl)-pyrrolidine-2,5-dione **(15x)**
3-(6-chloro-2-methyl-4*H*-quinazolin-3-yl)-pyrrolidine-2,5-dione **(15y)**
3-(2-methyl-6-nitro-4*H*-quinazolin-3-yl)-pyrrolidine-2,5-dione **(15z)**
3-(2-methyl-7-trifluoromethyl-4*H-*quinazolin-3-yl)-pyrrolidine-2,5-dione **(15aa)**
3-(7-chloro-2-methyl-4*H-*quinazolin-3-yl)-pyrrolidine-2,5-dione **(15bb)**
3-(7-fluoro-2-methyl-4*H-*quinazolin-3-yl)-pyrrolidine-2,5-dione **(15cc)**
3-(7-methoxy-2-methyl-4*H*-quinazolin-3-yl)-pyrrolidine-2,5-dione **(15dd)**
3-(2-methyl-7-nitro-4*H-*quinazolin-3-yl)-pyrrolidine-2,5-dione **(15ee)**
3-(8-bromo-2,6-dimethyl-4*H-*quinazolin-3-yl)-pyrrolidine-2,5-dione **(15ff)**
3-(2-methyl-8-trifluoromethyl-4*H-*quinazolin-3-yl)-pyrrolidine-2,5-dione **(15gg)**
3-(8-chloro-2-methyl-4*H-*quinazolin-3-yl)-pyrrolidine-2,5-dione **(15hh)**
3-(8-methoxy-2-methyl-4*H-*quinazolin-3-yl)-pyrrolidine-2,5-dione **(15ii)**
3-(5-(2-methoxyethoxy)-2-methyl-4*H-*quinazolin-3-yl) pyrrolidine-2,5-dione **(15jj)**
3-(7-amino-4*H-*quinazolin-3-yl)-pyrrolidine-2,5-dione hydrochloride **(16a)**
3-(7-amino-2-methyl-4*H-*quinazolin-3-yl)-pyrrolidine-2,5-dione hydrochloride **(16b)**
3-(6-amino-4*H*-quinazolin-3-yl)-pyrrolidine-2,5-dione hydrochloride **(16c)**
3-(6-amino-2-methyl-4*H-*quinazolin-3-yl)-pyrrolidine-2,5-dione hydrochloride **(16d)**
3-(5-amino-4*H-*quinazolin-3-yl)-pyrrolidine-2,5-dione hydrochloride **(16e)**
3-(5-amino-2-methyl-4*H-*quinazolin-3-yl)-pyrrolidine-2,5-dione hydrochloride **(16f)**
3-(5-hydroxy-4*H-*quinazolin-3-yl)-pyrrolidine-2,5-dione hydrochloride **(17a)**
3-(6-hydroxy-2-methyl-4*H-*quinazolin-3-yl)-pyrrolidine-2,5-dione hydrochloride **(17b)**
3-(5-hydroxy-2-methyl-4*H-*quinazolin-3-yl)-pyrrolidine-2,5-dione hydrochloride **(17c)**
3-(5-hydroxy-2-methyl-4*H-*quinazolin-3-yl)-pyrrolidine-2,5-dione hydrochloride **(17d)**
3-(7-fluoro-4*H-*quinazolin-3-yl)-5-thioxopyrrolidin-2-one hydrobromide **(18a)**
3-(4*H-*quinazolin-3-yl)-5-thioxopyrrolidin-2-one hydrobromide **(18b)**
3-methyl-3-(4*H-*quinazolin-3-yl)-pyrrolidine-2,5-dione **(19a)**
3-(5-bromo-4*H-*quinazolin-3-yl)-3-methyl-pyrrolidine-2,5-dione **(19b)**
3-(2-oxo-1,4-dihydro-2*H-*quinazolin-3-yl)-pyrrolidine-2,5-dione **(20a)**
3-(7-fluoro-2-oxo-1,4-dihydro-2*H-*quinazolin-3-yl)-pyrrolidine-2,5-dione **(20b)**
**3-(2-oxo-1,4-dihydro-2*H-*quinazolin-3-yl)-4-phenylpyrrolidine-2,5-**dione **(20c)**
3-(2-thio-1,4-dihydro-2*H-*quinazolin-3-yl)-pyrrolidine-2,5-dione **(20d)**
3-(2-(methylthio)-4*H-*quinazolin-3-yl)pyrrolidine-2,5-dione hydroiodide **(21)**
3-(2-(dimethylamino)-4-quinazolin-3-yl)pyrrolidine-2,5-dione hydroiodide **(22)**
3-(4*H-*quinazolin-3-yl)-pyrrolidin-2-one **(23).**

5. Process for the preparation of pyrrolidine(thi)ones substituted by heterocyclic substituents in the 3-position of the general formula (I) according to claim 1, wherein
a) for the preparation of the compounds where X¹ and X² are oxygen, 2-aminobenzylamines of the general formula (II) 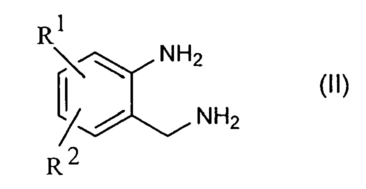 in which R¹ and R² are as defined above, are first reacted with pyrrole-2,5-diones of the general formula (III) 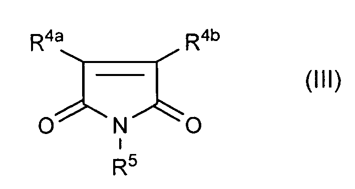 in which R^{4a} and R^{4b} are as defined above, to give amines of the general formula (IVa) 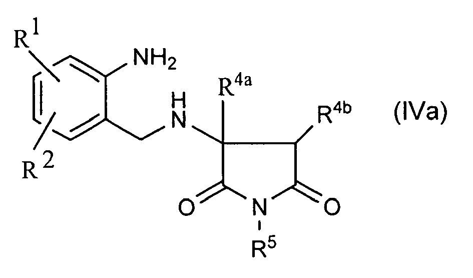 wherein Instead of the pyrrole-2,5-diones, 3-bromo-pyrrolidine-2,5-diones of the general formula (IIIa) 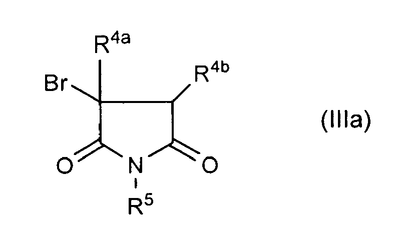 are optionally employed, and
b) for the preparation of the compounds in which at least one of the radicals X¹ and X² denotes sulfur compounds of the general formula (IVb) in which at least one O from formula (IVa) is replaced by S 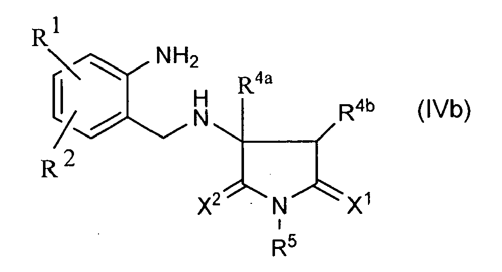 are prepared from (IVa), preferably after protection of the two nitrogen atoms which are not bonded to R⁵, by exchange of at least one O for X¹ or X² as S with thionation reagents known to the person skilled in the art, and subsequent deprotection,
wherein by subsequent reaction of (IVa) or (IVb) with compounds of the general formula (V)
R³-C(OR^{y})₃ (V)
in which R³ denotes hydrogen or the methyl group and R^{y} represents a straight-chain or branched C₁-C₄-alkyl radical, or with amidine salts of the general formula (VI) 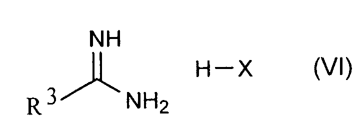 in which R³ is as defined above and X represents the anion of a suitable acid, or with carboxylic acid esters of the general formula (Vb) 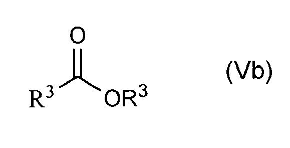 compounds of the general formula (I) with the corresponding meaning of R³ are then prepared both from (IVa) and from (IVb).

6. Process according to claim 5, **characterized in that** compounds of the general formula (I) in which R³ together with the C atom represents a geminally substituted C atom are prepared by reaction of (IVa) or (IVb) with ketones of the general formula (Vc) 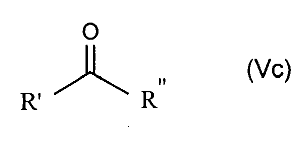 in which R' and R" independently of one another represent an alkyl, aryl or heteroaryl radical.

7. Process according to claim 5, **characterized in that** compounds of the general formula (I) in which R³ together with the C atom represents a carbonyl group are prepared by reaction of (IVa) or (IVb) with Cl units of the general formula (VII) 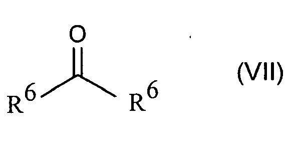 in which R⁶ represents Cl, the imidazol-1-yl radical, a C₁-C₄-alkoxy group, a phenyloxy group or a phenyloxy group substituted by the nitro group, chlorine or fluorine or a thiomethyl group, or by reaction of (IVa) or (IVb) with C₁-C₄-alkyl, phenyl or substituted phenyl esters, preferably 4-nitro-, 4-chloro- or 4-fluorophenyl esters, of chloroformic acid.

8. Process according to claim 5, **characterized in that** compounds of the general formula (I) in which R³ together with the C atom represents a carbonyl group are prepared by reaction of (IVa) or (IVb) with a Cl unit of the general formula VIII
C(OR⁷)₄ (VIII)
in which R⁷ represents the methyl or ethyl radical.

9. Process according to claim 5, **characterized in that** the reaction of the aminobenzylamines of the general formula (II) with the pyrrole-2,5-diones of the general formula (III) is carried out in inert solvents, preferably ethyl acetate, at room temperature.

10. Process according to claim 5, **characterized in that** the reaction of the compounds of the general formula (IVa and IVb) with the compounds of the general formula (V) is carried out either without a solvent or in an organic carboxylic acid, preferably acetic acid, in a temperature range of from 10 to 150 °C.

11. Process for the preparation of pyrrolidine(thi)ones substituted by heterocyclic substituents in the 3-position of the general formula (I) according to claim 1, wherein an amino compound of the general formula (IX) 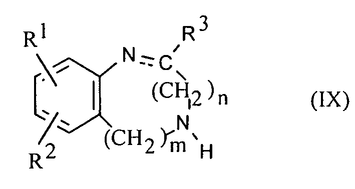 is first alkylated with a pyrrole-2,5-dione of the general formula (III) or a 3-bromopyrrolidine-2,5-dione derivative of the general formula (IIIa) 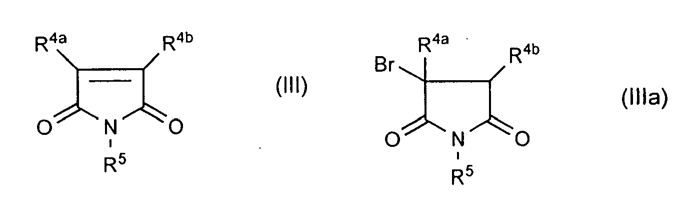 to give a compound of the general formula (Ia) 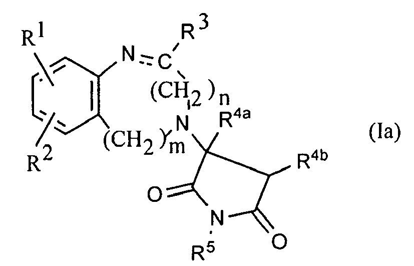 wherein in the compounds (Ia), (II) and (III) the radicals R¹ to R^{4b} are as defined above, and, if R⁵ is not to represent hydrogen, this radical is subsequently introduced by reaction with formaldehyde, optionally together with an amine of the general formula HNR⁶R⁷, in which R⁶ and R⁷ are as defined above, and optionally for X¹ and/or X² as S the sulfurization process described above is used.

12. Process for the preparation of pyrrolidine(thi)ones substituted by heterocyclic substituents in the 3-position of the general formula (I) according to claim 1, wherein an amino compound of the formula (II) is first alkylated with a 3-bromopyrrolidin-2-one derivative of the formula (X) 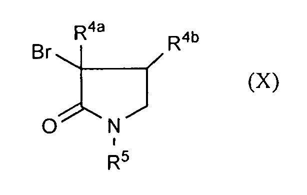 to give a compound of the general formula (Ib) 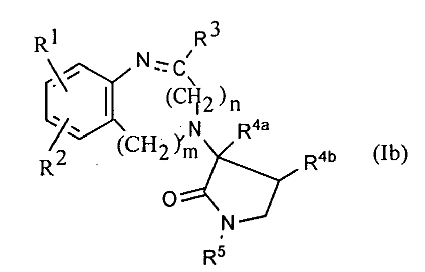 this is oxidized to give a compound of the abovementioned formula (Ia) and other radicals R⁴ and/or the radical R⁵ are optionally introduced.

13. Process for the preparation of pyrrolidine(thi)ones substituted by heterocyclic substituents in the 3-position of the general formula (I) in which m = 1 and n = 0 and R³ represents H or OH, by first oxidizing a formamide derivative of the general formula (XXIII) 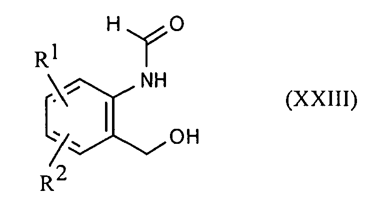 in which R¹ and R² are as defined above, in a manner known per se to give a benzaldehyde of the general formula (XXIV), 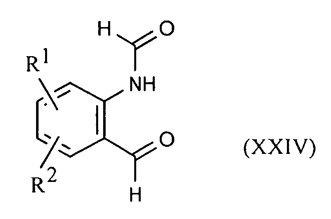 which is converted by reductive amination with asparagine or corresponding derivatives having a radical R⁴ which is not H using complex borohydrides into a compound of the general formula (XXV) 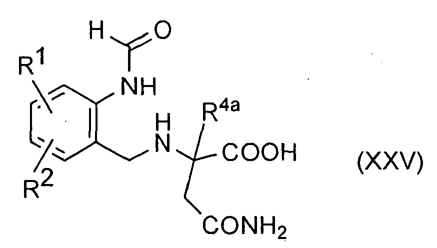 which is cyclized, preferably after prior protection of the amine function, which is subsequently split off again, to give a succinimide of the general formula (XXVI), 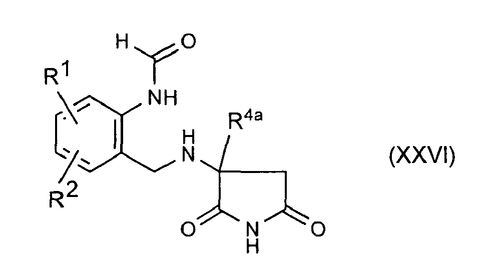 from which compounds of the general formula (I) in which R¹, R² and R^{4a} are as defined above, m represents 1, n represents 0, a C=N double bond is present and R³ and R⁵ represent a hydrogen atom which, in the case of R⁵, can optionally be exchanged for the remaining substituents according to the definition, are finally obtained in protic solvents under acid catalysis.
and wherein
if after the step of reductive amination of the compound of the general formula (XXIV) the reaction mixture is treated with acids, compounds of the general formula (XXVII) in which R¹, R² and R⁴ are as defined above 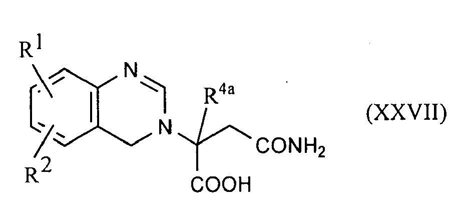 are prepared therefrom, and are converted into compounds of the general formula (I) in which m = 1 and n = 0, a C=N double bond is present, R¹, R² and R⁴ are as defined above and R³ and R⁵ represent hydrogen, by cyclization,
after which other radicals R⁴ and/or R⁵ according to the definition are optionally introduced.

14. Medicament containing as the active compound at least one compound of the general formula (I) according to claim 1 and/or their enantiomers, diastereomers, bases or salts of physiologically acceptable organic or inorganic acids.

15. Medicament according to claim 14 having an immunomodulatory action for treatment and/or prophylaxis of inflammatory and autoimmune diseases and/or haematological-oncological diseases.

16. Use of at least one compound of the general formula (I) according to claim 1 and/or their enantiomers, diastereomers, bases or salts of physiologically acceptable organic or inorganic acids for the preparation of a medicament for treatment and/or prophylaxis of inflammatory and autoimmune diseases.

## Revendications

1. Pyrrolidine(thi)ones à substitution hétérocyclique en position 3 de formule générale (I) 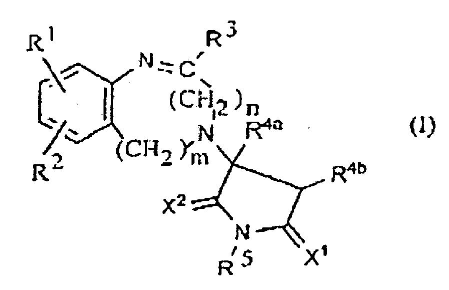 dans lesquelles
R¹ et R², identiques ou différents l'un de l'autre, peuvent être H, Br, Cl, F, I, CF₃, OH, NO₂, NR^{5'}R^{6'}, un groupe alkyle, alkoxy, alkylthio, alkylsulfinyle, alkylsulfonyle, aryle, hétéroaryle, respectivement ramifiés ou non ramifiés ou pris conjointement désignent un cycle benzocondensé, les cycles étant éventuellement substitués par R¹ et/ou R² et R¹ et R² étant tels que définis ci-dessus, et R⁵' et R⁶' étant choisis parmi H, des radicaux alkyle ou acyle,
R³ est H, le groupe méthyle ou un groupe carbonyle au cas où il existe une liaison simple C-N, conjointement avec l'atome de carbone,
R^{4a} et/ou R^{4b} est H, un groupe alkyle, aryle ou hétéroaryle
R⁵ désigne H, un groupe aryle, hétéroaryle, alkyle, un groupe CH₂-OH ou un radical CH₂-NR⁶R⁷ dans lequel R⁶ est R⁷ sont identiques ou différents et représentent un groupe alkyle comportant 1 à 6 atomes de carbone (linéaire ou ramifié) ou conjointement avec l'atome de N, un cycle pyrrolidine, pipéridine, hexaméthylène-imine ou morpholine,
X¹ et/ou X² désignent O ou S et les X¹ou X² restants désignent H₂,
n a la valeur de 0 ou 1 et
m a la valeur de 1 ou 2
et leurs énantiomères purs ou leurs mélanges d'énantiomères non racémiques, racémates, diastéréomères ou mélanges de diastéréomères et sous la forme de leurs bases libres et également de sels avec des acides organiques ou inorganiques physiologiquement acceptables.

2. Composés selon la revendication 1, **caractérisés en ce qu'**ils présentent une double liaison C=N et R¹ et R², identiques ou différents l'un de l'autre représentent H, Br, Cl, F, CF₃, NO₂, alkyle en C₁ à C₃, alkoxy en C₁ à C₃ ou pris conjointement désignent un cycle benzocondensé, R³ représente H ou un groupe méthyle, R^{4a} représente H ou un groupe méthyle, R^{4b} représente H ou un groupe phényle et R⁵ représente H ou un groupe méthyle, X¹ et X² sont égaux à O et n = 0 et m = 1.

3. Composés selon l'une des revendications 1 ou 2, **caractérisés en ce qu'**ils présentent une double liaison C=N et R¹ et R², identiques ou différents l'un de l'autre désignent H, Cl ou F, R³, R^{4a}, R^{4b} et R⁵ désignent un atome d'hydrogène, X¹ et X² désignent un atome d'oxygène, et n = 0 et m = 1.

4. Composés selon la revendication 1 :
3-(5-chloro-7-fluoro-4*H-*quinazoline-3-yl)-pyrrolidine-2,5-dione (1),
3-(7-chloro-5-fluoro-4*H-*quinazoline-3-yl)-pyrrolidine-2,5-dione (2),
3-(7-fluoro-4*H-*quinazoline-3-yl)-pyrrolidine-2,5-dione et son chlorhydrate (3)
3-(5,7-difluoro-4*H-*quinazoline-3-yl)-pyrrolidine-2,5-dione et son chlorhydrate (4)
3-(5,7-dichloro-4*H-*quinazoline-3-yl)-pyrrolidine-2,5-dione (5)
3-(5-bromo-4*H-*quinazoline-3-yl)-pyrrolidine-2,5-dione (6)
3-(7-trifluorométhyl-4*H-*quinazoline-3-yl)-pyrrolidine-2,5-dione (7)
3-(5,8-dichloro-4*H-*quinazoline-3-yl)-pyrrolidine-2,5-dione (8a)
3-(5-méthyl-4*H-*quinazoline-3-yl)-pyrrolidine-2,5-dione (8b)
3-(4*H-*benzo[g]quinazoline-3-yl)-pyrrolidine-2,5-dione (8c)
3-(6,7-difluoro-4*H-*quinazoline-3-yl)-pyrrolidine-2,5-dione (8d)
3-(6,8-dichloro-4*H-*quinazoline-3-yl)-pyrrolidine-2,5-dione (8e)
3-(6-nitro-4*H-*quinazoline-3-yl)-pyrrolidine-2,5-dione (8f)
3-(7-chloro-4*H-*quinazoline-3-yl)-pyrrolidine-2,5-dione (8g)
3-(7-nitro-4*H-*quinazoline-3-yl)-pyrrolidine-2,5-dione (8h)
3-(8-bromo-6-méthyl-4*H-*quinazoline-3-yl)-pyrrolidine-2,5-dione (8i)
3-(8-chloro-4*H-*quinazoline-3-yl)-pyrrolidine-2,5-dione (8j)
3-(8-méthoxy-4*H-*quinazoline-3-yl)-pyrrolidine-2,5-dione (8k)
3-(6-benzyloxy-4*H-*quinazoline-3-yl)-pyrrolidine-2,5-dione (81)
3-(5,6-dichloro-4*H-*quinazoline-3-yl)-pyrrolidine-2,5-dione (9a)
3-(7-méthoxy-4*H-*quinazoline-3-yl)-pyrrolidine-2,5-dione (9b)
3-(5-chloro-4*H-*quinazoline-3-yl)-pyrrolidine-2,5-dione (10a)
3-(6,7-diméthoxy-4*H-*quinazoline-3-yl)-pyrrolidine-2,5-dione (10b)
3-(6-chloro-4*H-*quinazoline-3-yl)-pyrrolidine-2,5-dione (10c)
3-(4*H-*quinazoline-3-yl)-pyrrolidine-2,5-dione (11a)
3-(5-fluoro-4*H-*quinazoline-3-yl)-pyrrolidine-2,5-dione (11b)
3-(8-trifluorométhyl-4*H-*quinazoline-3-yl)-pyrrolidine-2,5-dione (11c)
1-méthyl-3-(4*H-*quinazoline-3-yl)-pyrrolidine-2,5-dione (11d)
7-fluoro-1-méthyl-3-(4*H-*quinazoline-3-yl)-pyrrolidine-2,5-dione (11e)
3-(5-méthoxy-4*H-*quinazoline-3-yl)-pyrrolidine-2,5-dione (12a)
3-(5-éthoxy-4*H-*quinazoline-3-yl)-pyrrolidine-2,5-dione (12b)
3-(5-pentyloxy-4*H-*quinazoline-3-yl)-pyrrolidine-2,5-dione (12c)
3-(5-benzyloxy-4*H-*quinazoline-3-yl)-pyrrolidine-2,5-dione (12d)
3-(5-isopropoxy-4*H-*quinazoline-3-yl)-pyrrolidine-2,5-dione (12e)
3-(5-(2-méthoxyéthoxy)-4*H-*quinazoline-3-yl)-pyrrolidine-2,5-dione (12f)
3-(5-éthanesulfonyl-4*H-*quinazoline-3-yl)-pyrrolidine-2,5-dione (13a)
3-(5-éthanesulfinyl-4*H-*quinazoline-3-yl)-pyrrolidine-2,5-dione (13c)
3-(5-éthylthio-4*H-*quinazoline-3-yl)-pyrrolidine-2,5-dione (13b)
3-(5-nitro-4*H-*quinazoline-3-yl)-pyrrolidine-2,5-dione (14)
1-méthyl-3-(2-méthyl-4*H-*quinazoline-3-yl)-pyrrolidine-2,5-dione (15a)
3-(2-méthyl-4*H-*quinazoline-3-yl)-pyrrolidine-2,5-dione (15b)
3-(5,6-dichloro-2-méthyl-4*H-*quinazoline-3-yl)-pyrrolidine-2,5-dione (15c)
3-(5,7-dichloro-2-méthyl-4*H-*quinazoline-3-yl)-pyrrolidine-2,5-dione (15d)
3-(5,7-difluoro-2-méthyl-4*H-*quinazoline-3-yl)-pyrrolidine-2,5-dione et son chlorhydrate (15e)
3-(5,8-dichloro-2-méthyl-4*H-*quinazoline-3-yl)-pyrrolidine-2,5-dione (15f)
3-(5-benzyloxy-2-méthyl-4*H-*quinazoline-3-yl)-pyrrolidine-2,5-dione (15g)
3-(5-bromo-2-méthyl-4*H-*quinazoline-3-yl)-pyrrolidine-2,5-dione (15h)
3-(5-chloro-2-méthyl-4*H-*quinazoline-3-yl)-pyrrolidine-2,5-dione (15i)
3-(5-chloro-7-fluoro-2-méthyl-4*H-*quinazoline-3-yl)-pyrrolidine-2,5-dione et son chlorhydrate (15j)
3-(5-éthoxy-2-méthyl-4*H-*quinazoline-3-yl)-pyrrolidine-2,5-dione (15k)
3-(5-fluoro-2-méthyl-4*H-*quinazoline-3-yl)-pyrrolidine-2,5-dione (151)
3-(5-isopropoxy-2-méthyl-4*H-*quinazoline-3-yl)-pyrrolidine-2,5-dione (15m)
3-(2,5-diméthyl-4*H-*quinazoline-3-yl)-pyrrolidine-2,5-dione (15n)
3-(5-méthoxy-2-méthyl-4*H-*quinazoline-3-yl)-pyrrolidine-2,5-dione (15o)
3-(2-méthyl-5-nitro-4*H-*quinazoline-3-yl)-pyrrolidine-2,5-dione (15p)
3-(2-méthyl-5-pentyloxy-4*H-*quinazoline-3-yl)-pyrrolidine-2,5-dione (15q)
3-(5-éthylthio-2-méthyl-4*H-*quinazoline-3-yl)-pyrrolidine-2,5-dione (15r)
3-(5-éthanesulfonyl-2-méthyl-4*H-*quinazoline-3-yl)-pyrrolidine-2,5-dione (15s)
3-(2-méthyl-4*H-*benzo[g]quinazoline-3-yl)-pyrrolidine-2,5-dione (15t)
3-(6,7-difluoro-2-méthyl-4*H-*quinazoline-3-yl)-pyrrolidine-2,5-dione (15u)
3-(6,7-diméthoxyhloro-2-méthyl-4*H-*quinazoline-3-yl)-pyrrolidine-2,5-dione (15v)
3-(6,8-dichloro-2-méthyl-4*H-*quinazoline-3-yl)-pyrrolidine-2,5-dione (15w)
3-(6-benzyloxy-2-méthyl-4*H-*quinazoline-3-yl)-pyrrolidine-2,5-dione (15x)
3-(6-chloro-2-méthyl-4*H-*quinazoline-3-yl)-pyrrolidine-2,5-dione (15y)
3-(2-méthyl-6-nitro-4*H-*quinazoline-3-yl)-pyrrolidine-2,5-dione (15z)
3-(2-méthyl-7-trifluorométhyl-4*H-*quinazoline-3-yl)-pyrrolidine-2,5-dione (15aa)
3-(7-chloro-2-méthyl-4*H-*quinazoline-3-yl)-pyrrolidine-2,5-dione (15bb)
3-(7-fluoro-2-méthyl-4*H-*quinazoline-3-yl)-pyrrolidine-2,5-dione (15cc)
3-(7-méthoxy-2-méthyl-4*H-*quinazoline-3-yl)-pyrrolidine-2,5-dione (15dd)
3-(2-méthyl-7-nitro-4*H-*quinazoline-3-yl)-pyrrolidine-2,5-dione (15ee)
3-(8-bromo-2,6-diméthyl-4*H-*quinazoline-3-yl)-pyrrolidine-2,5-dione (15ff)
3-(2-méthyl-8-trifluorométhyl-4*H-*quinazoline-3-yl)-pyrrolidine-2,5-dione (15gg)
3-(8-chloro-2-méthyl-4*H-*quinazoline-3-yl)-pyrrolidine-2,5-dione (15hh)
3-(8-méthoxy-2-méthyl-4*H-*quinazoline-3-yl)-pyrrolidine-2,5-dione (15ii)
3-(5-(2-méthoxyéthoxy)-2-méthyl-4*H-*quinazoline-3-yl)-pyrrolidine-2,5-dione (15jj)
chlorhydrate de 3-(7-amino-4*H-*quinazoline-3-yl)-pyrrolidine-2,5-dione (16a)
chlorhydrate de 3-(7-amino-2-méthyl-4*H-*quinazoline-3-yl)-pyrrolidine-2,5-dione (16b)
chlorhydrate de 3-(6-amino-4*H-*quinazoline-3-yl)-pyrrolidine-2,5-dione (16c)
chlorhydrate de 3-(6-amino-2-méthyl-4*H-*quinazoline-3-yl)-pyrrolidine-2,5-dione (16d)
chlorhydrate de 3-(5-amino-4*H-*quinazoline-3-yl)-pyrrolidine-2,5-dione (16e)
chlorhydrate de 3-(5-amino-2-méthyl-4*H-*quinazoline-3-yl)-pyrrolidine-2,5-dione (16f)
chlorhydrate de 3-(5-hydroxy-4*H-*quinazoline-3-yl)-pyrrolidine-2,5-dione (17a)
chlorhydrate de 3-(6-hydroxy-2-méthyl-4*H-*quinazoline-3-yl)-pyrrolidine-2,5-dione (17b)
chlorhydrate de 3-(5-hydroxy-2-méthyl-4*H-*quinazoline-3-yl)-pyrrolidine-2,5-dione (17c)
chlorhydrate de 3-(5-hydroxy-2-méthyl-4*H-*quinazoline-3-yl)-pyrrolidine-2,5-dione (17d)
bromhydrate de 3-(7-fluoro-4*H-*quinazoline-3-yl)-5-thioxopyrrolidine-2-one (18a)
bromhydrate de 3-(4*H-*quinazoline-3-yl)-5-thioxopyrrolidine-2-one (18b)
3-méthyl-3-(4*H-*quinazoline-3-yl)-pyrrolidine-2,5-dione (19a)
3-(5-bromo-4*H-*quinazoline-3-yl)-3-méthyl-pyrrolidine-2,5-dione (19b)
3-(2-oxo-1,4-dihydro-2*H-*quinazoline-3-yl)-pyrrolidine-2,5-dione (20a)
3-(7-fluoro-2-oxo-1,4-dihydro-2*H-*quinazoline-3-yl)-pyrrolidine-2,5-dione (20b)
3-(2-oxo-1,4-dihydro-2*H-*quinazoline-3-yl)-4-phënyl-pyrrolidine-2,5-dione (20c)
3-(2-thio-1,4-dihydro-2*H-*quinazoline-3-yl)-pyrrolidine-2,5-dione (20d)
iodhydrate de 3-(2-(méthylthio)-4*H-*quinazoline-3-yl)-pyrrolidine-2,5-dione (21)
iodhydrate de 3-(2-(diméthylamino)-4-quinazoline-3-yl)-pyrrolidine-2,5-dione (22)
3-(4*H-*quinazoline-3-yl)-pyrrolidine-2-one (23).

5. Procédé de production de pyrrolidine(thi)ones à substitution hétérocyclique en position 3 de formule générale (I) selon la revendication 1, dans lequel on fait réagir
a) pour la production des composés avec x¹et x² étant de l'oxygène, une 2-aminobenzylamine de formule générale (II), 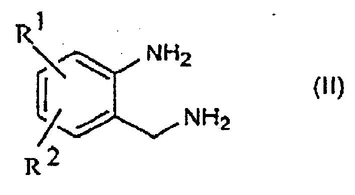 dans laquelle R¹ et R² sont tels que définis ci-dessus, tout d'abord avec des pyrrole-2,5-diones de formule générale (III), 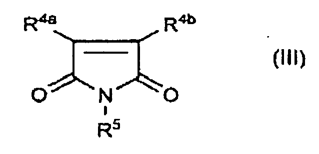 dans laquelle R^{4a} et R^{4b} sont tels que définis ci-dessus, en amines de formule générale (IVa), 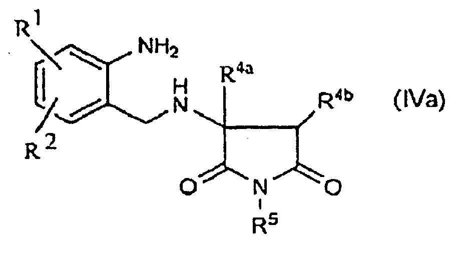 sachant qu'éventuellement, on utilise à la place de la pyrrole-2,5-dione, la 3-bromo-pyrrolidine-2,5-dione de formule générale (IIIa), 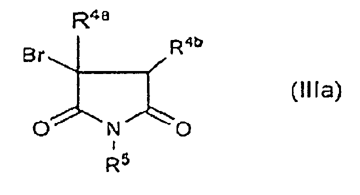 et
b) pour la production des composés dans lesquels au moins l'un des radicaux X¹ et X² désigne le soufre,
on produit des composés de formule générale (IVb), dans lesquels au moins un atome d'oxygène de formule (Iva) est remplacé par un atome de soufre 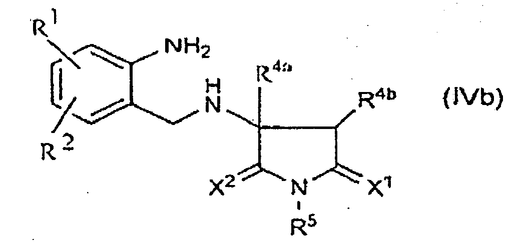 de préférence après la protection des deux atomes d'azote qui ne sont pas liés à R⁵, avec des réactifs de thionisation connus de l'homme du métier, de formule (IVA) par échange d'au moins un atome d'oxygène par X¹ ou X² = à S et déprotection consécutive,
où sont produits par réaction consécutive du composé (Iva) ou (IVb) avec les composés de formule générale (V),
R³-C(OR^{y})₃ (V)
dans laquelle R³ est un atome d'hydrogène ou le groupe méthyle et R^{y} désigne un radical alkyle en C₁ à C₄ à chaîne droite ou ramifiée ou avec des sels d'amidine de formule générale (VI), 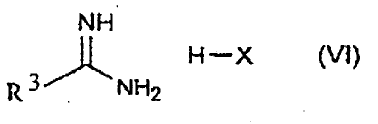 dans laquelle R³ est tel que défini ci-dessus et X est l'anion d'un acide approprié, ou avec des esters d'acide carboxylique de formule générale (Vb) 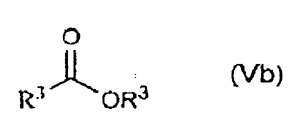 à partir des composés (IVa) ou également (IVb) de formule générale (I) ayant la signification correspondante de R³.

6. Procédé selon la revendication 5, **caractérisé en ce que** des composés de formule générale (1), dans laquelle R³ conjointement avec l'atome de carbone présente un atome de carbone géminé substitué, sont produits par réaction de composés (IVa) ou (IVb) avec des cétones de formule générale (Vc), 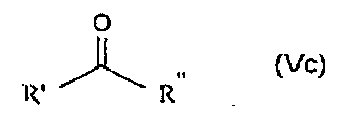 dans laquelle R' et R" désignent indépendamment l'un de l'autre un radical alkyle, aryle ou hétéroaryle.

7. Procédé selon la revendication 5, **caractérisé en ce que** les composés de formule générale (1), dans laquelle R³ représente conjointement avec l'atome de carbone, un groupe carbonyle, sont produits par réaction des composés (IVa) ou (IVb) avec des composants en C₁ de formule générale (VII), 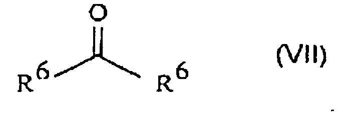 dans laquelle R⁶ représente Cl, le radical imidazole-1-yle, un groupe alkoxy en C₁ à C₄, un groupe phényloxy ou un groupe phényloxy substitué par un groupe nitro, chloro ou fluoro ou désigne un groupe thiométhyle, ou par réaction des composés (IVa) ou (IVb) avec des alkyl- en C₁ à C₄, phényl- ou phénylesters substitués, de préférence des 4-nitro-, 4-chloro- ou 4-fluorophénylesters de l'acide chloroformique.

8. Procédé selon la revendication 5, **caractérisé en ce que** les composés de formule générale (I), dans laquelle R³ forme, conjointement avec l'atome de carbone, un groupe carbonyle, sont produits par réaction de composés (IVa) ou (IVb) avec un composant en C₁ de formule générale VIII,
C(OR⁷)₄ (VIII)
dans laquelle R⁷ désigne un radical méthyle ou éthyle.

9. Procédé selon la revendication 5, **caractérisé en ce que** la réaction de l'aminobenzylamine de formule générale (II) s'effectue avec les pyrène-2,5-diones de formule générale (III) dans des solvants inertes, de préférence de l'éthylester d'acide acétique, à température ambiante.

10. Procédé selon la revendication 5, **caractérisé en ce que** la réaction des composés de formule générale (IVa et IVb) avec les composés de formule générale (V) s'effectue soit sans solvant, soit dans un acide carboxylique organique, de préférence de l'acide acétique, dans une plage de température de 10 à 150°C.

11. Procédé de production de pyrrolidine(thi)ones à substitution hétérocyclique en position 3 de formule générale (I) selon la revendication 1, dans lequel on alkyle tout d'abord un composé à fonction amino de formule générale (IX) 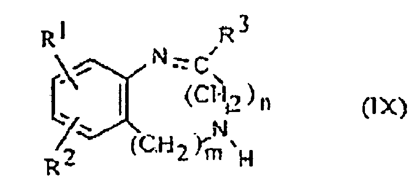 avec une pyrrole-2,5-dione de formule générale (III) ou un dérivé de 3-bromopyrrolidine-2,5-dione de formule générale (IIIa) 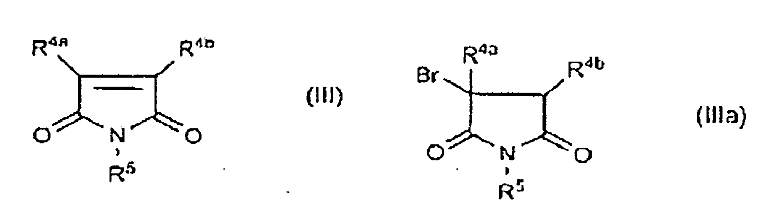 en un composé de formule générale (1A), 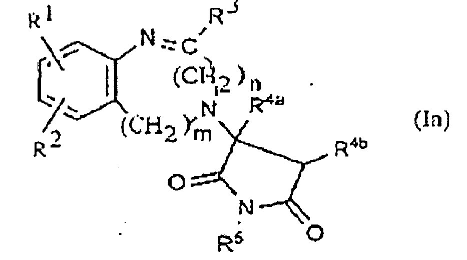 dans laquelle on introduit dans les composés (Ia), (II) et (III), les radicaux R¹ à R^{4b} tels que définis ci-dessus, et ensuite, lorsque R⁵ ne désigne pas un atome d'hydrogène, ce radical est utilisé par conversion avec du formaldéhyde, éventuellement conjointement avec une amine de formule générale HNR⁶R⁷, dans laquelle R⁶ et R⁷ sont tels que définis ci-dessus, et éventuellement, pour X¹ et/ou X² = S, on utilise le procédé de sulfurisation décrit ci-dessus.

12. Procédé de production de pyrrolidine(thi)ones à substitution hétérocyclique en position 3 de formule générale (I) selon la revendication 1, dans lequel on alkyle un composé amino de formule (II) tout d'abord avec un dérivé de 3-bromopyrrolidine-2-one de formule (X) 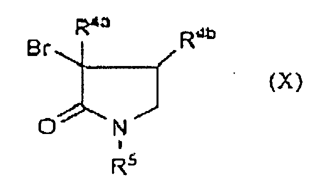 en un composé de formule générale (Ib) 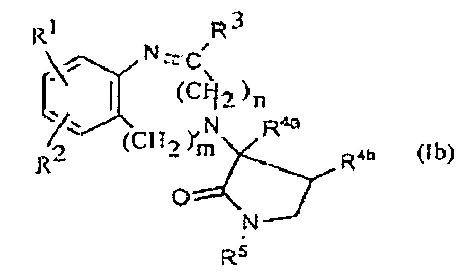 on oxyde celui-ci en un composé de formule générale (Ia) mentionnée ci-dessus et éventuellement, on introduit d'autres radicaux R⁴ et/ou le radical R⁵.

13. Procédé de production de pyrrolidine(thi)diones à substitution hétérocyclique en position 3, de formule (I), dans laquelle m = 1 et n = 0 et R³ désigne H ou OH, un dérivé de formamide de formule générale (XXIII), 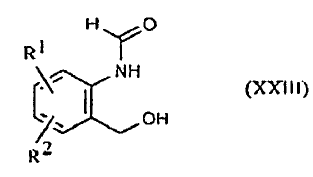 dans laquelle R¹ et R² sont tels que définis ci-dessus, étant tout d'abord oxydé de façon en elle-même connue en un benzaldéhyde de formule générale (XXIV) 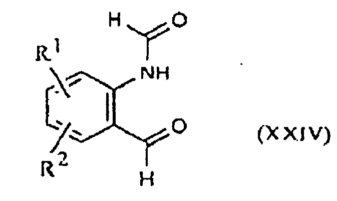 qui est converti par amination réductrice avec de l'asparagine ou des dérivés correspondants avec un radical R⁴ différent de H en utilisant des borohydrures complexes dans un composé de formule générale (XXV), 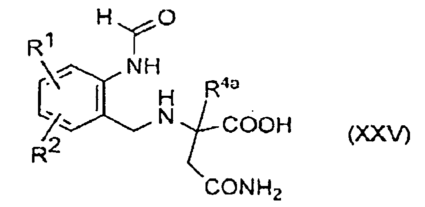 qui est cyclisé en un succinimide de formule générale (XXVI), de préférence après protection préalable de la fonction amine qui est ensuite à nouveau clivé 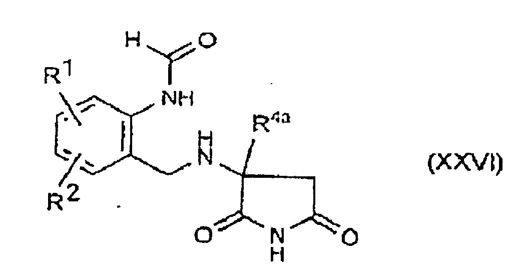 à partir duquel on obtient finalement, dans des solvants protiques par catalyse acide, des composés de formule générale (I), dans lesquels R¹, R² et R^{4a} sont tels que définis ci-dessus, m est égal à 1, n est égal à 0, une double liaison C=N existe et R³ ou R⁵ représentent un atome d'hydrogène qui, dans le cas de R⁵ est échangé éventuellement contre les substituants restants selon les définitions,
et dans laquelle
si après l'étape d'amination réductrice du composé de formule générale (XXIV), le mélange réactionnel est traité avec des acides, des composés de formule générale (XXVII) dans lesquels R¹, R² et R⁴ sont tels que définis ci-dessus, sont produits 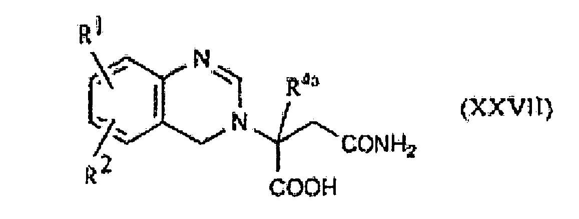 qui sont convertis par cyclisation en composés de formule générale (I) dans lesquels m = 1 et n = 0, une double liaison C=N est présente, R¹, R² et R⁴ sont tels que définis ci-dessus et R³ ou R⁵ désignent un atome d'hydrogène,
éventuellement, d'autres radicaux R⁴ et/ou R⁵ tels que définis étant éventuellement introduits.

14. Médicament contenant comme substance active, au moins un composé de formule générale (I) selon la revendication 1 et/ou leurs énantiomères, diastéréomères, bases ou sels d'acides organiques ou inorganiques physiologiquement acceptables.

15. Médicament selon la revendication 14, présentant une action immunomodulatrice pour le traitement et/ou la prophylaxie de maladies inflammatoires et auto-immunes et/ou de maladies hématologiques - oncologiques

16. Utilisation d'au moins un composé de formule générale (I) selon la revendication 1 et/ou leurs énantiomères, diastéréomères, bases ou sels d'acides organiques ou inorganiques physiologiquement acceptables pour la production d'un médicament destiné au traitement et/ou la prophylaxie de maladies infectieuses et auto-immunes.
